(19) Europäisches Patentamt
European Patent Office
Office européen des brevets

(11) EP 3 644 964 B1

(12) **EUROPEAN PATENT SPECIFICATION**

(45) Date of publication and mention of the grant of the patent:
**10.04.2024 Bulletin 2024/15**

(21) Application number: **18823790.3**

(22) Date of filing: **28.06.2018**

(51) International Patent Classification (IPC):
***A61K 9/00*** *(2006.01)* ***A61K 38/18*** *(2006.01)*
***C12N 5/00*** *(2006.01)* ***A61P 11/00*** *(2006.01)*

(52) Cooperative Patent Classification (CPC):
**A61K 38/1866; A61K 9/0019; A61K 9/0043; A61K 9/0075; A61K 9/0078; A61K 9/008; A61K 9/12; A61K 38/1808; A61P 11/00**

(86) International application number:
**PCT/US2018/040073**

(87) International publication number:
**WO 2019/006155 (03.01.2019 Gazette 2019/01)**

(54) **PROMOTING LUNG GROWTH**

FÖRDERUNG DES LUNGENWACHSTUMS

PROMOTION DE LA CROISSANCE PULMONAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2017 US 201762526230 P**

(43) Date of publication of application:
**06.05.2020 Bulletin 2020/19**

(73) Proprietor: **Children's Medical Center Corporation
Boston, MA 02115 (US)**

(72) Inventor: **PUDER, Mark
Medfield, Massachusetts 02052 (US)**

(74) Representative: **Fish & Richardson P.C.
Highlight Business Towers
Mies-van-der-Rohe-Straße 8
80807 München (DE)**

(56) References cited:
**WO-A1-2016/198121**
**WO-A2-02/086497**
**US-A1- 2006 040 860**
**US-A1- 2008 066 741**
**US-A1- 2009 018 061**
**US-A1- 2010 144 592**
**US-A1- 2013 224 161**

- **MAROMI K. SAKURAI ET AL: "Vascular endothelial growth factor accelerates compensatory lung growth after unilateral pneumonectomy", AMERICAN JOURNAL OF PHYSIOLOGY - LUNG CELLULAR AND MOLECULAR PHYSIOLOGY, vol. 292, no. 3, 1 March 2007 (2007-03-01) , pages 742-747, XP055768256, US ISSN: 1040-0605, DOI: 10.1152/ajplung.00064.2006**
- **JAMES I A O ET AL: "HB-EGF Protects the Lungs after Intestinal Ischemia/Reperfusion Injury", JOURNAL OF SURGICAL RESEARCH, vol. 163, no. 1, 1 September 2010 (2010-09-01), pages 86-95, XP027212281, ACADEMIC PRESS INC., SAN DIEGO, CA, US ISSN: 0022-4804 [retrieved on 2010-04-24]**
- **DUY T. DAO ET AL: "Intranasal delivery of VEGF enhances compensatory lung growth in mice", PLOS ONE, vol. 13, no. 6, E0198700, 7 June 2018 (2018-06-07), XP055768263, DOI: 10.1371/journal.pone.0198700**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- Vuong J T: "42.19 Nasal Delivery of VEGF Increases Shedding of HB-EGF in a Murine Model of Compensatory Lung Growth", 13th Annual Academic Surgical Congress , 31 January 2018 (2018-01-31), XP002801821, Jacksonville, Florida, 20180130-20180201 Retrieved from the Internet: URL:https://www.asc-abstracts.org/abs2018/42-19-nasal-delivery-of-vegf-increases-shedding-of-hb-egf-in-a-murine-model-of-compensatory-lung-growth/ -& Anonymous: "13th Annual Academic Surgical Congress- MEETING PROGRAM", ACADEMIC SURGICAL CONGRESS , 30 January 2018 (2018-01-30), XP055768609, Jacksonville, Florida, 20180130-20180201 Retrieved from the Internet: URL:https://www.academicsurgicalcongress.org/wp-content/uploads/2018/01/ASC18-Final-Program.pdf [retrieved on 2021-01-26]
- KUNIG et al.: "Recombinant human VEGF treatment enhances alveolarization after hyperoxic lung injury in neonatal rats", American Journal of Physiology, vol. 289, no. 4, 1 October 2005 (2005-10-01), pages 529-535, XP055556580,
- BARNEWOLT et al.: "Percent predicted lung volumes as measured on fetal magnetic resonance imaging: a useful biometric parameter for risk stratification in congenital diaphragmatic hernia", Journal of Pediatric Surgery, vol. 42, no. 1, 31 January 2007 (2007-01-31), pages 193-197, XP005821097,
- TAN et al.: "Controlled release of chitosan/heparin nanoparticle-delivered VEGF enhances regeneration of decellularized tissue-engineered scaffolds", International Journal of Nanomedicine, vol. 6, 2 May 2011 (2011-05-02), pages 929-942, XP055556582,
- RAMASAMY et al.: "Regulation of tissue morphogenesis by endothelial cell -derived signals", Trends in Cell Biology, vol. 25, no. 3, 17 December 2014 (2014-12-17), pages 148-157, XP055556585,

## Description

### TECHNICAL FIELD

This disclosure relates to compositions for use in methods of promoting lung growth and improving respiratory functions.

### BACKGROUND

Congenital diaphragmatic hernia (CDH) is a birth defect of the diaphragm. It is a life-threatening pathology in infants and a major cause of death. It occurs in 1/2,500 live births, and the mortality rate of CDH is as high as 50%. An important presentation of CDH is the abdominal organs presence in the chest cavity which causes the lungs to be severely undersized. Thus, promoting lung growth and improving pulmonary functions is important for treating CDH.

US 2006/0040860 describes intra-tracheal application of vascular endothelial growth factor (VEGF) for the prevention of lung damage caused by hyperoxia.

WO 02/086497 describes the use of hypoxia inducible factor 2 alpha for curing neonatal respiratory distress syndrome and as a target for the treatment of pulmonary hypertension.

WO 2016/198121 describes compositions of nanodiamond particles conjugated to VEGF for the treatment of conditions suffering from lung hypoplasia and lung hypertension.

Many other disorders or symptoms can also benefit from a treatment that can promote lung growth and improve pulmonary function, e.g., pulmonary hypoplasia, bronchopulmonary dysplasia (BPD), and pneumonectomy. Thus, there is a need to develop such a treatment.

### SUMMARY

This disclosure relates to, *inter alia,* compositions for use in methods of promoting lung growth and improving respiratory functions.

The present invention is defined by the independent claims. The dependent claims depict other embodiments of the invention.

In accordance with the invention, the disclosure provides compositions comprising VEGF-A for use in methods of promoting lung growth in a subject that include the steps of identifying a subject in need of promoting lung growth; and administering to the subject an effective amount of the composition, wherein the composition is administered through respiratory tract by inhalation, the subject is a newborn, a child, or an adult, and wherein the subject has pulmonary hypoplasia or congenital diaphragmatic hernia (CDH).

In one embodiment, the VEGF-A is rhVEGF-165.

In one embodiment, the percent-predicted lung volume (PPLV) in the subject is less than 15%.

In one embodiment, the subject is monitored for hypotension during and/or after administering the composition to the subject. In one embodiment, one or more agents selected from the group consisting of midodrine, norepinephrine, norepinephrine bitartrate, phenylephrine, droxidopa, ephedrine, and N-Nitroarginine methyl ester are administered to the subject before, during, and/or after administering the composition to the subject.

In yet another aspect, disclosed herein are compositions comprising VEGF-A for use in methods of increasing parenchymal volume, alveolar volume, and/or septal surface area in a subject that include the steps of administering to the subject an effective amount of the composition , wherein the composition is administered through respiratory tract by inhalation, the subject is a newborn, a child, or an adult, and wherein the subject has pulmonary hypoplasia. In one embodiment, the VEGF-A is rhVEGF-165.

In yet another aspect, disclosed herein are compositions comprising VEGF-A for use in methods of promoting lung growth in a subject that include administering to the subject an effective amount of the composition via intravenous administration; and, subsequently, administering to the subject an effective amount of a second composition comprising VEGF-A via the respiratory tract by inhalation, wherein the subject has pulmonary hypoplasia or congenital diaphragmatic hernia (CDH). In one embodiment, the VEGF-A in the composition is rhVEGF-165. In one embodiment, the VEGF-A in the second composition is rhVEGF-165. In one embodiment, the second composition comprises HB-EGF. In one embodiment, the composition is administered to a 0-1 month old baby, a 1-3 month old baby, a 3-6 month old baby, a 6-9 month old baby, or a 9-12 month old baby.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

## DESCRIPTION OF DRAWINGS

The patent or application file contains at least one drawing executed in color. Copies of this patent or patent application publication with color drawing(s) will be provided by the Office upon request and payment of the necessary fee.

**FIG. 1A** is a graph showing the lung volume/body weight ratio of mice receiving daily intraperitoneal (ip) injections of VEGF or saline for either 4 or 10 days postoperatively (shown are mean ± SE).

**FIG. 1B** is a graph showing the liver/body weight ratio of mice receiving daily intraperitoneal (ip) injections of VEGF or saline for either 4 or 10 days postoperatively (shown are mean ± SE).

**FIG. 1C** is a graph showing the kidney/body weight ratio of mice receiving daily intraperitoneal (ip) injections of VEGF or saline for either 4 or 10 days postoperatively (shown are mean ± SE).

**FIG. 1D** is a graph showing the spleen/body weight ratio of mice receiving daily intraperitoneal (ip) injections of VEGF or saline for either 4 or 10 days postoperatively (shown are mean ± SE).

**FIG. 2A** is a graph showing the inspiratory capacity/body weight ratio of mice receiving daily intraperitoneal (ip) injections of VEGF or saline for either 4 or 10 days postoperatively (shown are mean ± SE).

**FIG. 2B** is a graph showing the area under the P-V loop/body weight ratio of mice receiving daily intraperitoneal (ip) injections of VEGF or saline for either 4 or 10 days postoperatively (shown are mean ± SE).

**FIG. 2C** is a graph showing elastance × body weight of mice receiving daily intraperitoneal (ip) injections of VEGF or saline for either 4 or 10 days postoperatively (shown are mean ± SE).

**FIG. 2D** is a graph showing compliance/body weight ratio of mice receiving daily intraperitoneal (ip) injections of VEGF or saline for either 4 or 10 days postoperatively (shown are mean ± SE).

**FIG. 3A** is a graph showing the parenchymal volume/body weight ratio of mice receiving daily intraperitoneal (ip) injections of VEGF or saline for either 4 or 10 days postoperatively (shown are mean ± SE).

**FIG. 3B** is a graph showing the alveolar volume/body weight ratio of mice receiving daily intraperitoneal (ip) injections of VEGF or saline for either 4 or 10 days postoperatively (shown are mean ± SE).

**FIG. 3C** is a graph showing total alveolar count of mice receiving daily intraperitoneal (ip) injections of VEGF or saline for either 4 or 10 days postoperatively (shown are mean ± SE).

**FIG. 3D** is a graph showing septal surface area/body weight ratio of mice receiving daily intraperitoneal (ip) injections of VEGF or saline for either 4 or 10 days postoperatively (shown are mean ± SE).

**FIG. 3E** is a graph showing septal volume/body weight ratio of mice receiving daily intraperitoneal (ip) injections of VEGF or saline for either 4 or 10 days postoperatively (shown are mean ± SE).

**FIG. 3F** is a graph showing mean septal thickness between the control group and the VEGF group on post-operative day (POD) 4 and 10 (shown are mean ± SE).

**FIG. 4A** is a graph showing alveolar density of the control and the VEGF group on post-operative day 4 (shown are mean ± SE).

**FIG. 4B** is a graph showing alveolar count of the control and the VEGF group on post-operative day 4 (shown are mean ± SE).

**FIG. 5** is a graph showing the mRNA level of several genes as determined by Quantitative PCR for both the VEGF group and the control group.

**FIG. 6A** is a set of Western blot images showing protein expression levels of several genes for both the VEGF group and the control group.

**FIG. 6B** is a graph showing fold difference in protein expression levels for both the control group and the VEGF group on post-operative day 4 (shown are mean ± SE).

**FIG. 7A** is a graph showing lung volume/body weight ratio of mice receiving VEGF (iVEGF group) and saline (iSALINE group) via nasal aspiration on post-operative day 4 (shown are mean ± SE).

**FIG. 7B** is a graph showing liver/body weight ratio for the iSALINE and iVEGF group on post-operative day 4 (shown are mean ± SE).

**FIG. 7C** is a graph showing kidney/body weight ratio for the iSALINE and iVEGF group on post-operative day 4 (shown are mean ± SE).

**FIG. 7D** is a graph showing spleen/body weight ratio for the iSALINE and iVEGF group on post-operative day 4 (shown are mean ± SE).

**FIG. 8A** is a graph showing elastance × body weight for the iSALINE and iVEGF group on post-operative day 4 (shown are mean ± SE).

**FIG. 8B** is a graph showing compliance/body weight ratio for the iSALINE and iVEGF group on post-operative day 4 (shown are mean ± SE).

**FIG. 9A** is a graph showing parenchymal volume/body weight ratio for the iSALINE and iVEGF group on post-operative day 4 (shown are mean ± SE).

**FIG. 9B** is a graph showing alveolar volume/body weight ratio for the iSALINE and iVEGF group on post-operative day 4 (shown are mean ± SE).

**FIG. 9C** is a graph showing septal surface area/body weight ratio for the iSALINE and iVEGF group on post-operative day 4 (shown are mean ± SE).

**FIG. 9D** is a graph showing alveolar count for the iSALINE and iVEGF group on post-operative day 4 (shown are mean ± SE).

**FIG. 10** is a graph showing the mRNA level of several genes as determined by Quantitative PCR for the iSALINE and iVEGF group.

**FIG. 11A** is a set of Western blot images showing protein expression levels of several genes for the iSALINE and iVEGF group on post-operative day 4.

**FIG. 11B** is a graph showing fold difference in protein expression levels for the iSALINE and iVEGF group on post-operative day 4 (shown are mean ± SE).

**FIG. 12** is a diagram showing sagittal view of the left pulmonary hilum as seen from the left side and with the right lung in the background.

**FIGS. 13A-13F** are images showing the procedure of left pneumonectomy in piglets.

**FIG. 13A** is an image showing incision marking (I), tip of the scapula (S), and inferior portion of the thirteenth rib (R).

**FIG. 13B** is an image showing the procedure for isolation of the superior pulmonary vein.

**FIG. 13C** is an image showing the procedure for isolation of the pulmonary artery.

**FIG. 13D** is an image showing the procedure for isolation of the left main bronchus.

**FIG. 13E** is an image showing the procedure for isolation of the inferior pulmonary vein.

**FIG. 13F** is an image showing empty left thoracic cavity with the (1) aorta, (2) azygous vein, (3) vagus nerve, (4) phrenic nerve, and (5) bronchial stump.

**FIG. 14A** is a graph showing total lung volume and body weight on post-operative day (POD) 0, 7, 14, and 21 after left pneumonectomy (shown are mean ± SE).

**FIG. 14B** is a graph showing lung volume/body weight ratio on post-operative day (POD) 0, 7, 14, and 21 after left pneumonectomy (shown are mean ± SE).

**FIG. 15A** is a graph showing lung volume/body weight ratio of the control and VEGF group on post-operative day 7 (shown are mean ± SE).

**FIG. 15B** is a graph showing liver/body weight ratio of the control and VEGF group on post-operative day 7 (shown are mean ± SE).

**FIG. 15C** is a graph showing kidney/body weight ratio of the control and VEGF group on post-operative day 7 (shown are mean ± SE).

**FIG. 15D** is a graph showing spleen/body weight ratio of the control and VEGF group on post-operative day 7 (shown are mean ± SE).

**FIG. 16A** is a graph showing parenchymal volume/body weight ratio of the control and VEGF group on post-operative day 7 (shown are mean ± SE).

**FIG. 16B** is a graph showing alveolar volume/body weight ratio of the control and VEGF group on post-operative day 7 (shown are mean ± SE).

**FIG. 16C** is a graph showing septal surface area/body weight ratio of the control and VEGF group on post-operative day 7 (shown are mean ± SE).

**FIG. 16D** is a graph showing alveolar count of the control and VEGF group on post-operative day 7 (shown are mean ± SE).

**FIG. 17A** is a Western blot image showing the stability of rh VEGF in Bronchioalveolar Lavage (BAL) fluid.

**FIG. 17B** is a graph showing the stability of rh VEGF in BAL fluid.

**FIG. 18** is a graph showing the weight change of all piglets in the VEGF group and in the control group.

**FIG. 19A** is a graph showing the total lung volume before formalin fixation.

**FIG. 19B** is a graph showing the total lung volume after formalin fixation.

**FIG. 20A** is a graph showing liver/body weight ratio of the control and VEGF group (shown are mean ± SE).

**FIG. 20B** is a graph showing kidney/body weight ratio of the control and VEGF group (shown are mean ± SE).

**FIG. 20C** is a graph showing spleen/body weight ratio of the control and VEGF group (shown are mean ± SE).

**FIG. 20D** is a graph showing heart/body weight ratio of the control and VEGF group (shown are mean ± SE).

**FIG. 21A** is a graph showing parenchymal volume/body weight ratio of the control and VEGF group (shown are mean ± SE).

**FIG. 21B** is a graph showing alveolar volume/body weight ratio of the control and VEGF group (shown are mean ± SE).

**FIG. 21C** is a graph showing septal surface area/body weight ratio of the control and VEGF group (shown are mean ± SE).

**FIG. 21D** is a graph showing alveolar count of the control and VEGF group (shown are mean ± SE).

**FIG. 22A** is a graph showing double reciprocal plots (1/OD vs 1/[$VEGF_{165}$]) illustrating the binding between $VEGF_{165}$ and VEGFR1 in different concentrations of heparin (Kd values are expressed as mean ± SE).

**FIG. 22B** is a graph showing double reciprocal plots (1/OD vs 1/[$VEGF_{165}$]) illustrating the binding between $VEGF_{165}$ and VEGFR2 in different concentrations of heparin (Kd values are expressed as mean ± SE).

**FIG. 23A** shows a western blot image (left) and a bar graph showing relative intensity (right) of phosphorylated VEGFR2 harvested from $VEGF_{165}$-stimulated HMVEC-L (human lung microvascular endothelial cells) under different concentrations of heparin. HMVEC-L were expanded on plastic plates.

**FIG. 23B** shows a western blot (left) and a bar graph showing relative intensity (right) of phosphorylated VEGFR2 harvested from $VEGF_{165}$-stimulated HMVEC-L under different concentrations of heparin. HMVEC-L were expanded on Matrigel®-coated plates.

**FIG. 23C** is a bar graph showing HMVEC-L viability as measured by a tetrazolium assay after 3 days of incubation with $VEGF_{165}$ and different concentrations of heparin. Cells were expanded on plastic plates.

**FIG. 23D** is a bar graph showing HMVEC-L viability as measured by a tetrazolium assay after 3 days of incubation with $VEGF_{165}$ and different concentrations of heparin. Cells were expanded on Matrigel®-coated plates.

**FIG. 24A** is a graph showing comparison of lung volume among the 4 experimental groups. Lungs were harvested on POD 4 and volume was measured by the water displacement method and normalized for body weight.

**FIG. 24B** is a graph showing comparison of other liver (left) and kidney (right) mass among the 4 experimental groups. Organs were harvested on POD 4, weighed, and normalized for body weight.

**FIG. 24C** is a graph showing comparison of pulmonary functions among the 4 experimental groups. Pulmonary function tests were done in vivo on POD 4 and normalized for body weight.

**FIG. 25A** is a graph showing parenchymal volume among the 4 experimental groups.

**FIG. 25B** is a graph showing alveolar volume among the 4 experimental groups.

**FIG. 25C** is a graph showing septal surface area among the 4 experimental groups.

**FIG. 25D** is a graph showing septal volume among the 4 experimental groups.

**FIG. 25E** is a graph showing mean septal thickness among the 4 experimental groups.

**FIG. 25F** is a graph showing alveolar density among the 4 experimental groups.

**FIG. 26A** shows western blot results and measured relative intensities of tissue VEGF, P-Y1175- and P-Y1214-VEGFR2 between the heparin and control group (five biological replicates were used for each group).

**FIG. 26B** is a graph showing the results of an assay comparing heparanase activity between the heparin and control group (five biological replicates were used for each group).

**FIG. 27** is a graph illustrating relative mRNA levels of VEGFR2 signaling genes in the heparin group compared to the control group (three biological variates were used for each group).

**FIG. 28A** is a graph showing quantified VEGF levels measured by enzyme-linked immunosorbent assay (ELIZA) for mice treated with VEGF or saline (Control) administered either by nasal instillation or by intraperitoneal injection (shown are mean ± SE).

**FIG. 28B** is a graph showing quantified post-euthanasia lung volume on POD 4 and POD 10 (shown are mean ± SE).

**FIG. 28C** is a graph showing quantified total lung capacity of mice on POD 4 treated with VEGF or saline (Control) administered by nasal instillation (shown are mean ± SE).

**FIG. 28D** is a graph showing quantified pulmonary compliance of mice on POD 4 treated with VEGF or saline (Control) administered by nasal instillation (shown are mean ± SE).

**FIG. 28E** is a graph showing results of post-treadmill rest time for mice on POD 10 treated with VEGF or saline (Control) administered by nasal instillation (shown are mean ± SE).

**FIG. 28F** is a graph showing results of walking distance for mice on POD 10 treated with VEGF or saline (Control) administered by nasal instillation (shown are mean ± SE).

**FIG. 28G** is a graph showing results of basic movement count for mice on POD 10 treated with VEGF or saline (Control) administered by nasal instillation (shown are mean ± SE).

**FIG. 28H** is a graph showing results of fine movement count for mice on POD 10 treated with VEGF or saline (Control) administered by nasal instillation (shown are mean ± SE).

**FIG. 29A** is a graph showing results of lung parenchymal volume for mice treated with VEGF or saline (Control) (shown are mean ± SE).

**FIG. 29B** is a graph showing results of alveolar volume for mice treated with VEGF or saline (Control) (shown are mean ± SE).

**FIG. 29C** is a graph showing results of septal surface area for mice treated with VEGF or saline (Control) (shown are mean ± SE).

**FIG. 29D** is a graph showing results of mean septal thickness for mice treated with VEGF or saline (Control) (shown are mean ± SE).

**FIG. 29E** is a graph showing results of total alveolar count for mice treated with VEGF or saline (Control) (shown are mean ± SE).

**FIG. 30A** are images of immune-stained lung endothelial cells in POD 2 samples from mice treated with VEGF or saline (Control) administered by nasal instillation. Cells are labeled with anti-ERG antibody and proliferating cells are marked with anti-Ki67 antibody. Double-stained Ki67-positive endothelial cells are marked by arrows. Representative sections of control and VEGF-treated lungs shown at 20X magnification.

**FIG. 30B** are images of immune-stained lung endothelial cells in POD 4 samples from mice treated with VEGF or saline (Control) administered by nasal instillation. Cells are labeled with anti-ERG antibody and proliferating cells are marked with anti-Ki67 antibody. Double-stained Ki67-positive endothelial cells are marked by arrows. Representative sections of control and VEGF-treated lungs shown at 20X magnification.

**FIG. 30C** is a graph showing results of endothelial proliferation measured in POD 2 and POD 4 samples from mice treated with VEGF or saline (Control) administered by nasal instillation (shown are mean ± SE).

**FIG. 31A** is a graph of results from protein expression analyses measured in POD 4 samples from mice treated with VEGF or saline (Control) administered by nasal instillation (shown are mean ± SE).

**FIG. 31B** is a graph of results from gene expression analyses measured in POD 4 samples from mice treated with VEGF or saline (Control) administered by nasal instillation (shown are mean ± SE).

**FIG. 31C** are western blots showing levels of P-VEGFR2, VEGFR2, P-EGFR, EGFR, and heparin-binding EGF-like growth factor (HB-EGF) on POD 4 measured in samples from mice treated with VEGF or saline (Control) administered by nasal instillation (shown are mean ± SE).

**FIG. 31D** is a graph of protein levels quantified from western blots (shown are mean ± SE).

**FIGS. 32A-32C** show protein content results from assays performed with human lung microvascular endothelial cells (HMVEC-L).

**FIG. 32D** shows cell density of HBEC cells over time in presence or absence of VEGF165; VEGF165 is labeled as "VEGF."

**FIG. 32E** shows a cartoon representation of the HMVEC2 and HVEC co-culture system. The insert for the dish contained a porous membrane that restricted movement of HMVEC-2 cells, but did not restrict diffusion of VEG165 that was initially placed in medium held by the insert.

**FIG. 32F** shows cell density of HBEC cells over time in presence or absence of HMVEC-L cells treated or not treated with VEGF165; VEGF 165 is labeled as "VEGF."

**FIG. 32G** shows a cartoon representation of HMVEC-L and HBEC co-culture competition assays performed with differing amounts of VEG165 present in the medium located in the insert and with different amounts of HB-EGF neutralizing antibody (HB-EGF antibody) present in the medium of the well.

**FIG. 32H** shows the quantified results of the HMVEC-L and HBEC co-culture competition assays depicted in FIG 32G.

**FIG. 32I** shows the western blot results of the HMVEC-L and HBEC co-culture competition assays.

**FIG. 32J** shows the quantified western blot analysis of the HMVEC-L and HBEC co-culture competition assays.

**FIG. 33** lists the sequences for VEGF-A, PGF, VEGF-B, VEGF-C, VEGF-D, and several isoforms of VEGF-A.

## DETAILED DESCRIPTION

Normal lung development depends on vascular endothelial growth factor (VEGF), the absence of which results in decreased lung maturation marked by alveolar and vascular hypoplasia. Therapies to accelerate lung development in the postnatal period to reduce mortality and morbidity from pulmonary hypoplasia in patients with congenital diaphragmatic hernia are needed. This disclosure relates to compositions for use in methods of promoting lung development in a subject. The methods involve administering to the subject an effective amount of a composition comprising VEGF.

Angiogenesis is important in both pathological and physiological processes, e.g., wound healing and embryonic organ development. It has also been well-established that tumor tissue growth depends on angiogenesis. Tumors cannot survive or grow without adequate nutrients and oxygen, which are made available by blood vessels. Without appropriate sustenance, tumor cells die via apoptosis or necrosis. Tumors secrete various angiogenic factors to recruit new blood vessels, a process that is critical for tumor growth. The physiological equivalents to tumor growth are *de novo* tissue growth (embryogenesis) and tissue regeneration. Angiogenesis is a critical factor in these processes as well. This disclosure suggests a significant role for angiogenesis in the growth of the lung.

Vascular endothelial growth factor (VEGF) is a proangiogenic factor which is endogenously produced and is important in the normal vascular development of numerous organ systems. VEGF proteins are deposited in the subepithelial matrix at the leading edges of branching airways where it stimulates angiogenesis. In the pulmonary system, angiogenesis is of particular importance because the blood-air interface is the sole source of oxygen delivery to the body. This disclosure has shown that administering VEGF to a subject can promote lung growth and improve pulmonary functions, thus can be used to treat various lung diseases.

## Lung Disorders

VEGF is of particular importance to the pulmonary system, because VEGF is a proangiogenic factor and the blood-air interface in the lung is formed, in part, by the endothelial cells of the capillaries. As shown in the present disclosure, VEGF can be used to promote lung growth and improve pulmonary functions. VEGF can be used to treat various lung diseases.

Diseases that can be treated using the compositions for use in methods described herein include, e.g., pulmonary hypoplasia, congenital diaphragmatic hernia (CDH), Poland syndrome, Chest wall diseases (e.g., pectus excavatum and constricting chest wall disorder), pneumonectomy, bronchopulmonary dysplasia (BPD), pulmonary hypertension, lung injury, inhalation injury (e.g., smoke inhalation), and Moyamoya.

CDH is a birth defect of the diaphragm. It is a life-threatening pathology in infants and a major cause of death. It affects 1 in 3000-5000 live births and continues to carry a mortality of 20-30% in spite of advances in neonatal critical and surgical care. The abdominal organs presence in the chest cavity causes the lungs to be severely undersized, especially on the side of the hernia, leading to pulmonary hypoplasia and frequently pulmonary hypertension. Infants born with diaphragmatic hernia experience respiratory failure due to both pulmonary hypertension and pulmonary hypoplasia. Mortality can be as high as 60% in those infants with large diaphragmatic defects and approaches 90% in infants with severe pulmonary hypoplasia due to a combination of pulmonary hypertension and refractory hypoxia. Development of therapies to accelerate lung development and maturation in the postnatal period as a means to reduce the mortality and morbidity associated with prolonged ventilation and extracorporeal membrane oxygenation (ECMO) would be useful.

Pulmonary hypoplasia is incomplete development of the lungs. It is often characterized by an abnormally low number or size of bronchopulmonary segments or alveoli. Pulmonary hypoplasia can have many causes, e.g., congenital diaphragmatic hernia (CDH), congenital cystic adenomatoid malformation, fetal hydronephrosis, caudal regression syndrome, mediastinal tumor, sacrococcygeal teratoma, fetal hydrops, oligohydramnios, and dextrocardia of embryonic arrest. Large masses of the neck (e.g., cervical teratoma) can also cause pulmonary hypoplasia. Pulmonary hypoplasia is often measured by percent-predicted lung volume (PPLV). Usually, severe pulmonary hypoplasia (PPLV<15%) predicts poor prognosis. The mortality rate for neonates with severe pulmonary hypoplasia can be as high as 60%.

Poland syndrome is a rare birth defect characterized by underdevelopment or absence of the chest muscle. Pulmonary hypoplasia sometimes is a complication of Poland syndrome.

Pectus excavatum is a congenital deformity of the anterior thoracic wall in which the sternum and rib cage grow abnormally.

Bronchopulmonary dysplasia is a serious lung condition that affects infants. It usually involves abnormal development of lung tissue. It is also one of the most common chronic lung diseases in children. The lung is characterized by fibrosis and fewer numbers of alveolar sacs of larger size.

Pneumonectomy refers to a surgical procedure of removing a lung. Removal of one lobe of the lung is specifically referred to as a lobectomy, and removal of a segment of the lung is referred to as a wedge resection. The most common reason for performing pneumonectomy is to remove tumorous tissue in the lung. Following removal of the lung (e.g., left lung) or part of the lung (e.g., a lobe), the remaining lung will grow beyond its original size to compensate for that loss of lung mass. Compensatory lung growth is often measured as a function of lung (volume and mass) to weight ratio. The therapeutic agents described herein can be used to accelerate lung growth.

Similarly, lung injury and inhalation injury (e.g., smoke inhalation), in some cases, also require lung growth. Thus, administering therapeutic agents as described herein can be used to treat subjects with lung injury or inhalation injury.

Moyamoya is a disease in which certain arteries in the brain are constricted. Many patients with Moyamoya disease have loss of vasculature in the lungs. Thus, the therapeutic agents as described in this disclosure can be used to treat or alleviate symptoms of patients with Moyamoya disease.

The therapeutic agents described herein can be used to treat many other lung diseases. Lung diseases characterized by insufficient, incomplete, or slow lung development can be treated using the compositions for use in methods described herein. Further, the compositions can be used to treat conditions that can benefit from lung growth or accelerated lung growth.

## VEGF

Treatments describe herein involve administering VEGF to a patient. VEGF is a key regulator of physiological angiogenesis during embryogenesis, skeletal growth and reproductive functions. VEGF has also been implicated in pathological angiogenesis associated with tumors, intraocular neovascular disorders and other conditions. The biological effects of VEGF are mediated by two receptor tyrosine kinases (RTKs), VEGFR-1 and VEGFR-2, which differ considerably in signaling properties. Non-signaling co-receptors also modulate VEGF RTK signaling. A detailed description of VEGF and its receptors can be found, e.g., in Ferrara, Napoleone, Hans-Peter Gerber, and Jennifer LeCouter. "The biology of VEGF and its receptors." Nature medicine 9.6 (2003): 669-676.

The VEGF family comprises five members: VEGF-A (SEQ ID NO: 3), placenta growth factor (PGF) (SEQ ID NO: 4), VEGF-B (SEQ ID NO: 5), VEGF-C (SEQ ID NO: 6), and VEGF-D (SEQ ID NO: 7).

VEGF-A is a protein is encoded by the *VEGFA* gene. There are multiple isoforms of VEGF-A, e.g., VEGF-164 (mouse) (SEQ ID NO:1), VEGF-165 (human) (SEQ ID NO:2), rhVEGF-165 (SEQ ID NO: 23), VEGF189 (human) (SEQ ID NO: 8), VEGF183 (human) (SEQ ID NO: 9), VEGF148 (human) (SEQ ID NO: 10), VEGF145 (Human)(SEQ ID NO: 11), VEGF165B (Human)(SEQ ID NO: 12), VEGF121 (Human)(SEQ ID NO: 13), VEGF111 (Human)(SEQ ID NO: 14), L-VEGF165 (Human)(SEQ ID NO: 15), L-VEGF121 (Human)(SEQ ID NO: 16), L-VEGF189 (Human)(SEQ ID NO: 17), L-VEGF206 (Human)(SEQ ID NO: 18), VEGFA Isoform 15 (Human)(SEQ ID NO: 19), VEGFA Isoform 16 (Human)(SEQ ID NO: 20), VEGFA Isoform 17 (Human)(SEQ ID NO: 21), and VEGFA Isoform 18 (Human)(SEQ ID NO: 22). Many of these isoforms result from alternative splicing. The alternate splicing often occurs at exon 6 and 7, and alters the heparin-binding affinity and amino acid number. The three major isoforms of the VEGF-A family, $VEGF_{121}$, $_{165}$, $_{and\ 189}$, differ from each other mainly by the presence of HBDs encoded by exon 6 and 7 (K. A. Houck, D. W. Leung, A. M. Rowland, J. Winer, and N. Ferrara, "Dual regulation of vascular endothelial growth factor bioavailability by genetic and proteolytic mechanisms.," J. Biol. Chem., vol. 267, no. 36, pp. 26031-7, Dec. 1992). While $VEGF_{121}$ does not possess any HBD, $VEGF_{165}$ contains one HBD coded by exon 7 and $VEGF_{189}$ carries two HBDs that are present in both exon 6a and 7 (D. Krilleke, et al., "The heparin-binding domain confers diverse functions of VEGF-A in development and disease: a structure-function study.," Biochem. Soc. Trans., vol. 37, no. Pt 6, pp. 1201-6, Dec. 2009). The presence of HBD largely determines the distribution of each isoform between plasma and tissue, with higher numbers indicating stronger affinity for the ECM. It is interesting to note that $VEGF_{121}$ and $_{165}$ have equal mitogenic activity on endothelial cells *in vitro.* Mice that are engineered to express only $VEGF_{120}$ display defects in blood vessel sprouting, capillary morphology, and complexity of the vascular system as a result of VEGF's failure to localize into the ECM. On the other hand, *Vegf*$^{164/164}$ mice demonstrate normal retinal angiogenesis *in vivo.* In some embodiments, VEGF is VEGF165 or VEGF121.

VEGF-B is encoded by the *VEGF-B* gene and plays an important role in the maintenance of newly formed blood vessels during pathological conditions.

VEGF-C is encoded by the *VEGF-C* gene and is involved in lymphangiogenesis.

VEGF-D is encoded by *FIGF* gene and is involved in angiogenesis, lymphangiogenesis, and endothelial cell growth.

PGF is encoded by *PGF* gene, and is important for vasculogenesis. It is also needed for angiogenesis during ischemia, inflammation, wound healing, and cancer.

VEGF stimulates cellular responses by binding to tyrosine kinase receptors (the VEGFRs) on the cell surface, causing them to dimerize and become activated through transphosphorylation. VEGF-A binds to VEGFR-1 (Flt-1) and VEGFR-2 (KDR/Flk-1), and it usually does not bind or activate VEGFR-3 (Flt-4). VEGF-B exerts its effects via the FLT1 receptor. VEGF-C and VEGF-D, but not VEGF-A, are ligands for a third receptor (VEGFR-3/Flt4), which mediates lymphangiogenesis. VEGF-C can stimulate lymphangiogenesis (via VEGFR3) and angiogenesis via VEGFR2.

Accordingly, a therapeutic agent for treating various lung diseases can comprise, consist of, or consist essentially of VEGF-A, PGF, VEGF-B, VEGF-C, or VEGF-D, or any isoforms thereof (e.g., VEGF-164 (mouse) (SEQ ID NO:1), VEGF-165 (human) (SEQ ID NO:2), rhVEGF-165 (SEQ ID NO: 23), VEGF189 (human) (SEQ ID NO: 8), VEGF183 (human) (SEQ ID NO: 9), VEGF148 (human) (SEQ ID NO: 10), VEGF145 (Human)(SEQ ID NO: 11), VEGF165B (Human)(SEQ ID NO: 12), VEGF121 (Human)(SEQ ID NO: 13), VEGF111 (Human)(SEQ ID NO: 14), L-VEGF165 (Human)(SEQ ID NO: 15), L-VEGF121 (Human)(SEQ ID NO: 16), L-VEGF189 (Human)(SEQ ID NO: 17), L-VEGF206 (Human)(SEQ ID NO: 18), VEGFA Isoform 15 (Human)(SEQ ID NO: 19), VEGFA Isoform 16 (Human)(SEQ ID NO: 20), VEGFA Isoform 17 (Human)(SEQ ID NO: 21), and VEGFA Isoform 18 (Human)(SEQ ID NO: 22)), or any combination thereof.

The therapeutic agent can be, e.g., a peptide having a sequence that is at least 50%, 60%, 70%, 80%, 90%, 95%, 96%, 97%, 98%, or at least 99% identical to VEGF-A, PGF, VEGF-B, VEGF-C, and VEGF-D, or any isoforms thereof (e.g., VEGF-164 (mouse) (SEQ ID NO:1), VEGF-165 (human) (SEQ ID NO:2), rhVEGF-165 (SEQ ID NO: 23), VEGF189 (human) (SEQ ID NO: 8), VEGF183 (human) (SEQ ID NO: 9), VEGF148 (human) (SEQ ID NO: 10), VEGF145 (Human)(SEQ ID NO: 11), VEGF165B (Human)(SEQ ID NO: 12), VEGF121 (Human)(SEQ ID NO: 13), VEGF111 (Human)(SEQ ID NO: 14), L-VEGF165 (Human)(SEQ ID NO: 15), L-VEGF121 (Human)(SEQ ID NO: 16), L-VEGF189 (Human)(SEQ ID NO: 17), L-VEGF206 (Human)(SEQ ID NO: 18), VEGFA Isoform 15 (Human)(SEQ ID NO: 19), VEGFA Isoform 16 (Human)(SEQ ID NO: 20), VEGFA Isoform 17 (Human)(SEQ ID NO: 21), and VEGFA Isoform 18 (Human)(SEQ ID NO: 22)). In some embodiments, the peptide can have a sequence comprising, consisting of, or consisting essentially of any sequences that are described in the present disclosure.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). The length of a reference sequence aligned for comparison purposes is at least 50% of the length of the reference sequence, and in some embodiments is at least 60%, 70%, 80%, 90%, 95%, or 100%. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences. For purposes of the present invention, the comparison of sequences and determination of percent identity between two sequences can be accomplished using a Blossum 62 scoring matrix with a gap penalty of 12, a gap extend penalty of 4, and a frameshift gap penalty of 5.

**Heparin-binding EGF-like growth factor**

Heparin-binding EGF-like growth factor (HB-EGF) is a member of the EGF family. HB-EGF is a ligand for EGFR and its upregulation has been shown as a key event in the tissue repair and regeneration of many organs ranging from the skin and cornea to liver and gastrointestinal tract. It is synthesized as a 208 amino acid (aa) transmembrane protein called proHB-EGF, which undergoes further proteolytic cleavage to release the mature, soluble HB-EGF, a process called ectodomain shedding. The concurrent increase in multiple proteases, such as cathepsin B and MMP-7 in VEGF-treated HMVEC-L further supports the occurrence of ectodomain shedding as a mechanism for HB-EGF upregulation in this study. HB-EGF shedding is a key event after activation of VEGFR2 and inhibition of this process significantly impaired CLG.

HB-EGF typically includes a 19 amino acid (aa) signal sequence, a 43 aa prosegment, an 86 aa mature region (aa 63-148), an 11 aa juxtamembrane cleavage peptide, a 24 aa transmembrane segment, and a 25 aa cytoplasmic tail (aa 184-208). Mature HB-EGF is a soluble peptide that arises from proteolytic processing of the transmembrane form. It possesses an EGF-like domain between aa 104-144, and a heparin-binding motif between aa 93-113. Although the aa range for "mature" HB-EGF is typically stated to be Asp63-Leu148, potential N-terminal start (cleavage) sites also exist at Gly32, Arg73, Val74, Ser77 and Ala82.

This disclosure shows HB-EGF may play a key mechanistic role in mediating the effects of VEGF on CLG given its upregulation with VEGF treatment and mitogenic properties on lung epithelial cells. Thus, a therapeutic agent for treating various lung diseases can comprise, consist of, or consist essentially of proHB-EGF or soluble HB-EGF or any combination thereof. The therapeutic agent can be, e.g., a peptide having a sequence that is at least 50%, 60%, 70%, 80%, 90%, 95%, 96%, 97%, 98%, or at least 99% identical to proHB-EGF (NP_001936.1; SEQ ID NO: 24) or soluble HB-EGF. In some embodiments, the soluble HB-EGF has amino acids 63-148 of SEQ ID NO:24, amino acids 32-148 of SEQ ID NO:24, amino acids 73-148 of SEQ ID NO:24, amino acids 74-148 of SEQ ID NO:24, amino acids 77-148 of SEQ ID NO:24, or amino acids 82-148 of SEQ ID NO:24.

**Subjects**

The terms "subject" and "patient" are used interchangeably throughout the specification and describe an animal, human or non-human, to whom treatment according to the compositions for use in methods of the present invention is provided. Veterinary and non-veterinary applications are contemplated by the present invention.

A human patient can be an adult or a child, such as a newborn child. For example, the human subject can be at least or about 60 years old, e.g., at least about 65 years old, 70 years old, 75 years old, or at least or about 80 years old. The human subject can be below or about the age of 18 years old, e.g., 15 years old, 10 years old, 9 years old, 8 years old, 7 years old, 6 years old, 5 years old, 4 years old, 3 years old, 2 years old, or below or about 1 year old. The human subject can be a newborn or an infant, for example, the subject can be a premature baby, or a baby born at about normal term, e.g., a 0-1 month old baby, a 1-3 month old baby, a 3-6 month old baby, a 6-9 month old baby, or a 9-12 month old baby.

In addition to humans, subjects can include, but are not limited, non-humans, e.g., mice, rats, hamsters, guinea-pigs, rabbits, ferrets, cats, dogs, and primates. Included are, for example, non-human primates (e.g., monkey, chimpanzee, gorilla, and the like), rodents (e.g., rats, mice, gerbils, hamsters, ferrets, rabbits), lagomorphs, swine (e.g., pig, miniature pig), equine, canine, feline, bovine, and other domestic, farm, and zoo animals.

The subject can have any lung disease that may benefit from the treatment, e.g., a disease described herein, e.g., pulmonary hypoplasia, congenital diaphragmatic hernia (CDH), Poland syndrome, Chest wall diseases (e.g., pectus excavatum), pneumonectomy, bronchopulmonary dysplasia, lung injury, and inhalation injury, etc. For example, the subject may have pulmonary hypoplasia. The percent-predicted lung volume (PPLV) in the subject can be less than or about 50%, e.g., less than or about 45%, 40%, 35%, 30%, 25%, 20%, 15%, or less than or about 10%. In certain cases, the subject is a neonate with severe pulmonary hypoplasia (e.g., percent-predicted lung volume (PPLV) $< 15\%$).

The subject may be identified as having insufficient, incomplete, or slow lung development. The subject can be identified as a subject that can benefit from lung growth or accelerated lung growth. Alternatively or in addition, the subject may have some damage in the lungs. For example, the subject can be a habitual smoker or a worker frequently exposed to smoke inhalation.

## Compositions for use in methods of Treatment

The compositions described herein include methods for the treatment of various lung diseases. Generally, the methods include administering a therapeutically effective amount of a therapeutic agent comprising VEGF (e.g., VEGF-165) as described herein and/or HB-EGF (e.g., proHB-EGF or soluble HB-EGF) as described herein, to a subject who is in need of, or who has been determined to be in need of, such treatment.

As used in this context, to "treat" means to ameliorate at least one symptom of the disorder (e.g., shortness of breath, fast breathing, a low oxygen level in the blood, heart failure (e.g., right heart failure)). In some embodiments, the treatment results in lung growth or lung regeneration. In some cases, the treatment can result in an increase of lung volume to body weight ratio, parenchymal volume, alveolar volume, septal surface area, alveolar density, and/or alveolar number. Thus, administration of an effective amount of a therapeutic agent described herein can also improve pulmonary functions. Furthermore, the lungs in VEGF-treated subjects are histologically indistinguishable from saline-treated subjects, suggesting that normal pulmonary architecture can be preserved.

Treatment may also result in an increase of the expression level of EGF, and an increase of the amount of VEGFR2 and/or phosphorylated VEGFR2 (P-VEGFR2) in the lung.

Administering the therapeutic agent as described herein (e.g., VEGF or HB-EGF) can be accomplished by various means, for example, intravenous, intradermal, subcutaneous, transdermal (topical), transmucosal, rectal administration. nasal administration, nasal instillation, insufflation (e.g., nasal sprays), inhalation (through nose or mouth), intrapulmonary administration, intratracheal administration, intraperitoneal injection, infusion, or any combinations thereof.

However, systemic administration of VEGF or HB-EGF may result in undesirable side effects if used in humans. Past clinical studies of the potential therapeutic uses of VEGF in critical limb ischemia and coronary artery disease noted side effects such as hypotension and decreased cardiac stroke volume. In addition, VEGF promotes vascular permeability and elevated levels of VEGF have been associated with skeletal muscle and macular edema. Systemic VEGF administration may also exacerbate pathogenic angiogenesis, such as that seen in diabetic retinopathy and cancer metastasis. Given the possible adverse systemic effects, the ability to locally administer VEGF for potential therapeutic purposes would therefore be advantageous. Among these routes of administrations, nasal delivery offers a less invasive and more specific alternative to systemic administration, reducing opportunities for the development of side effects. When instilled intranasally, 75% of dye deposits to the airways, with no detectable amounts in non-targeted areas, such as the stomach or esophagus. The present disclosure shows that topical administration of exogenous murine VEGF through nasal instillation is a comparable alternative to systemic VEGF administration.

In one aspect, the present disclosure provides compositions for use in methods of promoting lung growth in a subject. The methods include administering to the subject an effective amount of a composition comprising VEGF and/or HB-EGF. In some embodiments, the composition is administered through respiratory tract. In some embodiments, the subject is a newborn, a child, or an adult. In some embodiments, the composition comprises VEGF. In some embodiments, the composition comprises HB-EGF (e.g., soluble HB-EGF).

The disclosure also provides other compositions for use in methods of promoting lung growth in a subject. The methods include administering to the subject an effective amount of a first composition comprising VEGF and/or HB-EGF through systematic administration (e.g., intravenous infusion); and then (e.g., after a pharmaceutical effect on the lungs is detected), administering to the subject an effective amount of a second composition comprising VEGF and/or HB-EGF through respiratory tract (e.g., nasal instillation, or inhalation). In some embodiments, the first composition and the second composition both comprise VEGF. In some embodiments, the first composition and the second composition both comprise HB-EGF (e.g., soluble HB-EGF). In some embodiments, the first composition comprises VEGF and the second composition comprises HB-EGF. In some embodiments, the first composition comprises HB-EGF and the second composition comprises VEGF.

Because of the occasionally limited functionally for the lungs that are in need of treatment, a therapeutic agent sometimes cannot be effectively delivered to the target sites in the lungs (e.g., bronchioles or alveoli) through respiratory tract administration. An agent that can clear the airways can be administered to the subject first. In some embodiments, these agents can induce dilation of bronchial passages, and/or vasodilation in muscle and liver. Such agents include, but are not limited to, beta2 adrenergic receptor agonists, anticholinergic agents, corticosteroids. In some embodiments, an agent for treating asthma can be used.

## Pharmaceutical Compositions

The pharmaceutical compositions described herein for use in methods comprise VEGF and/or HB-EGF (e.g., proHB-EGF or soluble HB-EGF) as an active ingredient. Pharmaceutical compositions can include, e.g., a pharmaceutically acceptable carrier. As used herein the language "pharmaceutically acceptable carrier" includes saline, solvents, dispersion media, coatings, antibacterial and antifungal agents, isotonic and absorption delaying agents, and the like, compatible with pharmaceutical administration. Also provided herein are pharmaceutical compositions comprising VEGF and/or HB-EGF. In some embodiments, the pharmaceutical compositions comprise the combination of both VEGF and HB-EGF.

Pharmaceutical compositions can be formulated to be compatible with its intended route of administration. Examples of routes of administration include parenteral, e.g., intravenous, intradermal, subcutaneous, oral (e.g., inhalation), transdermal (topical), transmucosal, and rectal administration.

Methods of formulating suitable pharmaceutical compositions are known in the art, see, e.g., Remington: The Science and Practice of Pharmacy, 21st ed., 2005; and the books in the series Drugs and the Pharmaceutical Sciences: a Series of Textbooks and Monographs (Dekker, NY). For example, solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent that delays absorption, for example, aluminum monostearate and gelatin.

Sterile injectable solutions can be prepared by incorporating the active agent in the required amount in an appropriate solvent with one or a combination of ingredients enumerated above, as required, followed by filtered sterilization. Generally, dispersions are prepared by incorporating the active agent into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yield a powder of the active ingredient plus any additional desired ingredient from a previously sterile-filtered solution thereof.

In some instances, systemic administration of a therapeutic agent described herein can be by transmucosal means. For transmucosal administration, penetrants appropriate to the barrier to be permeated are used in the formulation. Such penetrants are generally known in the art, and can include, for example, detergents, bile salts, and fusidic acid derivatives. Transmucosal administration can be accomplished through the use of nasal sprays or suppositories.

In some instances, the therapeutic agents are prepared with carriers that will protect the therapeutic agent against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques, or obtained commercially, e.g., from Alza Corporation and Nova Pharmaceuticals, Inc. Liposomal suspensions (including liposomes targeted to selected cells with monoclonal antibodies to cellular antigens) can also be used as pharmaceutically acceptable carriers. These can be prepared according to methods known to those skilled in the art, for example, as described in U.S. Patent No. 4,522,811.

When administered through certain routes, such as the respiratory tract (e.g., nose, a tracheostomy, or by endotracheal tube (ETT)), surfactants can allow the composition to reach the distal airways. Thus, in some embodiments, compositions described herein (e.g., liquid solutions, aqueous solutions (where water-soluble), aerosol formulations, dispersions, sterile powders etc.) can comprise one or more surfactants.

The surfactant can be selected from the group consisting of non-ionic, cationic, anionic, and zwitterionic surfactants. Mixtures of surfactants can also be used. Exemplary classes of surfactants include alcohol ether sulfates, alcohol sulfates, alkanolamides, alkyl sulfonates, amine oxides, amphoteric surfactants, anionic surfactants, betaine derivatives, cationic surfactants, disulfonates, dodecylbenzene, sulfonic acid, ethoxylated alcohols, ethoxylated alkyl phenols, ethoxylated fatty acids, glycerol esters hydrotropes, lauryl sulfates, mono and diglycerides, non-ionic surfactants, phosphate esters, quaternary surfactants, and sorbitan derivatives.

To be suitable for delivery through respiratory tract, appropriate pharmaceutical carriers can also be used. These carries include e.g., liposomes, nano- and microparticles, cyclodextrins, microemulsions, micelles, suspensions, or solutions. In some embodiments, these carriers are made of lipids (e.g., liposomes, niosomes, microemulsions, lipidic micelles, solid lipid nanoparticles) or composed of polymers (e.g., polymer micelles, dendrimers, polymeric nanoparticles, nonogels, nanocapsules). These carriers can carry the therapeutic agent to the lung in a controlled manner from the site of administration to the therapeutic target. Nanocarriers can also be used. In general, nanocarriers aim to minimize drug degradation and loss, prevent harmful side effects and increase the availability of the drug at the disease site. Localized therapy of the target organ generally requires smaller total doses to achieve clinically effective results. To reach this goal, nanocarriers can be engineered to slowly degrade, react to stimuli and to be site specific.

Pharmaceutical compositions described herein can be included in a container, pack, or dispenser together with instructions for administration.

## Administration through Respiratory Tract

As VEGF is a key mediator of angiogenesis in cancer, systematic administration of VEGF may cause undesirable consequences (e.g., cause cancer). In some cases, directly administering VEGF to the target region can reduce or minimize undesirable effects. However, although the alveolar epithelium and capillary endothelium have high permeability to water, gases and lipophilic substances, the permeation of hydrophilic substances of large molecular size (e.g., a protein with more than 100 amino acid residues) is limited (See Sayani, Amyn P., and Yie W. Chien. "Systemic delivery of peptides and proteins across absorptive mucosae." Critical reviews in therapeutic drug carrier systems 13.1-2 (1995): 85-184). Particularly, the respiratory mucus, alveolar lining layer, alveolar epithelium, basement membrane all create barriers to pulmonary absorption of the protein (See Niven, Ralph W. "Delivery of biotherapeutics by inhalation aerosol." Critical Reviews™ in Therapeutic Drug Carrier Systems 12.2-3 (1995)).

The mucus (1-10 μm thick) that lines the pulmonary epithelium and the surfactant that lines the alveoli (0.1-0.2 μm thick) constitute physical barriers to pulmonary absorption of peptides and proteins. Furthermore, membrane-associated (epithelial and endothelial) and intracellular (macrophages, lymphocytes, neutrophils and mast cells) proteases and peptidases readily degrade administered peptides and proteins (Agu, Remigius Uchenna, et al. "The lung as a route for systemic delivery of therapeutic proteins and peptides." Respiratory research 2.4 (2001): 198). The pulmonary macrophages have also been shown to secrete or release short-lived peroxidases, inflammatory and immunomodulatory mediators (including granulocyte colony-stimulating factor, interleukins, leukotrienes and proteases), and other molecules as part of a host defense mechanism. These molecules are able to degrade peptides and proteins. Sayani, Amyn P., and Yie W. Chien. "Systemic delivery of peptides and proteins across absorptive mucosae." Critical reviews in therapeutic drug carrier systems 13.1-2 (1995): 85-184).

Nevertheless, the present disclosure shows that administering VEGF through the respiratory tract can effectively promote lung growth. Furthermore, as administering VEGF through the respiratory tract is a targeted delivery approach, in some cases, it only requires a smaller dose of VEGF as compared to administering VEGF by intraperitoneal injection or IV administration. In addition, administering VEGF through the respiratory tract can also minimize the systemic side effects of administering VEGF to a subject, e.g., edema and hypotension.

Administering the therapeutic agent as described herein (e.g., VEGF or HB-EGF) through respiratory tract can be accomplished by various means, for example, nasal administration, nasal instillation, insufflation (e.g., nasal sprays), inhalation (through nose or mouth), intrapulmonary administration, intratracheal administration, or any combinations thereof. As used herein, the term "nasal instillation" refers to a procedure that delivers a therapeutic agent directly into the nose and onto the nasal membranes, wherein a portion of the therapeutic agent can pass through tracheas and is delivered into the lung.

Pharmaceutical compositions suitable for administering through respiratory tract can include, e.g., liquid solutions, aqueous solutions (where water soluble), dispersions, or sterile powders, etc. In some embodiments, these compositions can comprise one or more surfactants.

For administration through the respiratory tract, in certain cases, a useful device is a small, hard bottle to which a metered dose sprayer is attached. The metered dose can be delivered by drawing the composition into a chamber of defined volume, which chamber has an aperture dimensioned to aerosolize and aerosol formulation by forming a spray when a liquid in the chamber is compressed. The chamber is compressed to administer the composition. In certain devices, the chamber is a piston arrangement. Such devices are commercially available.

Alternatively, a squeeze bottle with an aperture or opening dimensioned to aerosolize an aerosol formulation by forming a spray when squeezed can be used. The opening is usually found in the top of the bottle, and the top is generally tapered to partially fit in the nasal passages for efficient administration of the aerosol formulation. Preferably, the nasal inhaler will provide a metered amount of the aerosol formulation, for administration of a measured dose of the therapeutic agent.

Often, the aerosolization of a liquid or a dry powder formulation for inhalation into the lung will require a propellant. The propellant may be any propellant generally used in the art. Specific non-limiting examples of such useful propellants are a chlorofluorocarbon, a hydrofluorocarbon, a hydrochlorofluorocarbon, or a hydrocarbon, including trifluoromethane, dichlorodifluoromethane, dichlorotetrafluoroethanol, and 1,1,1,2-tetrafluoroethane, or combinations thereof.

In general, the therapeutic agent (e.g., VEGF or HB-EGF) can be introduced into the subject (e.g., in the aerosolized and/or liquid (e.g., with surfactant) form) in an amount between about 0.001 mg per kg body weight of the subject up to about 1 mg per kg body weight of the subject, e.g., from about 0.01 mg/kg to about 0.5 mg/kg, from about 0.01 mg/kg to about 0.4 mg/kg, from about 0.05 mg/kg to about 0.5 mg/kg, from about 0.1 mg/kg to about 0.5 mg/kg, from about 0.1 mg/kg to about 0.3 mg/kg, from about 0.05 mg/kg to about 0.3 mg/kg, or from about 0.05 mg/kg to about 0.2 mg/kg, inclusive. In some embodiments, the dosage is about 0.005 mg/kg, 0.01 mg/kg, about 0.02 mg/kg, about 0.03 mg/kg, about 0.04 mg/kg, about 0.05 mg/kg, about 0.06 mg/kg, about 0.07 mg/kg, about 0.08 mg/kg, about 0.09 mg/kg, about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, or about 0.9 mg/kg, or about 1 mg/kg. In some embodiments, an effective dosage of VEGF and/or HB-EGF is administered to the subject, at about or less than 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, or 1 mg/kg. In some embodiments, the dosage is about or greater than 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, or 1 mg/kg. In certain embodiments, the dosage is administered as needed.

One of ordinary skill in the art can readily determine a volume or weight of aerosol corresponding to this dosage based on the concentration of the therapeutic agent in an aerosol formulation.

The present disclosure also provides aerosol formulations and dosage forms for use in treating subjects with various lung diseases. In general, such dosage forms contain the therapeutic agent in a pharmaceutically acceptable diluent. Pharmaceutically acceptable diluents in such aerosol formulations include but are not limited to sterile water, saline, buffered saline, dextrose solution, and the like. In certain embodiments, a diluent that may be used in the present invention or the pharmaceutical formulation is phosphate buffered saline or a buffered saline solution generally between the pH 7.0-8.0 range (e.g., pH 7.4), or water.

The aerosol formulation also may optionally include pharmaceutically acceptable carriers, diluents, solubilizing or emulsifying agents, surfactants and excipients.

The formulation may include a carrier. The carrier is a macromolecule which is soluble in the circulatory system and which is physiologically acceptable where physiological acceptance means that those of skill in the art would accept injection of said carrier into a patient as part of a therapeutic regime. The carrier preferably is relatively stable in the circulatory system with an acceptable plasma half-life for clearance. Such macromolecules include but are not limited to soy lecithin, oleic acid and sorbitan trioleate.

The formulations can also include other agents useful for pH maintenance, solution stabilization, or for the regulation of osmotic pressure. Examples of the agents include but are not limited to salts, such as sodium chloride, or potassium chloride, and carbohydrates, such as glucose, galactose or mannose, and the like.

The present disclosure further contemplates aerosol formulations comprising the composition as described herein (e.g., VEGF or HB-EGF) and another therapeutically effective agent, e.g., an agent for increasing blood pressure.

It is also contemplated that the present aerosol formulation can be prepared as a dry powder formulation comprising a finely divided powder form of the therapeutic agent and a dispersant. For example, the dry powder formulation can comprise a finely divided dry powder containing the therapeutic agent, a dispersing agent and also a bulking agent. Bulking agents useful in conjunction with the present formulation include such agents as lactose, sorbitol, sucrose, or mannitol, in amounts that facilitate the dispersal of the powder from the device.

Compositions for inhalation or insufflation include solutions and suspensions in pharmaceutically acceptable, aqueous or organic solvents, or mixtures thereof, and powders. The liquid or solid compositions can contain suitable pharmaceutically acceptable excipients as described herein. In some embodiments, the compositions are administered by the oral or nasal respiratory route for local or systemic effect. Compositions in pharmaceutically acceptable solvents can be nebulized by use of inert gases. Nebulized solutions can be inhaled directly from the nebulizing device or the nebulizing device can be attached to a face mask, or intermittent positive pressure breathing machine. Solution, suspension, or powder compositions can be administered, orally or nasally, from devices that deliver the formulation in an appropriate manner. For administration by inhalation, the compounds can be delivered in the form of an aerosol spray from a pressured container or dispenser that contains a suitable propellant, e.g., a gas such as carbon dioxide, or a nebulizer. Such methods include those described in U.S. Patent No. 6,468,798; U.S. Patent No. 6,695,227; U.S. Patent No. 7,431,222; US20050224608.

The composition can be administered through various devices, e.g., a metered dose sprayer, a squeeze bottle, a pressurized metered dose inhaler, a dry powder inhaler, a ventilator, or face mask and tent. Systems of aerosol delivery, such as the pressurized metered dose inhaler and the dry powder inhaler are disclosed in Newman, S. P., Aerosols and the Lung, Clarke, S. W. and Davia, D. editors, pp. 197-22. The subject can also inhale the composition through a ventilator, which will be set to a flow rate based on patient comfort and needs. This is determined by pulmonary graphics (i.e., respiratory rate, tidal volumes, etc.). The composition can also be prepared to allow inhalation by the subject using a facemask or tent. In certain cases, the composition can be packaged into a portable device (e.g., a portable inhaler device) and administered in a metered dose, for example, to permit intermittent treatment of a recipient who is not in a hospital setting. Different concentrations of composition could be packaged in the containers.

The composition can be aerosolized by any appropriate method. For use with humans or other primates, the aerosol can be generated using a medical nebulizer system which delivers the aerosol through a mouthpiece, facemask, etc., from which the subject can draw the aerosol into the lungs. Various nebulizers are known in the art and can be used in the method as described herein. See, e.g., U.S. Pat. No. 4,268,460; U.S. Pat. No. 4,253,468; U.S. Pat. No. 4,046,146; U.S. Pat. No. 3,826,255; U.S. Pat. No. 4,649,911; U.S. Pat. No. 4,510,829. The selection of a nebulizer system will depend on whether alveolar or airway delivery (i.e., trachea, primary, secondary or testiary bronchi, etc.) is desired.

A convenient way to effectively deliver the composition to the alveoli is to select a nebulizer which produces sufficiently small particles (e.g., producing particles with a mean particle diameter of less than 5.0 microns (μm), more preferably having a mean particle diameter of about 0.2 to about 4.0 μm, and most preferably having a mean diameter of about 0.2 to about 2 μm). As an alternative to selecting small mean particle diameters to achieve substantial alveoli deposition, a very high dosage of the composition can be administered, with a larger mean particle diameter. In such an approach, it is beneficial if the particular composition is not too irritating at the required dosage and that there are a sufficient number of particles in the total population having a diameter in the 0.5 to about 5 μm range to allow for deposition in the alveoli. For proximal airway delivery, the mean particle size may be larger. For example, suitable mean particle diameters may generally be less than about 15 μm, e.g., from about 4 μm to about 15 μm, e.g., from about 5 μm to about 10 μm.

Examples of nebulizers useful for alveolar delivery include the Acorn 1 nebulizer, and the Respirgard II™ Nebulizer System, both available commercially from Marquest Medical Products, Inc., Inglewood, Colo. Other commercially available nebulizers for use with the instant invention include the Ultra Vent™ nebulizer available from Mallinckrodt, Inc. (Maryland Heights, Mo.); the Wright nebulizer (Wright, B. M., Lancet (1958) 3:24-25); and the DeVilbiss nebulizer (Mercer et al., Am. Ind. HYR. Assoc. J. (1968) 29:66-78; T. T. Mercer, Chest (1981) 80:6 (Sup) 813-817). Nebulizers useful for airway delivery include those typically used in the treatment of asthma. Such nebulizers are also commercially available. One of skill in the art can determine the usefulness of a particular nebulizer by measuring the mean particle size generated thereby with e.g. a 7 stage Mercer cascade impactor (Intox Products, Albuquerque, N. Mex.).

The total amount of the compound delivered to the subject will depend upon several factors, including the total amount aerosolized, the type of nebulizer, the particle size, subject breathing patterns, severity of lung disease, and concentration in the aerosolized solution, and length of inhalation therapy. The amount of composition measured in the alveoli may be substantially less than what would be expected to be from the amount of compound present in the aerosol, since a large portion of the compound may be exhaled by the subject or trapped on the interior surfaces of the nebulizer apparatus.

Skilled practitioners will be able to readily design effective protocols, particularly if the particle size of the aerosol is optimized. Based on estimates of nebulizer efficiency, an effective dose delivered may lie in the range of about 1 mg/treatment to about 500 mg/treatment, although more or less may be found to be effective depending on the subject and desired result. In some instances, it is useful to administer higher doses when treating more severe conditions. If necessary, the treatment can be repeated on an *ad hoc* basis depending upon the results achieved. If the treatment is repeated, the mammalian host can be monitored to ensure that there is no adverse immune response to the treatment. The frequency of treatments depends upon a number of factors, such as the amount of compound administered per dose, as well as the health and history of the subject. As used herein, the "amount nebulized" or "amount aerosolized" of the compound means the amount that actually leaves the apparatus as an aerosol, i.e., the amount placed into the apparatus less the amount retained in the reservoir and on the inner surfaces of the apparatus at the conclusion of a treatment session.

**Systemic Administration**

Systemic administration refers to a route of administration of the therapeutic agent into the circulatory system so that the entire body is affected. Systemic administration of a therapeutic agent as described herein can be achieved by intraperitoneal injection or infusion (e.g., IV infusion or IV injection). Intraperitoneal injection is the injection of the therapeutic agent into the peritoneum. In humans, it is often used to inject a large amount of fluids in infants. Infusion involves the administration of the therapeutic agent through a needle or catheter. The therapeutic agent can be administered into a blood vessel (e.g., veins or arteries) or subcutaneously.

Systemic administration of VEGF can cause vasodilation and can help with the vasoconstriction (pulmonary artery) in the subjects in the need thereof. However, as shown in the present disclosure, systematic administration of VEGF may further lead to somnolence, emesis, or hypotension. Hypotension can be dangerous. Slow administration (e.g., slow infusion) can address the hypotensive side effects of VEGF. Slow administration is especially important in the beginning as the body is the most sensitive to the effects of VEGF at this point. Once the VEGF is administered into the body, the body becomes desensitized to VEGF to some extent and the rate of administration can be ramped up slowly.

Thus, the duration of administration (e.g., IV infusion) of the therapeutic agent (e.g., VEGF or HB-EGF) for one dose (e.g., 50 mcg/kg, 100 mcg/kg, 150 mcg/kg, and 200 mcg/kg) can be more than 60 minutes, more than 70 minutes, more than 80 minutes, more than 90 minutes, more than 100 minutes, more than 110 minutes, more than 2 hours, more than 3 hours, or more than 4 hours. In some embodiments, the duration of administration is less than 3 hours, less than 2 hours, or less than 90 minutes (e.g., between 60 and 90 minutes).

Furthermore, as slow administration is especially important in the beginning, in the first administration period (e.g., the first 5, 10, 20 or 30 minutes), only less than 40%, less than 35%, less than 30%, less than 25%, less than 20%, less than 15%, less than 10%, or less than 9%, less than 8%, less than 7%, less than 6%, less than 5%, less than 4%, less than 3%, less than 2%, or less than 1% of the required dose (e.g., 50 mcg/kg, 100 mcg/kg, 150 mcg/kg, and 200 mcg/kg) is administered to the subject. Then, the rate of administration in the following period (e.g., the next 30, 40, 50, 60 minutes) can be 2, 3, 4, 5, 6, 7, 8, 9, 10, or more than 10 times higher than the rate of administration in the first period.

In some therapy regimens, the infusion rate can be between about 1 to about 2.5 mcg/kg/min, between about 1 to about 2 mcg/kg/min, or between about 1.5 to about 2 mcg/kg/min (e.g., about 1.5, 1.6, 1.7, 1.8, 1.9, 2.0 mcg/kg/min) in the first administration period (e.g., the first 5, 10, 20 or 30 minutes). The infusion rate can be then gradually increased to between about 2 to about 3 mcg/kg/min (e.g., about 2.0, 2.1, 2.2, 2.3, 2.4, 2.5, 2.6, 2.7, 2.8, 2.9, or 3.0 mcg/kg/min) in the next administration period (e.g., the next 5, 10, 20 or 30 minutes). If hypotension is controlled or not a concern, the infusion rate can be further increased to the maximum infusion rate. The maximum infusion rate is between about 3 to about 5 mcg/kg/min, between about 3 to about 4.5 mcg/kg/min, between about 3 to about 4 mcg/kg/min, or between about 3 to about 3.5 mcg/kg/min. Thus, the maximum infusion rate can be, e.g., about 3.0, 3.1, 3.2, 3.3, 3.4, 3.5, 3.6, 3.7, 3.8, 3.9, or about 4.0 mcg/kg/min.

The subject may experience somnolence and/or emesis during the infusion. These symptoms need not necessarily result in cessation of infusion. However, infusion can be held or the rate can be reduced if certain concerning symptoms are manifested (e.g., large emesis, diaphoresis, and labored respiration). In some cases, subjects can be treated by extracorporeal membrane oxygenation (ECMO), thus these subjects can better tolerate hypotension. Furthermore, the cannulation of major vessels to establish the ECMO circuit requires the use of systemic anticoagulants, i.e. heparin, to avoid thrombotic complications. Some other anticoagulants (e.g., direct thombin inhibitors (DTI), i.e. argatroban and bivalirudin) can be used as alternatives.

### Alleviating side effects of heparin

Heparin is a medication which is often used as an anticoagulant. It can be used to treat and prevent deep vein thrombosis, pulmonary embolism, and arterial thromboembolism. It is also used in the treatment of heart attacks and unstable angina. Common side effects include bleeding, pain at the injection site, and low blood platelets. Serious side effects include heparin induced thrombocytopenia.

As shown in the present disclosure, heparin impairs compensatory lung growth. Thus, for a subject who is need of lung growth, heparin should be avoided. But if a treatment requires heparin, VEGF can be co-administered to the subject to alleviate the side effects of heparin. Therefore, the present disclosure provides compositions for use in methods of treating a subject, involving administering to the subject an effective amount of a composition comprising VEGF, and an effective amount of heparin. The disclosure also provides compositions for use in methods of alleviating the side effects of heparin. The methods involve, e.g., administering to the subject an effective amount of heparin; assessing the side effects of heparin; and administering an effective amount of a composition comprising VEGF to the subject. In methods involving co-administration, VEGF and heparin can be administered essentially simultaneously or at different time points. For example, at least one dose of VEGF can be administered followed by at least one dose of heparin at least one time point thereafter. Alternatively, at least one dose of heparin can be administered followed by at least one dose of VEGF at least one time point thereafter. As yet another alternative, heparin and VEGF can be administered at the same time, e.g., in a single or multiple doses. In some instances, heparin and VEGF can be administered as two separate compositions. Alternatively or in addition, a single composition comprising both heparin and VEGF can be administered to a patient. Skilled practitioners will appreciate that that a dosage regimen of heparin and VEGF can be modified based on the needs and condition of the patient, as well as on the procedure with which the patient is being treated.

## Managing Side Effects Induced by VEGF

This disclosure also shows that administering VEGF can in some instances induce systemic hypotension and edema. Thus, in some embodiments, the blood pressure of the subject is monitored (e.g., by blood pressure monitor). The blood pressure of the subject can be monitored before, during, and after the administration. In some cases, the blood pressure can be monitored for at least 30 minutes, e.g. at least or about 1 hour, 2 hours, 3 hours, or at least or about 4 hours after the administration.

Many agents are available to control the hypotensive effect of VEGF. These agents generally have the effects of increasing blood pressure or inducing hypertension. These agents include, for example, midodrine, norepinephrine, norepinephrine bitartrate, phenylephrine, droxidopa, ephedrine, and N-Nitroarginine methyl ester (CHEMBL7890). Thus, in some embodiments, an agent that can increase blood pressure is administered before, during, or after administering VEGF to a subject. In some embodiments, VEGF and an agent that can increase blood pressure is formulated in the same pharmaceutical composition.

VEGF can be administered to subjects who are also treated by Extracorporeal membrane oxygenation (ECMO). ECMO provides both cardiac and respiratory support to patients whose heart and lungs are unable to provide an adequate amount of gas exchange to sustain life. The blood pressure of these subjects receiving ECMO intervention are often closely monitored. Thus, if VEGF causes any side effects, they can be promptly treated.

## Dosage

An "effective amount" is an amount sufficient to effect beneficial or desired results. For example, a therapeutic amount is one that achieves the desired therapeutic effect. This amount can be the same or different from a prophylactically effective amount, which is an amount necessary to prevent onset of disease or disease symptoms. An effective amount can be administered in one or more administrations, applications or dosages. A therapeutically effective amount of a therapeutic agent (i.e., an effective dosage) depends on the therapeutic agents selected. The compositions can be administered one from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of the therapeutic agents described herein can include a single treatment or a series of treatments.

Dosage, toxicity and therapeutic efficacy of the therapeutic agents can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Agents which exhibit high therapeutic indices are preferred. While agents that exhibit toxic side effects may be used, care should be taken to design a delivery system that targets such agents to the site of affected tissue in order to minimize potential damage to uninfected cells and, thereby, reduce side effects. How to estimate a safe range for a human patient is described, e.g., in Food and Drug Administration. "Guidance for industry: estimating the maximum safe starting dose in initial clinical trials for therapeutics in adult healthy volunteers." Center for Drug Evaluation and Research (CDER) (2005).

The data obtained from cell culture assays and animal studies can be used in formulating a range of dosage for use in humans. The dosage of such agents lies preferably within a range of circulating concentrations that include the ED50 with little or no toxicity. The dosage may vary within this range depending upon the dosage form employed and the route of administration utilized. For any agent used in the method of the invention, the therapeutically effective dose can be estimated initially from cell culture assays. A dose may be formulated in animal models to achieve a circulating plasma concentration range that includes the IC50 (i.e., the concentration of the test agent which achieves a half-maximal inhibition of symptoms) as determined in cell culture. Such information can be used to more accurately determine useful doses in humans. Levels in plasma may be measured, for example, by high performance liquid chromatography.

An appropriate dosage can also be determined from the dosage that is used in animal models. In some embodiments, the conversion between dosage for piglets and dosage for humans is 1.1:1. For systemic administration (e.g., intravenous administration), the dosage can be 10 - 200 mcg/kg, 50 - 200 mcg/kg, or 50 - 150 mcg/kg for a human subject. In some embodiments, the dosage can be about 50 mcg/kg, about 75 mcg/kg, about 100 mcg/kg, about 125 mcg/kg, about 150 mcg/kg, about 175 mcg/kg, or about 200 mcg/kg. In some embodiments, the dosage is less than 400 mcg/kg.

## EXAMPLES

The invention is further described in the following examples, which do not limit the scope of the invention described in the claims.

### EXAMPLE 1. Systemic VEGF in the Rodent Compensatory Lung Growth Model

Experiments were preformed to determine the effects of administering VEGF to mice via intraperitoneal (ip) injection.

#### Methods

*Experimental Groups and Surgical Procedure*

Eight to ten week old C57BL/6 male mice (Jackson Laboratories, Bar Harbor, ME) were randomized into control or VEGF experimental groups. Prior to left pneumonectomy, VEGF groups received 0.5 mg/kg of recombinant murine VEGF-164 (GenScript, Piscataway, NJ) (SEQ ID NO: 1) via intraperitoneal (ip) injection. Control groups received an isovolumetric volume of normal saline via ip injection. Mice were anesthetized with 120-400 mg/kg of Avertin (Sigma, St. Louis, MO) via ip injection and orotracheally intubated. The animal was ventilated on room air with a rodent ventilator (HSE-HA Minivent, Harvard Apparatus, Holliston, MA) at 70 breaths/minute. The left chest was sterilized with povidone-iodine and ethanol, and a skin incision was made along the left anterior axillary line, followed by a thoracotomy at the fifth intercostal space. The left lung was then gently delivered onto the operative field and ligation of the hilum was achieved with a silk suture. Following closure of the thoracotomy and skin incisions, the animal was resuscitated with 3 mL of warm normal saline via subcutaneous injection. Post-operative pain control was achieved with twice daily subcutaneous injection of buprenorphine for 72 hours. Postoperatively, VEGF and control groups were further divided into two treatment-duration groups, receiving daily ip injections of VEGF or saline for either 4 or 10 days postoperatively. All procedures were carried out according to the National Institutes of Health Guide for the Care and Use of Laboratory Animals and approved by the Institutional Animal Care and Use Committee at Boston Children's Hospital.

*Pulmonary Mechanical Studies*

On postoperative day (POD) 4 or 10 and immediately before euthanasia, animals underwent pulmonary mechanical measurements with the Flexivent® system (SCIREQ, Montreal, Canada). Mice were anesthetized with Avertin. A midline neck incision was made to expose the trachea, followed by tracheotomy and insertion of a 20-gauge hollow bore needle, which was connected to the Flexivent® system. Elastance and compliance were measured with the single frequency forced oscillation technique, which registered the animal's response to an applied sinusoidal waveform. The system also generated pressure-volume (PV) loops, which allowed for the calculation of the inspiratory capacity and area under the PV loop. To normalize these parameters for the animal's size, inspiratory capacity, area under the PV loop and compliance were divided by body weight, while elastance was multiplied by body weight.

*Organ Harvest, Mass and Volume Measurement*

Animals were euthanized upon disconnection from the Flexivent® system. Animals were weighed and the remaining right lung was removed en-bloc with the tracheobronchial tree. Specimens for morphometric analysis were inflated with 10% formalin at 20 $cmH_2O$ via the previously intubated 18-gauge needle and total lung volume was measured with the water displacement method. The trachea was then ligated with a 4-0 silk suture to maintain alveolar distension and the specimen was placed in 10% formalin overnight at 4°C. The liver, spleen, and kidneys were also harvested, weighed, and preserved in a similar fashion. All specimens were transferred into 70% ethanol after 24 hours of formalin fixation and subsequently embedded in paraffin for histologic analyses. Lung volume and organ mass measurements were normalized for body weight. Lung specimens for RNA/protein analysis were immediately flash frozen in liquid nitrogen for future tissue analysis.

*Morphometric Analysis*

Quantitative microscopy was performed on hematoxylin and eosin (H&E) stained lung sections based on the principles of stereology. Five lung specimens from each group were randomly selected for morphometric analyses. One 5-micron section from each specimen was examined at 200X magnification using the principle of systemic uniform random sampling: the first lung field on each section was selected randomly, followed by systemic sampling of every other field to achieve a total of 40-50 lung fields per section. For each field, a 42-point test lattice with predefined grid line length was used to facilitate point and intersection counting. Point counting allowed for estimation of parenchymal volume, alveolar volume, alveolar to parenchymal volume ratio, and septal volume while intersection counting facilitated the measurement of septal surface area and mean septal thickness. Parenchyma was defined as the gas-exchange compartment of the lung, which included alveoli, terminal ducts, and alveolar septa, while airways, vessels, and pleura were classified as non-parenchyma. All volume and area measurements were normalized for body weight.

Under the assumptions that alveolar units assume a spherical shape and are randomly distributed throughout the lung parenchyma, alveolar density was mathematically calculated from counting the number of alveolar transections on randomly chosen lung fields using the method that is described in Weibel ER, Gomez DM. A principle for counting tissue structures on random sections. J. Appl. Physiol. 17, 343-8 (1962):

$$N = \frac{n^{3/2}}{1.382 \times \sqrt{p}}$$

where p is the alveolar to parenchymal volume ratio, n is the number of alveolar transections per unit of area, and N is the number of alveoli per corresponding unit of volume. For each specimen, twenty random lung fields were selected for alveolar counting. The total number of alveolar units per specimen was derived from the product of alveolar density and parenchymal volume.

*Quantitative Real-Time Polymerase Chain Reaction (qPCR)*

Total RNA was extracted from lung tissues with the RNeasy Mini Kit (Qiagen, Hilden, Germany) according to the manufacturer's protocol. RNA concentration was determined with the NanoDrop 8000 Spectrophotometer (Thermo Fisher Scientific, Waltham, MA). Amplification reactions were performed with the StepOne Real-Time PCR Systems (Applied Biosystems, Foster City, CA) using pre-designed and validated TaqMan® primers for VEGF receptor (VEGFR) 1, VEGFR2, neuropilin (NRP) 1, VEGF-A, epidermal growth factor (EGF), and EGF receptor (EGFR) (Applied Biosystems, Foster City, CA). All target genes were normalized to the housekeeping gene GAPDH and their mRNA expression level was calculated using the $2^{\Delta\Delta Ct}$ method.

*Western Blot and Enzyme-Linked Immunosorbent Assay (ELISA)*

Lung tissue samples from animals euthanized on POD 4 were immediately suspended in radioimmunoprecipitation assay (RIPA) buffer (Boston Bio Products, Ashland, MA) containing protease and phosphatase inhibitors (Thermo Fisher Scientific, Waltham, MA). Tissue samples were then homogenized in a Bullet Blender (Next Advance Inc., Averill Park, NY) and centrifuged for 15 minutes at 4°C and 14,000 rpm. The supernatants were collected and protein concentration was measured with the Bradford colorimetric assay (Bio-Rad Laboratories Inc, Hercules, CA). Forty micrograms of protein from each sample were mixed with 4x Laemmli buffer (Boston Bio Products, Ashland, MA), heated at 95°C for 5 minutes, and separated on a 4-12% Bis-Tris polyacrylamide gel (Thermo Fisher Scientific, Waltham, MA). The proteins were then transferred to a nitrocellulose membrane and blocked in a TBST solution (50 mM Tris-HCl - pH 7.4, 150 mM NaCl, and 0.1% Tween 20) containing 5% non-fat dry milk (Bio-Rad Laboratories Inc, Hercules, CA) for 1 hour. Incubation with primary antibodies diluted in blocking buffer was done overnight at 4°C. Primary antibodies included anti-VEGFR1, -MMP14 (Abcam, Cambridge, UK), -VEGFR2, -P-1175-VEGFR2, -NRP1, - EGFR, -P-1068-EGFR (Cell Signaling Technology, Danvers, MA), -HB-EGF (R&D Systems, Minneapolis, MN), and -β-Actin (Sigma-Aldrich, St. Louis, MO). On the following day, the membrane was washed with TBST for 10 minutes x3 before incubation in secondary antibodies, horseradish peroxidase conjugated goat anti-rabbit, anti-mouse (Santa Cruz Biotechnology, Dallas, TX), or anti-rat IgG (R&D Systems, Minneapolis, MN) antibodies for 1 hour at room temperature. The membrane was again washed with TBST for 10 minutes x3, followed by signal visualization with the enhanced chemiluminescence (ECL) reagents (Thermo Fisher Scientific, Waltham, MA). Lung tissue levels of EGF were determined with ELISA (R&D Systems, Minneapolis, MN) according to the manufacturer's protocol.

*Immunohistochemistry (IHC)*

Formalin-fixed, paraffin-embedded sections were deparaffinized in xylene and rehydrated through a graded series of alcohols to water. Antigen retrieval was achieved with citrate-based buffer (pH = 6) at 95°C for 5 minutes, followed by endogenous peroxidase inhibition with $H_2O_2$ for 12 minutes and protein blocking with 5% non-fat dry milk. Sections were then incubated in anti-mouse VE-Cadherin antibody (R&D Systems, Minneapolis, MN) overnight at 4°C, followed by rabbit anti-goat IgG antibody (Vector Laboratories, Burlingame, CA). Signal visualization was achieved with the VECTASTAIN® kit and DAB® chromogen (Vector Laboratories, Burlingame, CA), followed by counterstaining with hematoxylin (Thermo Fisher Scientific, Waltham, MA).

*Statistical Analysis*

Outcomes, adjusted for body weight, were analyzed with a 2-way analysis of variance containing a main effect for POD, a main effect for group, and the interaction effect POD $\times$ group. The interaction effect comprised six pairwise comparisons, two of which were considered a priori to be of interest (POD 4, control vs POD 4, VEGF; POD 10, control vs POD 10, VEGF). These comparisons were adjusted for multiplicity using a step-up Bonferroni adjustment. All outcomes were normally distributed except inspiratory capacity, dynamic elastance, parenchymal volume, alveolar volume, and mean septal thickness. For these, a sensitivity analysis was conducted by transforming each outcome using normal scores and comparing the results to the non-transformed outcomes. In all cases the results were consistent, and are presented in their original units. Tests of significance were two-sided, with $P < 0.05$ considered statistically significant. Results are presented as mean $\pm$ standard error (SE). All data analysis was conducted with SAS v9.4 (Cary, NC).

**Results**

*Physiologic Studies*

Mice in the VEGF group had a significantly higher lung volume to body weight ratio (LV/BW) on POD 4 compared to those in the control group ($5.86 \pm 0.18 \times 10^{-2}$ mL/g vs $5.20 \pm 0.18 \times 10^{-2}$ mL/g, $P = 0.03$). On POD 10, there was no difference in LV/BW between the two groups. When normalized for body weight, there was no difference in liver, kidney, or spleen weights between the two experimental groups at either time point (**FIGS. 1A-1D**).

*Pulmonary Mechanical Studies*

Normalized for body weight, there was no difference between the two treatment groups at either time point (POD 4 and 10) in inspiratory capacity, area under the PV loop, dynamic elastance, or dynamic compliance (**FIGS. 2A-2D**).

*Morphometric Studies*

Mice in the VEGF group displayed a significant increase in adjusted parenchymal volume ($4.7 \pm 0.2 \times 10^{-2}$ mL/g vs $3.8 \pm 0.2 \times 10^{-2}$ mL/g, $P = 0.001$), alveolar volume ($3.0 \pm 0.1 \times 10^{-2}$ mL/g vs $2.3 \pm 0.1 \times 10^{-2}$ mL/g, $P = 0.0003$), total alveolar count ($4.7 \pm 1.7 \times 10^7$ vs $2.8 \pm 1.7 \times 10^7$, $P < 0.0001$), and septal surface area ($19.0 \pm 0.7$ $cm^2$/g vs $15.5 \pm 0.7$ $cm^2$/g, $P = 0.007$) on POD 4 compared to the control group (**FIGS. 3A-3F**). On POD 10, all of these parameters equalized except for alveolar count, which remained higher in the VEGF group ($3.9 \pm 1.7 \times 10^7$ vs $3.1 \pm 1.7 \times 10^7$, $P = 0.006$). Mice in the VEGF group had higher adjusted septal volume compared to those in the control group on both POD 4 ($0.90 \pm 0.05 \times 10^{-2}$ mL/g vs $0.76 \pm 0.05 \times 10^{-2}$ mL/g, $P = 0.06$) and POD 10 ($1.00 \pm 0.05 \times 10^{-2}$ mL/g vs $0.85 \pm 0.05 \times 10^{-2}$ mL/g, $P = 0.06$), but these differences did not meet the established statistical cutoff of $P < 0.05$. There was no difference in mean septal thickness between the two groups. The alveolar density and number were significantly higher in the VEGF group compared to the control group on POD 4 ($4.23 \pm 0.07 \times 10^7$ per $cm^3$ vs $3.23 \pm 0.02 \times 10^7$ per $cm^3$, $P = 0.0007$, and $4.74 \pm 0.17 \times 10^7$ vs $2.79 \pm 0.17 \times 10^7$, $P = 0.00004$, respectively) (**FIGS. 4A-4B**).

*Gene and Protein Expression Analysis and IHC*

There was no difference in the messenger-RNA (mRNA) level of VEGF, VEGFR1, VEGFR2, NRP1, or EGFR between the VEGF and control groups. However, VEGF-treated mice displayed a 20.5-fold increase in the level of EGF on POD 4 compared to the control group ($P = 0.01$) (**FIG. 5**). There was no difference in protein expression levels of VEGFR1, VEGFR2, NRP1, or P-EGFR between the two experimental groups. However, the expression level of P-VEGFR2 on POD 4 was 4.2-fold higher in the VEGF group compared to the control group ($P = 0.04$). Concurrently, there was a 30% decrease in the expression level of EGFR in the VEGF group compared to the control group ($P = 0.01$) (**FIGS. 6A-6B**). IHC labeling of lung sections for VE-Cadherin revealed uniform staining of alveolar septum.

*Conclusion*

This example demonstrated that there were no differences in several measures of pulmonary mechanics between mice that received VEGF and those that received saline after left pneumonectomy, in spite of an increased total lung volume in the VEGF-treated group. The data in this example indicate that systemic VEGF therapy did not alter dynamic compliance or resistance, suggesting that the tissue changes that occurred in the VEGF-treated group did not alter overall mechanics. This finding is relevant since early report demonstrated that treatment of lung primordia from mice with and without exposure to nitrofen and subsequent treatment with fibroblast growth factor (FGF) may result in cystic changes within the lung. If treatment with VEGF were to produce a similar effect, increased compliance and decreased elastance would be expected, as observed in the case of emphysema; however, this was not observed in this example. There was also no difference in inspiratory capacity between VEGF-treated and saline-treated mice, despite increased total lung volume as measured by volume-displacement in VEGF-treated mice. It is possible that additional alveolar recruitment maneuvers are necessary to reflect the increased lung volume observed in the VEGF-treated group.

Accurate quantification of the pulmonary architecture is important in establishing a structure-function model and providing important information to supplement physiologic data. This is especially important in the post-pneumonectomy model where the asymmetric anatomy of the thoracic cavity and differential mechanical strain imposed on the remaining lung results in uneven rate of compensatory growth in different pulmonary lobes. The principles of stereology, which allowed for unbiased quantification of 3D pulmonary structures by uniform sampling of the entire lung section with 2D geometric probes, were used. VEGF-treated mice displayed both increased alveolar volume and septal surface area on POD 4. Additionally, these parameters, which constitute the alveolar units, increased in proportion to the parenchymal volume, an observation supported by the preserved alveolar to parenchymal volume ratio in both experimental groups. Most importantly, VEGF treatment resulted in an increase in total alveolar count, an effect that persisted even on POD 10. Additionally, IHC labeling of lung sections with VE-Cadherin showed uniform staining of alveolar septum, suggesting that the increase in alveolar units and septal surface area was accompanied by an increase in vascularization and that VEGF administration in fact led to an increase in total capillary volume. Elsewhere, morphometric studies done on immature dogs have shown that compensatory alveolar growth post-pneumonectomy is accompanied by proliferation of all septal tissue components and there is a good correlation between morphometric analyses and physiological measurements of pulmonary diffusing capacity.

The data also demonstrated that the observed volumetric, mechanical, and morphometric findings were associated with measurable changes in gene and protein expression. The activated state of pulmonary VEGFR2 was significantly increased by exogenous VEGF therapy after unilateral pneumonectomy in this example. Based on these findings, it is possible that exogenous VEGF accelerates compensatory lung growth by directly stimulating pulmonary capillary endothelial cell proliferation via a VEGFR2-dependent mechanism, which subsequently induces alveolar epithelial cell growth and formation of new alveolar units.

Additionally, this example demonstrated a significant increase in the expression of EGF mRNA in VEGF-treated mice after unilateral pneumonectomy. ELISA, however, failed to detect EGF levels in lung tissues, probably a result of its small size, secretory nature, and lack of interaction with the extracellular matrix that contributed to its low local concentrations. Interestingly, treatment with VEGF did not alter the levels of MMP14 or HB-EGF, suggesting that exogenous VEGF upregulated EGF in a mechanism that is separate from natural compensatory lung growth. In this study, a decrease in the protein expression level of EGFR in response to exogenous VEGF treatment was observed. This could be explained by increased internalization of the receptor in the presence of increased ligand concentration.

Although a compensatory lung growth model was used in this study to illustrate development of hypoplastic lungs, these two seemingly distinct processes share certain fundamental commonalities. They are both driven by mechanical stress created by either a symmetrically enlarging thorax (lung development) or by an asymmetrical empty space (compensatory growth), with the hypoxia-induced factor (HIF)-VEGF axis playing a critical role in both models. Additionally, at the level of gene transcription, post-pneumonectomy lungs have been shown to first dedifferentiate before undergoing redevelopment, a process that recapitulates the late alveolar stage of lung development. Data obtained from a compensatory lung growth model therefore can produce valuable insights into lung organogenesis, especially during its later stages.

In conclusion, VEGF accelerates compensatory lung growth in mice while maintaining normal pulmonary mechanics and by increasing both parenchymal and alveolar volume. The data suggest that VEGF may increase the gas-exchange capacity of the growing lung, as opposed to merely increasing its volume. These changes may be mediated by a VEGFR2 and EGF-dependent mechanism.

**EXAMPLE 2. Inhalational VEGF in the Rodent Compensatory Lung Growth Model**

Experiments were preformed to determine the effects of administering VEGF to mice via inhalation.

**Methods**

*Experimental Groups and Surgical Procedure*

Eight to ten week old C57BL/6 male mice (Jackson Laboratories, Bar Harbor, ME) were randomized into iSALINE or iVEGF experimental groups. Mice underwent left pneumonectomy. Immediately following surgery, mice in the iVEGF group received 0.5 mg/kg of recombinant murine VEGF-164 (GenScript, Piscataway, NJ) via nasal aspiration. The composition was administered deep into the lungs, which was essentially the same as inhalation. The iSALINE group received an isovolumetric volume of normal saline via nasal aspiration. Postoperatively, they continued to receive daily administration of VEGF or saline via nasal aspiration under anesthesia with isofluorane at 1-3% until POD 4. All procedures were carried out according to the National Institutes of Health Guide for the Care and Use of Laboratory Animals and approved by the Institutional Animal Care and Use Committee at Boston Children's Hospital.

*Pulmonary Mechanical Studies, Organ Harvest, and Mass and Volume Measurement*

On POD 4 and immediately before euthanasia, animals underwent pulmonary mechanical measurements with the Flexivent® system via tracheotomy. Specimens for morphometric analysis were infused with 10% formalin at 20 $cmH_2O$ and total lung volume was measured with the water displacement method. The liver, spleen, and kidneys were also harvested, weighed, and preserved in a similar fashion. All specimens were stored in 10% formalin at 4°C for 24 hours before being transferred into 70% ethanol and subsequently embedded in paraffin for histologic analyses. Lung volume and organ mass measurements were normalized for body weight. Lung specimens for RNA/protein analysis were immediately flash frozen in liquid nitrogen for future tissue analysis.

*Morphometric Analyses and Gene Expression Analyses*

Parenchymal volume, alveolar volume, septal volume, septal surface area, and mean septal thickness were calculated from 5 randomly selected specimens in each group with the point and intersection counting technique. Alveolar density and number were estimated with the transection counting technique.

mRNA expression level for VEGFR1, VEGFR2, VEGF, EGFR, and EGF was determined with quantitative real-time PCR using pre-designed and validated TaqMan® primers. Western blot for pulmonary protein expression of VEGFR2, P-1175- VEGFR2, EGFR, and P-1068-EGFR was also performed.

**Results**

*Physiologic Studies*

Mice in the iVEGF group had a significantly higher LV/BW on POD 4 compared to those in the iSALINE group ($5.49 \pm 0.11 \times 10^{-2}$ mL/g vs $4.91 \pm 0.07 \times 10^{-2}$ mL/g, $P$ = 0.0004). There was no difference in liver, kidney, or spleen weights between the two experimental groups (**FIGS. 7A-7D**).

*Pulmonary Mechanical Studies*

Normalized for body weight, there was no difference between the two treatment groups in dynamic elastance or dynamic compliance (**FIGS. 8A-8B**).

*Morphometric Studies*

Mice in the iVEGF group displayed a significant increase in adjusted parenchymal volume ($4.7 \pm 0.3 \times 10^{-2}$ mL/g vs $3.7 \pm 0.2 \times 10^{-2}$ mL/g, $P$ = 0.02), septal surface area ($19.2 \pm 0.8$ $cm^2$/g vs $15.6 \pm 0.8$ $cm^2$/g, $P$ = 0.01), and alveolar count ($3.61 \pm 0.16 \times 10^7$ vs $2.61 \pm 0.20 \times 10^7$, $P$ = 0.005) on POD 4 compared to the iSALINE group (**FIGS. 9A-9D**). Mice in the iVEGF group had higher adjusted alveolar volume compared to those in the control group on POD 4 ($2.8 \pm 0.2 \times 10^{-2}$ mL/g vs $2.2 \pm 0.1 \times 10^{-2}$ mL/g, $P$ = 0.06) although this difference did not achieve statistical significance. There was no difference in mean septal thickness between the two groups.

*Gene and Protein Expression Analysis*

There was no difference in the messenger-RNA (mRNA) level of VEGF, VEGFR1, VEGFR2, or EGFR between the iVEGF and iSALINE groups. However, mice that received VEGF displayed a 2.4-fold increase in the level of EGF on POD 4 compared to those receiving saline (*P* = 0.013) (**FIG. 10**). On Western blot analyses, mice in the iVEGF group displayed a 1.7-, 1.7-, 2.4-, and 1.5-fold increase in the expression level of P-VEGFR2, VEGFR2, P-EGFR, and EGFR, respectively (*P* = 0.03, 0.02, 0.05, and 0.02, respectively) (**FIGS. 11A-11B**).

**EXAMPLE 3. Systemic VEGF Administration in the Swine Compensatory Lung Growth Model**

Experiments were preformed to determine the effects of administering VEGF to piglets via infusion.

**Methods**

*Surgical Model of Left Pneumonectomy in Piglets*

All procedures were carried out according to the National Institute of Health' Guide for the Care and Use of Laboratory Animals and approved by the Institutional Animal Care and Use Committee at Boston Children's Hospital. Five-day old female Yorkshire piglets (Parsons Pig Farm, Hadley, MA) arrived at the facility 5-6 days before surgery for acclimation and pre-operative optimization. The piglets were bottle-fed with warmed Grade A Ultra 24 Milk Replacer (Milk Products, Chilton, WI) four times per day, ensuring that no more than 12 hours elapse between feeds. Piglets were made nothing by mouth overnight in preparation for the surgical procedure on the following morning.

Pre-operative induction was achieved with an intramuscular injection of atropine (0.04 mg/kg), tiletamine-zolazepam (4.4 mg/kg), and xylazine (2.2 mg/kg). Tracheal intubation is performed with a 2-4 mm cuffed endotracheal tube and a #1 Miller blade fiber-optic laryngoscope (Welch Allyn, Skaneateles Falls, NY). Anesthesia is maintained with isoflurane (1-4%). A 3-4 French central venous line was placed into the right external jugular vein for post-operative infusion of experimental treatment.

**FIG. 12** demonstrates the anatomy of the left pulmonary hilum with the right lung in the background. The piglet was placed in the right lateral decubitus position. The planned incision was located at the fourth intercostal space (**FIG. 13A**). After skin incision was made, subcutaneous and muscular layers were sequentially divided with electrocautery until entrance into the thoracic cavity was achieved. The superior lobe was then gently isolated and reflected caudally with a right-angle clamp. This maneuver allowed access to the pulmonary hilum and a view of its ventro-cranial aspect. Dissection of the hilum began with identification of the superior pulmonary vein (PV), which is the most ventral and cranial structure in the hilum (**FIG. 13B**). Division of the superior PV allowed access to the pulmonary artery (PA), which is dorsal and caudal to the PV (**FIG. 13C**). Once the PA was dissected off of the left main bronchus, it was ligated and divided. The left main bronchus is caudal to the PA and easily identified by its larger size and cartilaginous consistency (**FIG. 13D**). The most caudal hilar structure, the inferior PV, would come into view following ligation and division of the bronchus (**FIG. 13E**). After division of the inferior PV, the left lung was removed by dividing the inferior pulmonary ligament. After the specimen was removed, the empty left thoracic cavity (**FIG. 13F**) was filled with warm saline to check for both air leak and bleeding. The thoracotomy incision was closed with multiple interrupted PDS® sutures after removal of saline. The muscular layer was closed with running PDS® and the skin was closed with staples (Covidien, Dublin, Ireland).

The animal continued to be bottle-fed four times per day after surgery. After POD 14, the animal was introduced to creep feeds (Mini Pig Starter Diet, Scott's Distributing, Hudson, NH) and the frequency of formula feeds could be decreased to 2-3 times per day. Euthanasia was performed with sodium pentobarbital at a dose of 110 mg/kg at the end of the treatment period.

*Natural History of Compensatory Lung Growth in Piglets*

Nineteen female Yorkshire piglets were used to determine the natural history of compensatory lung growth. Five piglets were euthanized on day of life (DOL) 10-12 for baseline total and right lung volume measurement. The remaining 14 piglets underwent left pneumonectomy at the same age. Five piglets were euthanized on POD 7, four on POD 14, and five on POD 21. Lung specimens were infused with 10% formalin at 20 $cmH_2O$ and total lung volume was measured with the water displacement method and normalized for body weight.

*VEGF Infusion Tolerance and Pharmacokinetics Studies*

Neonatal female piglet's tolerance to VEGF infusion was tested at three different doses, 400, 200, and 100 mcg/kg. Ten-day old Yorkshire piglets were anesthetized. A central venous line was placed in the right external jugular vein for rhVEGF-165 (SEQ ID NO: 23) (Shire, Lexington, MA) infusion and right femoral vein for blood draw during infusion. An arterial line was also placed in the right carotid artery for blood pressure monitor during infusion. Volume of infusion was normalized at 10 mL/3 kg. At the end of infusion, the jugular venous line was left in place while both the arterial and femoral venous lines were removed and the vessels ligated. Serum sample was collected before and every 10 minutes during infusion via the femoral venous line and at 10 minutes, 20 minutes, 40 minutes, 1 hour, 1.5 hour, 2 hours, 4 hours, 8 hours, 12 hours, and 24 hours after infusion via the jugular venous line. Serum level of rhVEGF was determined with anti-human VEGF ELISA (R&D Systems, Minneapolis, MN) and used to generate the half life, volume of distribution (Vd), and rate of clearance (Cl) of rhVEGF in neonatal piglets.

*Systemic Administration of rhVEGF in the Swine Model of Compensatory Lung Growth*

Neonatal female Yorkshire piglets underwent left pneumonectomy and placement of jugular venous central line on DOL 10-11. They were then randomized to either the VEGF or the control group. Piglets in the VEGF group received daily infusion of rhVEGF-165 at the dose of 200 mcg/kg over 60-90 minutes while piglets in the control group received daily infusion of saline. Volume of infusion was again normalized to 10 mL/3 kg. Piglets were euthanized on POD7 for lung, heart, liver, spleen, left kidney, and right eye harvest. Lung specimens were infused with 10% formalin at 20 $cmH_2O$ and total lung volume was measured with the water displacement method. Lung volume and organ mass measurements were normalized for body weight.

Inflated lungs and organs were preserved in 10% formalin at 4°C for 48 hours before being switched to 70% ethanol. Every lung specimen was represented by a peripheral and a central H&E section for each of the 3 lung lobes, i.e. upper, middle, and lower. Morphometric data including parenchymal volume, alveolar volume, septal surface area, and alveolar number were measured.

**Results**

*Natural History of Compensatory Lung Growth in Piglets*

The average right lung volume at baseline was 92.1 ± 13.7 mL. This increased to 192.5 ± 24.8, 303.7 ± 25.9, and 332.7 ± 17.0 mL on POD 7, 14, and 21, respectively. Total LV/BW at baseline and before left pneumonectomy was $8.21 \pm 0.30 \times 10^{-2}$ mL/g. LV/BW of the right lung at baseline, on POD 7, 14, and 21 were $4.63 \pm 0.15 \times 10^{-2}$, $6.34 \pm 0.42 \times 10^{-2}$, $5.82 \pm 0.60 \times 10^{-2}$, and $5.42 \pm 0.31 \times 10^{-2}$ mL/g, respectively (**FIGS. 14A-14B**). Only LV/BW on POD 7 was significantly different from that at baseline ($P < 0.05$)

*VEGF Infusion Tolerance and Pharmacokinetics Studies*

One piglet underwent VEGF infusion at the dose of 400 mcg/kg. The infusion was started at a rate of 6.67 mcg/kg/min and resulted in a precipitous drop in mean arterial pressure (MAP) of 50%. The infusion was held, restarted at 3.33 mcg/kg/min, and subsequently increased to 5 mcg/kg/min. The infusion took 90 minutes to complete and the piglet displayed prolonged lethargy and anorexia for more than 8 hours after awakening from anesthesia.

Two piglets underwent infusion at the dose of 200 mcg/kg. One piglet tolerated the rate of 3.33 mcg/kg/min throughout the infusion and required 60 minutes to complete. The other piglet required a starting rate of 1.67 mcg/kg/min due to hypotension, which was subsequently increased back to 3.33 mcg/kg/min. This piglet required 80 minutes to complete the infusion.

Two piglets underwent infusion at the dose of 100 mcg/kg. One piglet tolerated the rate of 1.67 mcg/kg/min throughout the infusion and required 60 minutes to complete. The other piglet required a starting rate of 0.83 mcg/kg/min due to hypotension, which was subsequently increased back to 1.67 mcg/kg/min. This piglet required 70 minutes to complete the infusion.

The half-life of rhVEGF-165 at 400, 200, and 100 mcg/kg was 89.3, 107.9 ± 5.5, and 125.3 ± 19.2 minutes, respectively. The Vd was 126.2 mL/kg at 400 mcg/kg and increased to 147.1 ± 63.9 and 381.8 ± 69.9 mL/kg at 200 mcg/kg and 100 mcg/kg, respectively. Similarly, the Cl was 1.92 mL/min/kg at 400 mcg/kg and increased to 2.46 ± 0.90 and 6.10 ± 1.75 mL/min/kg at 200 mcg/kg and 100 mcg/kg, respectively (**Table 1**).

**Table 1. Pharmacokinetics properties of rhVEGF-165 in piglets at different doses of infusion**

| Animal ID (Dose) | Area under the curve (AUC) (ng/mL.min) | Half-life (min) | Volume of distribution (Vd) (mL/kg) | Rate of clearance (Cl) (mL/min/kg) |
|---|---|---|---|---|
| 959 (400 mcg/kg) | $2.08 \times 10^5$ | 89.3 | 126.2 | 1.92 |
| 960 (200 mcg/kg) | $1.28 \times 10^5$ | 113.4 | 83.1 | 1.56 |

(continued)

| Animal ID (Dose) | Area under the curve (AUC) (ng/mL.min) | Half-life (min) | Volume of distribution (Vd) (mL/kg) | Rate of clearance (Cl) (mL/min/kg) |
|---|---|---|---|---|
| 963 (200 mcg/kg) | $2.96 \times 10^4$ | 102.4 | 211.0 | 3.36 |
| 964 (100 mcg/kg) | $1.27 \times 10^4$ | 144.5 | 451.7 | 7.85 |
| 983 (100 mcg/kg) | $2.31 \times 10^4$ | 106.0 | 311.8 | 4.35 |

*Systemic Administration of rhVEGF in the Swine Model of Compensatory Lung Growth*

a. *Physiologic Studies:* Piglets in the VEGF group had a significantly higher LV/BW on POD 7 compared to those in the control group ($6.67 \pm 0.15 \times 10^{-2}$ mL/g vs $5.61 \pm 0.12 \times 10^{-2}$ mL/g, $P = 0.001$). There was no difference in liver, kidney, or spleen weights between the two experimental groups (**FIGS. 15A-15D**).

b. *Morphometric Studies:* Compared to the control group, piglets in the VEGF group had higher parenchymal volume ($5.23 \pm 0.05$ vs $4.21 \pm 0.03 \times 10^{-2}$ mL/g), alveolar volume ($3.44 \pm 0.08$ vs $2.85 \pm 0.07 \times 10^{-2}$ mL/g), septal surface area ($9.70 \pm 0.45$ vs $7.91 \pm 0.18$ $cm^2$/g), and alveolar count ($5.88 \pm 0.61$ vs $3.78 \pm 0.76 \times 10^8$) (**FIGS. 16A-16D**).

**EXAMPLE 4. Stability of rhVEGF in Bronchioalveolar Lavage (BAL) Fluid**

Experiments were preformed to determine the stability of VEGF in bronchioalveolar lavage fluid. Bronchoalveolar lavage is a medical procedure in which a bronchoscope is passed through the mouth or nose into the lungs and fluid is squirted into a small part of the lung and then collected for examination. As the mucus on the pulmonary epithelium and the surfactant on the alveoli have proteases and peptidases that can readily degrade administered peptides and proteins, it is important to determine the stability of VEGF in bronchioalveolar lavage fluid.

BAL fluid was obtained from an anesthetized piglet. rhVEGF was incubated in BAL fluid at 37°C. Isovolumetric aliquots were removed from BAL fluid that contains rhVEGF at 10 min, 20 min, 40 min, 1 hr, 2 hr, and 4 hr. Western Blot was performed using anti- human VEGF antibody. The results showed that VEGF is unexpectedly stable in BAL fluid (**FIGS. 17A and 17B**), and suggest that there is no need for development of pegylated version of VEGF.

**EXAMPLE 5. VEGF Administration in the Swine Model**

17 piglets were used to validate the effects of administering VEGF to piglets via infusion. Piglets on DOL (day of life) 5 were weighed between 2.3-2.7 kg. Left pneumonectomy was performed on DOL 11-12. 8 piglets in the VEGF group received daily infusion of VEGF via central line at 200 mcg/kg, and 9 piglets in the control group received daily infusion of saline. Euthanasia was performed on POD 7. The piglets were fed based on standard feeding region until euthanasia.

Total lung volume was measured immediately after inflation and after 2 days of fixation in 10% formalin. Point counting with sampling of central and peripheral section of all 3 right lung lobes was performed for 4 piglets in the control group and 5 piglets in the VEGF group.

The weight change of all piglets in the VEGF group and in the control group is shown in **FIG. 18. FIG. 19A** shows the total lung volume before formalin fixation. **FIG. 19B** shows the total lung volume after formalin fixation. The liver/body weight ratio, kidney/body weight ratio, spleen/body weight ratio, and heart/body weight ratio are shown in **FIGS. 20A-20D. FIGS. 21A-21D** show morphometric analyses, which include parenchymal volume/body weight ratio, alveolar volume/body weight ratio, septal surface area/body weight ratio, and alveolar count of the control and VEGF group.

**EXAMPLE 6. Heparin Impairs Compensatory Lung Growth by Sequestering VEGF into Extracellular Matrix and Reducing Mitogenic Signaling of VEGFR2**

Experiments were preformed to determine the effects of heparin on compensatory lung growth.

**Materials and Methods**

*Cell-Free VEGF Receptor Binding Assay*

Ninety-six well plates were coated with anti-Fc antibody (Jackson ImmunoResearch, West Grove, PA) at 4 μg/mL in phosphate-buffered saline (PBS) and incubated overnight at room temperature. Plates were washed five times with wash buffer (0.05% Tween-20 in PBS, pH 7.2-7.4) the next morning and blocked with blocking buffer (2% bovine serum albumin and 0.05% Tween-20 in PBS) for 1 hour at 37°C. After another round of washing, plates were incubated for 1 hour at room temperature with 9 mixtures of rhVEGF-165 (Shire, Lexington, MA) ranging from 0 - 160,000 pg/mL in rhVEGFR1-Fc or rhVEGFR2-Fc (R&D Systems, Minneapolis, MN) at 250 ng/mL and five concentrations of heparin (Hospira, Lake Forest, IL) ranging from 0 - 1 U/mL. For the heparin used in this study, one unit of activity was equivalent to 10 micrograms in mass. Plates were again washed and incubated with biotinylated goat anti-VEGF antibody (R&D Systems) prepared in blocking buffer at 100 ng/mL for 2 hours at room temperature. After the final round of washing, streptavidine-horseradish peroxidase (HRP) (R&D Systems, Minneapolis, MN) at 1:200 dilution was added and allowed to incubate for 20 minutes at room temperature, followed by the addition of substrate reagent A and B (R&D Systems, Minneapolis, MN) at a 1:1 ratio and another 15 minutes of incubation at room temperature. Substrate reaction was stopped with 2N sulfuric acid and the signal was visualized with a FLUOstar Omega microplate reader (BMG Labtech, Cary, NC) at 450 nm. For both VEGFR1 and VEGFR2 in each heparin concentration, the dissociative constant (Kd) with VEGF, defined as [VEGFR] × [VEGF]/[VEGFR-VEGF], was derived from the slope of the double reciprocal plot, 1/absorbance vs. 1/[VEGF].

*Cell-Based VEGF Activation and Proliferation Assay*

For the activation assay, HMVEC-L (Lonza, Basel, Switzerland) were plated at 70% confluence on plastic and Matrigel®-coated plates (Corning, Corning, NY) in basal medium (EBM-2 medium (Lonza, Basel, Switzerland) + 0.5% fetal bovine serum) and starved overnight at 37°C. Cells were washed after 12 hours and treated with basal medium, basal medium + 10 ng/mL rhVEGF-165 (Shire, Lexington, MA), or basal medium + 10 ng/mL rhVEGF-165 + 5 concentrations of heparin ranging from 0 - 1 U/mL for 5 minutes at 37°C. The medium was then removed and cells were quickly lysed on ice with 1× Laemmli buffer (Boston Bio Products, Ashland, MA). The supernatant was then analyzed for P-1175- VEGFR2 level with Western blot.

For proliferation assay, HMVEC-L were plated at 30% confluence on plastic and Matrigel®-coated plates in basal medium and starved overnight at 37°C. Cells were washed after 12 hours and treated with basal medium, basal medium + 10 ng/mL rhVEGF-165 (Shire, Lexington, MA), or basal medium + 10 ng/mL rhVEGF-165 + 5 concentrations of heparin ranging from 0 - 1 U/mL. After 3 days of incubation at 37°C, cell viability was assessed with Cell Counting Kit-8 (Dojindo Molecular Technologies, Rockville, MD) and colorimetric signal was detected with FLUOstar Omega microplate reader (BMG Labtech) at 450 and 650 nm.

*Surgical Procedure and Experimental Groups*

Eight-week old C57BL/6 male mice (Jackson Laboratories, Bar Harbor, ME) underwent left pneumonectomy. Mice were first anesthetized with 120-400 mg/kg of Avertin (Sigma, St. Louis, MO) via IP injection. The animal was then placed on its back and orotracheal intubation proceeded with gentle retraction of the tongue followed by direct visualization of the vocal cords via transillumination. Intubation was achieved by inserting an 18-gauge angiocatheter (Edwards Lifesciences, Charlton, MA) into the trachea over a guidewire. The animal was ventilated on room air with a rodent ventilator (HSE-HA Minivent, Harvard Apparatus, Holliston, MA) at 70 breaths/minute. The left chest was then shaved and sterilized with povidone-iodine and 70% ethanol. A skin incision was made along the left anterior axillary line, followed by a thoracotomy incision at the fifth intercostal space. The left lung was then gently delivered onto the operative field and ligated at the hilum with a 4-0 silk suture (Ethicon, Somerville, NJ). Skin closure was done with a running 4-0 nylon suture (Ethicon, Somerville, NJ) and the animal was resuscitated with 3 mL of warm normal saline via subcutaneous injection at the end of the procedure. Post-operative pain medication, buprenorphine, was given twice daily via subcutaneous injection for 72 hours.

Mice were randomized into four experimental groups: control, heparin, VEGF, and VEGF + heparin. Mice in the control group received 100 μL of normal saline via IP injection immediately after surgery and daily thereafter. Mice in the heparin group were systemically heparinized (Y. Li et al., "The effect of heparin administration in animal models of sepsis: a prospective study in Escherichia coli-challenged mice and a systematic review and metaregression analysis of published studies.," Crit. Care Med., vol. 39, no. 5, pp. 1104-12, May 2011). Briefly, mice received IP heparin at 500 units/kg/dose once immediately before surgery, followed by once every 12 hours for the first 48 hours after pneumonectomy and once every 24 hours for the next 48 hours. The VEGF group received 0.5 mg/kg of recombinant murine

$VEGF_{164}$ (GenScript, Piscataway, NJ) via IP injection. The VEGF + heparin group received both systemic heparinization and VEGF delivery in a similar fashion. Mice in all experimental groups were euthanized on post-operative day (POD) 4. All procedures were carried out according to the National Institutes of Health Guide for the Care and Use of Laboratory Animals and approved by the Institutional Animal Care and Use Committee at Boston Children's Hospital.

*Pulmonary Mechanical Studies*

Pulmonary mechanical measurements with the Flexivent® system (SCIREQ, Montreal, Canada) were done immediately before euthanasia on POD 4. After anesthetization with Avertin, a midline neck incision was made to expose the trachea, followed by tracheotomy and insertion of a 20-gauge hollow bore needle. The tracheostomy was then connected to the Flexivent® system. Dynamic elastance and compliance were measured with the single frequency forced oscillation technique, which registered the animal's response to an applied sinusoidal waveform. Inspiratory capacity was calculated from the pressure-volume loops. To normalize these parameters for the animal's size, inspiratory capacity and compliance were divided by body weight while elastance was multiplied by body weight.

*Organ Harvest, Mass and Volume Measurement*

Animals were euthanized upon disconnection from the Flexivent® system. Animals were weighed and the remaining right lung was removed en-bloc with the tracheobronchial tree. The lung was infused with formalin at 20-25 mmHg and total lung volume was measured with the water displacement method (W. Scherle, "A simple method for volumetry of organs in quantitative stereology.," Mikroskopie, vol. 26, no. 1, pp. 57-60, Jun. 1970). The trachea was then ligated and the specimen was placed in 10% formalin overnight at 4°C. The liver, spleen, and kidneys were harvested, weighed, and preserved in a similar fashion. All specimens were transferred into 70% ethanol after 24 hours of formalin fixation and subsequently embedded in paraffin for morphometric analyses. Lung volume and organ mass measurements were normalized for body weight.

Quantitative microscopy was performed on hematoxylin and eosin (H&E) stained lung sections based on the principles of stereology. Five lungs from each experimental group were represented by systematic uniform random sampling of 20-30 five-micron sections examined under light microscope at 200X magnification. Using a point and intersection counting technique, parenchymal volume, alveolar volume, septal volume, septal surface area, and mean septal thickness were calculated. Parenchyma was defined as the gas-exchange compartment of the lung, which included alveoli, terminal ducts, and alveolar septa, while airways, vessels, and pleura were classified as non-parenchyma. All volume and area measurements were normalized for body weight. Alveolar density was mathematically calculated from counting the number of alveolar transections on 10 randomly chosen fields at 400X magnification using the formula:

$$N = \frac{n^{3/2}}{1.382 \times \sqrt{p}}$$

where N is alveolar density, n is the number of alveolar transections, and p is the alveolar-to-parenchymal volume ratio (M. Ochs and C. Mühlfeld, "Quantitative microscopy of the lung: a problem-based approach. Part 1: basic principles of lung stereology.," Am. J. Physiol. Lung Cell. Mol. Physiol., vol. 305, no. 1, pp. L15-22, Jul. 2013).

*Western Blot and Heparanase Activity Assay*

Frozen lung tissue samples from the control and heparin groups were suspended in radioimmunoprecipitation assay (RIPA) buffer (Boston Bio Products, Ashland, MA) containing protease and phosphatase inhibitors (Thermo Fisher Scientific, Waltham, MA). Tissue samples were then homogenized and centrifuged for 15 minutes at 4°C and 14,000 rpm. The supernatants were collected and protein concentration was measured with the Bradford colorimetric assay (Bio-Rad Laboratories Inc, Hercules, CA). Forty micrograms of protein from each sample were mixed with 4x Laemmli buffer (Boston Bio Products, Ashland, MA), heated at 95°C for 5 minutes, and separated on a 4-12% Bis-Tris polyacrylamide gel (Thermo Fisher Scientific, Waltham, MA). The proteins were then transferred to a nitrocellulose membrane and blocked in a TBST solution (50 mM Tris-HCl - pH 7.4, 150 mM NaCl, and 0.1% Tween 20) containing 5% non-fat dry milk (Bio-Rad Laboratories Inc, Hercules, CA) for 1 hour. The membrane was incubated with primary antibodies prepared in blocking buffer overnight at 4°C. Primary antibodies included anti-VEGFR2, -P-1175-VEGFR2, -EGFR, -P-1068-EGFR (Cell Signaling Technology, Danvers, MA), -P-1214-VEGFR2 (EMD Millipore, Temecula, CA), -VEGF 120/164, -HB-EGF (R&D Systems, Minneapolis, MN) and -β-Actin (Sigma-Aldrich, St. Louis, MO). On the following day, the membrane was washed with TBST for 10 minutes x3 before incubation in secondary antibodies, horseradish peroxidase conjugated anti-rabbit IgG, anti-mouse IgG (Santa Cruz Biotechnology, Dallas, TX), anti-rat IgG (Jackson ImmunoResearch, West Grove, PA) or anti-sheep IgG (R&D Systems, Minneapolis, MN) for 1 hour at room temperature. The membrane was again washed with TBST for 10 minutes x3, followed by signal visualization with the enhanced chemiluminescence (ECL) reagents (Thermo Fisher Scientific, Waltham, MA).

Heparanase activity in lung tissue of the heparin and control group was assessed with the Heparan Degrading Enzyme Assay Kit (Takara Bio USA, Mountain View, CA). Briefly, total proteins were extracted from the frozen lung tissue according to the manufacturer's instructions and incubated with biotinylated heparan sulfate. The reaction mixture was then transferred to a different plate coated with immobilized FGF, which was designed to capture undegraded heparan sulfate. After avidin-peroxidase and substrate were added, colorimetric signal was detected at 450 nm.

*Quantitative Polymerase Chain Reaction (qPCR) Array of VEGFR2 Signaling*

Total RNA was extracted from lung tissues with the RNeasy Mini Kit (Qiagen, Hilden, Germany) according to the manufacturer's protocol. RNA concentration was determined with the NanoDrop 8000 Spectrophotometer (Thermo Fisher Scientific, Waltham, MA). Reverse transcription was performed with the $RT^2$ First Strand Kit (Qiagen, Hilden, Germany). Briefly, extracted RNA underwent genomic DNA elimination, followed by reverse transcription at 42°C for 15 minutes. Synthesized cDNA was then mixed with reaction reagents provided by the $RT^2$ Profiler PCR Array for VEGFR2 (Qiagen, Hilden, Germany). Amplification reactions were achieved with the StepOne Real-Time PCR Systems (Applied Biosystems, Foster City, CA). Analysis was performed on three biological replicates in the control and heparin groups. This array allowed for simultaneous comparison of 84 genes involved in the VEGFR2 signaling cascade. All target genes were normalized to the housekeeping gene β-Actin and their mRNA expression level was calculated using the $2^{\Delta\Delta Ct}$ method. Three lung specimens were used for each group, control vs heparin, and a difference in gene expression of more than 2-fold was considered significant.

*Statistical Analyses*

The dissociative constant (Kd) for the binding results was derived from the slope of the curve determined by regressing 1/OD on 1/[VEGF]. Since the data exhibited increasing variance with increasing levels of 1/[VEGF], a weighted least squares regression was used. The weight was calculated as the reciprocal of the sample variance for the outcome at each level of 1/[VEGF], such that levels with smaller variance were given greater weight in the regression model than those with larger variance. The slope for each of the 5 heparin levels was determined by including a 4 degrees of freedom (d.f.) interaction effect in the model for 1/[VEGF] (1 d.f.) with heparin (4 d.f.). All other outcomes were analyzed with analysis of variance. Results are reported as mean ± standard error, with contrasts among heparin levels adjusted for multiple comparisons. All tests of significance were two-sided. SAS version 9.4 (Cary, NC) was used to analyze the data and to produce the figures.

**Results**

*VEGF Binding Assay*

As Kd represents the ease at which a bond between two molecules is disrupted, smaller Kd indicates a stronger binding affinity. The Kd for $VEGF_{165}$-VEGFR1 binding was 8.14 ± 0.27 ng/mL. **FIGS. 22A-22B** shows the effects of heparin on $VEGF_{165}$ binding to VEGFR1 and VEGFR2. All experiments were done in quadruplicates. OD stands for optical density, and Kd stands for dissociative constant. Compared to no heparin, heparin at any concentration did not affect the binding affinity between VEGF165 and VEGFR1 ($P$=0.80; **FIG. 22A**). The Kd for $VEGF_{165}$-VEGFR2 binding was 1.49 ± 0.03 ng/mL. Compared to no heparin (Kd = 1.66 ± 0.05 ng/mL), the binding affinity between $VEGF_{165}$ and VEGFR2 was increased by the presence of heparin at 0.125 U/mL (Kd = 1.28 ± 0.05 ng/mL, $P$ < 0.0001), 0.5 U/mL (Kd = 1.49 ± 0.05 ng/mL, $P$ = 0.05), and 1 U/mL (Kd = 1.33 ± 0.05 ng/mL, $P$ < 0.0001) (**FIG. 22B**). Heparin at 0.125 U/mL resulted in the strongest $VEGF_{165}$-VEGFR2 binding, which was significantly better than that produced by heparin at 0.25 (1.69 ± 0.05 ng/mL, $P$ < 0.0001) and 0.5 U/mL ($P$ = 0.02).

*VEGF Activation and Proliferation Assay*

There was no difference in $VEGF_{165}$-induced activation of VEGFR2 in the presence of different concentrations of heparin when human lung microvascular endothelial cells (HMVEC-L) were grown on plastic plates (**FIG. 23A**). In the presence of Matrigel®, the addition of heparin at 0.25, 0.5, and 1 U/mL significantly impaired VEGFR2 activation compared to no heparin ($P$ = 0.02, 0.004, and 0.002, respectively) (**FIG. 23B**). In **FIG. 23A** and **FIG. 23B,** all experiments were done in duplicates.

On plastic plates, $VEGF_{165}$ at 10 ng/mL significantly increased HMVEC-L proliferation compared to the negative control (cells grown in the absence of VEGF and heparin) ($P$ = 0.0006) (**FIG. 23C**). Compared to $VEGF_{165}$ alone, the addition of heparin at 0.125, 0.25, 0.5, or 1 U/mL significantly decreased HMVEC-L density ($P$ = 0.001, 0.0005, 0.0005, and 0.0005, respectively). In **FIG. 23C,** all experiments were done in quadruplicates.

On Matrigel®-coated plates, $VEGF_{165}$ at 10 ng/mL similarly increased HMVEC-L proliferation compared to the negative control ($P$ = 0.01) (**FIG. 23D**). The addition of heparin at 0.125, 0.25, 0.5, and 1 U/mL again decreased $VEGF_{165}$'s mitogenic activity on HMVEC-L when compared to VEGF in the absence of heparin ($P$ = 0.04, 0.01, 0.003, and 0.001, respectively). In **FIG. 23D,** all experiments were done in quadruplicates.

*Lung Volume, Organ Weight, and Pulmonary Function Measurements*

Right lung volume, measured by the water displacement method and normalized for body weight, was significantly lower in the heparin group compared to the control group (4.9 ± 0.1 vs 5.4 ± 0.1 × $10^{-2}$ mL/g, $P$ = 0.04) (**FIG. 24A**). The VEGF group, on the other hand, displayed significantly higher lung volume compared to both the control and the heparin groups (5.8 ± 0.1 vs 5.4 ± 0.1 and 4.9 ± 0.1 × $10^{-2}$ mL/g, $P$ = 0.03 and < 0.0001, respectively). Compared to the heparin group, the combination of heparin and VEGF appeared to rescue lung volume (4.9 ± 0.1 vs 5.5 ± 0.2 × $10^{-2}$ mL/g, $P$ = 0.04). There was no difference in the weight of non-regenerating organs including the liver and kidney (**FIG. 24B**). There was also no difference in pulmonary function parameters including inspiratory capacity, dynamic compliance, or dynamic eslastance among the 4 experimental groups at 4 days after pneumonectomy (**FIG. 24C**).

*Morphometric Analyses*

Mice in the heparin group had significantly lower parenchymal volume compared to the control, VEGF, and heparin + VEGF groups (3.5 ± 0.2 vs 4.3 ± 0.2, 4.7 ± 0.2, and 4.2 ± 0.2 × $10^{-2}$ mL/g, $P$ = 0.03, 0.002, and 0.04, respectively) (**FIG. 25A**). Alveolar volume in the heparin group was also significantly lower in the heparin group compared to the control, VEGF, and heparin + VEGF groups (2.1 ± 0.1 vs 2.7 ± 0.1, 3.0 ± 0.1, and 2.8 ± 0.1 × $10^{-2}$ mL/g, $P$ = 0.01, 0.0006, and 0.002, respectively) (**FIG. 25B**). Similarly, the heparin group also displayed decreased septal surface area compared to the control, VEGF, and heparin + VEGF groups (14.1 ± 0.8 vs 17.2 ± 0.8, 19.0 ± 0.8, and 17.9 ± 0.8 $cm^2$/g, $P$ = 0.05, 0.004, and 0.02, respectively) (**FIG. 25C**). There was no difference in septal volume among the 4 groups (**FIG. 25D**). However, the mean septal thickness in the heparin group was the highest among all 4 experimental groups and significantly higher than that in the VEGF and heparin + VEGF groups (13.7 ± 0.8 vs 9.5 ± 0.8 and 9.0 ± 0.8 micron, $P$ = 0.01 and 0.006, respectively) (**FIG. 25E**). On alveolar counting, the heparin group again demonstrated reduced alveolar density compared to the control, VEGF, and heparin + VEGF groups (2.6 ± 0.1 vs 3.1 ± 0.1, 4.2 ± 0.1, and 3.4 ± 0.1 × $10^7$ per $cm^3$, $P$ = 0.03, $P$ < 0.0001, and $P$ = 0.002, respectively) (**FIG. 25F**). Additionally, the VEGF group also showed higher alveolar density compared to the control and heparin + VEGF groups ($P$ < 0.0001 and $P$ = 0.001, respectively).

*Western Blot and Heparanase Activity Assay*

Lung tissue level of $VEGF_{120/164}$ was significantly increased in the heparin group compared to the control group ($P$ = 0.0007) **(FIG. 26A).** Two versions of phosphorylated VEGFR2: P-Y1175-VEGFR2 and P-Y1214-VEGFR2 were analyzed. While tissue levels of P-Y1175-VEGFR2 were significantly reduced in the heparin group ($P$ = 0.05), P-Y1214-VEGFR2 levels were increased ($P$ = 0.03). There was no difference in the lung tissue activity of heparanase between the 2 groups **(FIG. 26B).**

*VEGFR2 Signaling Polymerase Chain Reaction (PCR) Array*

Compared to the control group, the heparin group displayed more than 2-fold reduction in the expression of specific groups of genes **(FIG. 27).** These included mitogen-activated protein kinases (*Map2k2, Mapk11, Mapk12,* and *Mapk13*), phospholipase A2 (*Pla2g12a, Pla2g2d, Pla2g3, Pla2g4a, Pla2g4b,* and *Pla2g5*), nuclear factor of activated T-cells (*Nfatc2* and *Nfatc4*), and protein phosphatase 3 (*Ppp3cb* and *Ppp3r1*). Additionally, there was also a significant reduction in the expression of *Hspb1, Nrp1, Pdgfc, Pik3r3, Prkcb, Shc2,* and *Vegfb* in mice that received heparin. Only Pik3r5 was significantly upregulated in the heparin group. Of note, there was no difference in the expression of *Vegfa* between the two groups.

**Table 2. List of genes displaying at least a 2-fold reduction in mRNA levels in the heparin group compared to the control group**

| Protein | Genes |
|---|---|
| **MAPK** | *Map2k2, Mapk11, Mapk12, and Mapk13* |
| **PLA2** | *Pla2g12a, Pla2g2d, Pla2g3, Pla2g4a, Pla2g4b, and Pla2g5* |
| **NFAT** | *Nfatc2 and Nfatc4* |
| **PPP3** | *Ppp3cb and Ppp3r1* |
| **Others** | *Hspb1, Nrp1, Pdgfc, Pik3r3, Prkcb, Shc2, and Vegfb* |

This example aimed to investigate the effects of heparin on lung growth in a model of compensatory lung growth (CLG). Mixed data suggest that heparin can impair angiogenesis and inhibit organ growth or regeneration. Compared to the absence of heparin, heparin at 0.25, 0.5 and 1 unit/mL decreased VEGF's activation of VEGFR2 (P = 0.02, 0.004 and 0.002, respectively) and mitogenic activity on human lung microvascular endothelial cells (HMVEC-L) (P = 0.01, 0.003, and 0.001, respectively) *in vitro.* Compared to the control group, heparinized mice displayed lower lung volume/body weight ($0.054 \pm 0.001$ vs. $0.049 \pm 0.001$ mL/g, *P* = 0.04), septal surface area ($17.2 \pm 0.8$ vs. $14.1 \pm 0.8$ $cm^2/g$, *P* = 0.05) and alveolar density ($3.1 \pm 0.1$ vs. $2.6 \pm 0.1 \times 10^7$ per $cm^3$, *P* = 0.03). Lungs of heparinized mice displayed increased VEGF tissue levels (*P* = 0.0007), reduced P-Y1175-VEGFR2 (*P* = 0.05), and increased P-Y1214-VEGFR2 (*P* = 0.03) compared to the control group. PCR array of VEGFR2 signaling cascade showed more than 2-fold reduction in mRNA levels of the mitogen-activated protein kinase (MAPK) genes in the heparin group compared to the control group. Results suggest that heparin impairs CLG by sequestering VEGF into lung tissue and reducing VEGFR2-mediated mitogenic signaling. These findings raise concern for the clinical use of heparin in the setting of organ growth or regeneration.

Heparin is one of the most widely used drugs in medicine. It currently remains the mainstay of anticoagulation therapy. Usually harvested from animal tissue such as porcine intestinal mucosa, heparin confers its anticoagulation activity by binding to antithrombin III (AT), a protease that cleaves and deactivates thrombin and activated factor X. Beyond its well-known roles as an anticoagulant, heparin and its closely related polysaccharide, heparan sulfate (HS), belong to a group of glycosaminoglycans (GAG) that are ubiquitously present in cellular biology and exert a wide range of activities that span the fields of embryology, skeletal development, inflammation, wound healing, tumor metastasis, and angiogenesis. Due to their highly similar structures, distinguishing the properties of heparin from HS or vice versa is not always straightforward. Perhaps the most simplistic definition is to characterize heparin as a mast cell-derived GAG while HS comprises all other structurally similar polysaccharides that are either present in the extracellular matrix (ECM) or attached to the cell surface. The synthesis and production of heparin and HS occur both in the endoplasmic reticulum and Golgi apparatus where they undergo three main phases: attachment of the GAG chain to the core protein, chain polymerization, and chain modifications. Each molecule of heparin is thus constructed by the repeated addition of D-glucuronic acid (1 - 4) N-acetyl-D-glucosamine disaccharide units onto a core protein, followed by epimerization of glucuronate to iduronate and sulfation at specific 2-O and 6-O regions. These GAG chains are then released from the proteoglycan in the form of free heparin via the action of the enzyme β-endo glucuronidase. In general, this process of post-translational modifications are less complete and uniform in HS, resulting in a much more heterogeneous group of polysaccharides characterized by lower iduronate content, less sulfation at the 2-O and 6-O positions, and clustering of the sulfate groups. It is this heterogeneity in molecular structure that confers HS the ability to interact with a wide variety of ligands such as adhesion molecules, cytokines, coagulation enzymes, and growth factors.

The ability of heparin and HS to interact with major angiogenic growth factors has been well documented in the literature. Members of the fibroblast growth factor (FGF) family depend on HS to form a stable ternary complex with their receptors FGFR (M. Presta, et al., "Fibroblast growth factor/fibroblast growth factor receptor system in angiogenesis," Cytokine Growth Factor Rev., vol. 16, no. 2, pp. 159-178, Apr. 2005; S. Guimond, et al., "Activating and inhibitory heparin sequences for FGF-2 (basic FGF). Distinct requirements for FGF-1, FGF-2, and FGF-4.," J. Biol. Chem., vol. 268, no. 32, pp. 23906-14, Nov. 1993). HS in the ECM serves as an important storage site for FGF; therefore ECM degradation leads to an increase in the bioavailability of FGF and its angiogenic activities. Different forms of exogenous heparin, from various sources and with different chemical features, can act as an antagonist by binding and sequestering FGF, disrupting its interaction with FGFR (D. Coltrini et al., "Different effects of mucosal, bovine lung and chemically modified heparin on selected biological properties of basic fibroblast growth factor.," Biochem. J., pp. 583-90, Oct. 1994). Vascular endothelial growth factor (VEGF), the master regulator of angiogenesis, is another family of growth factors that possess a heparin-binding domain (HBD). In a similar fashion to FGF, cell membrane-associated HS plays an important role in potentiating the binding between VEGF and its receptors while exogenous heparin, especially at a lower molecular weight, has been shown to inhibit VEGF's angiogenic effects on endothelial cells. Furthermore, high concentration of exogenous heparin can inhibit the binding between VEGF and its main receptor, VEGFR2, as well as reduce the receptor's phosphorylation, an effect that is dependent on proper sulfation at the O positions.

In addition to their well-studied roles in tumor growth and metastasis, angiogenic growth factors also significantly contribute significantly to other physiologic processes, such as organ regeneration. Compensatory lung growth (CLG) after unilateral pneumonectomy is another process that is highly dependent on VEGF. Activation of VEGFR2 has been shown to be pivotal in CLG as mice with endothelial cell-specific *Vegfr2* knock-down display impaired restoration of alveolar structure and functions following unilateral pneumonectomy. VEGF shows promise as a potential therapy for pulmonary hypoplasia associated with conditions such as congenital diaphragmatic hernia (CDH). Neonates with CDH who have severe pulmonary hypoplasia and hypertension often require cardiorespiratory support in the form of extracorporeal membrane oxygenation (ECMO) while awaiting for definitive surgical treatment. The cannulation of major vessels to establish the ECMO circuit requires the use of systemic anticoagulants, i.e. heparin, to avoid thrombotic complications. Given the interactions between heparin and multiple angiogenic growth factors including VEGF, systemic heparinization will have a significant impact on physiologic processes such as organ growth and regeneration. Additionally, in order to develop VEGF into a therapy for pulmonary hypoplasia, the effect of systemic heparinization on its efficacy needs to be understood.

It is evident that the complex interactions between heparin/HS and VEGF has a wide range of effects on angiogenesis-dependent physiologic processes. In a larger context, lung growth in patients with CDH and associated pulmonary hypoplasia, many of whom require ECMO and systemic anticoagulation (P. S. Puligandla et al., "Management of congenital diaphragmatic hernia: A systematic review from the APSA outcomes and evidence based practice committee," J. Pediatr. Surg., vol. 50, no. 11, pp. 1958-1970, Nov. 2015), could potentially be affected by the administration of heparin. For this reason, *in vitro* assays in the present example were conducted with heparin concentrations ranging from 0 to 1 U/mL, which contains the target clinical range of heparin used in patients on ECMO, e.g., 0.3-0.6 U/mL.

In fact, there exist conflicting data on how the presence of heparin can modulate the binding between $VEGF_{165}$ and its receptors. In a cell-free environment, it has been shown that heparin at low concentrations (0.1-1 μg/mL) potentiate the binding between $^{125}I$-$VEGF_{165}$ and VEGFR2 (S. Tessler et al., "Heparin modulates the interaction of VEGF165 with soluble and cell associated flk-1 receptors.," J. Biol. Chem., vol. 269, no. 17, pp. 12456-61, Apr. 1994). Using surface plasmon resonance technology, Wijelath et. al. demonstrated that heparin at 1 μg/mL resulted in the strongest binding between $VEGF_{165}$ and VEGFR2 (E. Wijelath et al., "Multiple mechanisms for exogenous heparin modulation of vascular endothelial growth factor activity," J. Cell. Biochem., vol. 111, no. 2, pp. 461-468, Jun. 2010). However, such binding in cell-based assays has proven to be much more variable. While the binding of $^{125}I$-$VEGF_{165}$ to NIH-3T3 cells transfected with a chimeric VEGFR2 or aortic arch-derived bovine endothelial cells (ABAE) is inhibited by various concentrations of heparin (S. Tessler et al., "Heparin modulates the interaction of VEGF165 with soluble and cell associated flk-1 receptors.," J. Biol. Chem., vol. 269, no. 17, pp. 12456-61, Apr. 1994), in the present study, it has been demonstrated that heparin in fact potentiated the binding between $VEGF_{165}$ and VEGFR2 in a cell-free environment, especially at either a relatively low (0.125 U/mL) or high (1 U/mL) concentration. However, an opposite effect was observed in cell-based assays where heparin was shown to impair the $VEGF_{165}$-induced activation of VEGFR2 on HMVEC-L. It should be noted that the decrease in activation was most prominent when cells were grown in Matrigel®, suggesting that the presence of ECM plays a further role in modulating the interactions between heparin and VEGF. It should also be noted that the presence of heparin only affected the binding between $VEGF_{165}$ and VEGFR2 while no significant changes were observed with regard to VEGFR1 binding. In another study, it has been shown that although heparin decreases the binding between $VEGF_{165}$ and VEGFR1, the remaining interactions result in stronger phosphorylation and thus preserve the receptor's functions (N. Ito and L. Claesson-Welsh, "Dual effects of heparin on VEGF binding to VEGF receptor-1 and transduction of biological responses.," Angiogenesis, vol. 3, no. 2, pp. 159-66, 1999). While some view VEGFR1 as simply a decoy receptor that serves to modulate the activity of VEGF, it is known to be vital to embryologic development yet its exact roles in angiogenesis remain disputed (S. Koch, S. Tugues, X. Li, L. Gualandi, and L. Claesson-Welsh, "Signal transduction by vascular endothelial growth factor receptors.," Biochem. J., vol. 437, no. 2, pp. 169-83, Jul. 2011).

Many studies have demonstrated the inhibitory effects of heparin on endothelial cell proliferation *in vitro*. Results from the *in vitro* assays suggested that systemic administration of heparin *in vivo* could negatively affect angiogenesis-dependent processes, especially those that have been shown to be heavily dependent on VEGF signaling such as CLG.

To the best of the knowledge, the present study was the first of its kind to explore these anti-angiogenic effects in a model of organ growth and regeneration. It has been demonstrated that the administration of heparin impaired CLG by decreasing both total lung volume as well as various morphometric parameters. Heparinized mice displayed decreased parenchymal volume, alveolar volume, and alveolar septal surface area, all of which suggest a reduced capacity for gas exchange. Furthermore, the mean septal thickness in heparinized mice had the tendency to be higher than that measured in the other groups. This feature indicates an arrest in alveolar development, as seen in diseases such as bronchopulmonary dysplasia (BPD). As expected, systemic heparin indeed diminished both alveolar density and total alveolar count, further supporting the antagonistic effects of heparin on alveolar development and providing additional evidence for its anti-angiogenic properties in a new animal model. The combination of heparin and VEGF seemed to alleviate the deleterious effects of heparin and restore normal lung growth although compared to VEGF alone, the combined therapies still resulted in decreased alveolar count. No differences in pulmonary function tests were observed among the experimental groups at 4 days after pneumonectomy, possibly because the lungs were still in the proliferative phase and a longer period of time would be required for any changes in pulmonary mechanics to manifest.

To understand the mechanism of heparin in impairing CLG, its effects on tissue heparanase activity were studied. Heparanase, which cleaves heparin and HS at specific sites, is known to be upregulated by certain tumors and implicated in tumor invasion and metastasis by releasing a myriad of pro-angiogenic factors such as FGF and VEGF from the ECM (L. Fux, N. Ilan, R. D. Sanderson, and I. Vlodavsky, "Heparanase: busy at the cell surface," Trends Biochem. Sci., vol. 34, no. 10, pp. 511-519, Oct. 2009). Parts of heparin's anti-angiogenic effects have been attributed to its ability to bind and antagonize heparanase. In this example, it has been demonstrated that the level of tissue heparanase activity was unchanged between the control and heparin group, suggesting that heparanase was not involved in mediating the effects of heparin on lung growth. The tissue levels of VEGF, VEGFR2, and P-VEGFR2 were also analzyed. Although there was an increase in the tissue level of $VEGF_{120/164}$, P-VEGFR2 at the Y1175 position was decreased. Phosphorylation at the Y1175 position is a well-known initiating event that triggers the MAPK/ERK pathway of cellular proliferation (T. Takahashi, S. Yamaguchi, K. Chida, and M. Shibuya, "A single autophosphorylation site on KDR/Flk-1 is essential for VEGF-A-dependent activation of PLC-gamma and DNA synthesis in vascular endothelial cells," EMBO J., vol. 20, no. 11, pp. 2768-2778, Jun. 2001). Concurrently, there was an increase in phosphorylation at the Y1214 position, a less characterized site of VEGFR2 thought to be involved in migration pathways. Since there was no evidence that VEGF was upregulated via quantitative PCR (qPCR), these findings suggested that systemic heparin resulted in tissue sequestration of VEGF and a shift of VEGFR2 activation from pro-proliferation to pro-migration pathways. Indirect evidence of heparin-induced sequestration of VEGF has been shown in a human study where a single dose of heparin was sufficient to significantly decrease the serum level of VEGF after just 10 minutes of infusion (M. A. East, D. I. Landis, M. A. Thompson, and B. H. Annex, "Effect of single dose of intravenous heparin on plasma levels of angiogenic growth factors.," Am. J. Cardiol., vol. 91, no. 10, pp. 1234-6, May 2003). Using computational biology, Clegg et. al. showed a similar phenomenon where compared to $VEGF_{121\ and\ 165}$, which primarily signal through the Y1175 site, $VEGF_{189}$ favored phosphorylation at the Y1214 site due to its strong association with the ECM and resultant effects on receptor recycling (L. W. Clegg and F. Mac Gabhann, "Site-Specific Phosphorylation of VEGFR2 Is Mediated by Receptor Trafficking: Insights from a Computational Model.," PLoS Comput. Biol., vol. 11, no. 6, p. e1004158, Jun. 2015). By sequestering VEGF into the ECM, heparin potentially caused $VEGF_{165}$ to behave in a more similar fashion to $VEGF_{189}$, with a consequential reduction in mitogenic activity.

In order to further confirm the effects of heparin on VEGF signal transduction, qPCR array of the VEGFR2 signaling cascade was performed. Compared to the control group, the heparin group displayed more than a 2-fold reduction in the expression of three main groups of downstream mediators: mitogen-activated protein kinases (MAPK), phospholipase A2 (PLA2), and nuclear factor of activated T cells (NFAT) and their associated genes, protein phosphatase 3 (PPP3). Activation of VEGFR2 triggers a series of pathways that promote endothelial cell proliferation, survival, migration, and permeability. Of note, auto-phosphorylation at the Y1175 position has been shown to be a key event in survival and endothelial cell proliferation as mice with knock-in mutation from tyrosine to phenylalanine died *in utero* in a similar fashion to *Vegfr2* knock-out mice. One of the major early mediators of P-Y1175-VEGFR2 is phospholipase C$\gamma$ (PLC$\gamma$). Activation of PLC$\gamma$ leads to $Ca^{2+}$ influx as well as inositol 3,4,5 triphosphate and diacylglycerol release with subsequent phosphorylation of MAPK and endothelial cell proliferation (T. Takahashi, et al., "A single autophosphorylation site on KDR/Flk-1 is essential for VEGF-A-dependent activation of PLC-gamma and DNA synthesis in vascular endothelial cells," EMBO J., vol. 20, no. 11, pp. 2768-2778, Jun. 2001). PLA2, a downstream effector of MAPK, plays a key role in regulating the cyclooxygenase 2 (COX2) pathway and subsequent eicosanoid release (J. Xu et al., "Role of PKC and MAPK in cytosolic PLA2 phosphorylation and arachadonic acid release in primary murine astrocytes.," J. Neurochem., vol. 83, no. 2, pp. 259-70, Oct. 2002). Its activity is dependent on $Ca^{2+}$ influx and phosphorylation by MAPK (X. Zhu et al., "IL-5-induced integrin adhesion of human eosinophils caused by ERK1/2-mediated activation of cPLA2.," J. Leukoc. Biol., vol. 72, no. 5, pp. 1046-53, Nov. 2002). NFAT, on the other hand, is under the control of PLC$\gamma$ through a calcineurin-dependent mechanism. Activation of this transcription factor triggers an inflammatory response as well as nitric oxide production (E. Hofer and B. Schweighofer, "Signal transduction induced in endothelial cells by growth factor receptors involved in angiogenesis.," Thromb. Haemost., vol. 97, no. 3, pp. 355-63, Mar. 2007). Downregulation of the MAPK, PLA2, and NFAT genes as shown on qPCR array further confirmed the reduction in activation of VEGFR2 at the Y1175 position. Interestingly, a similar decrease in expression of PLCy in the heparin group on POD 4 has not been observed. As PLCy is an upstream effector of VEGFR2 signaling, it is possible that differences in its expression could only be seen at earlier time points after pneumonectomy.

Heparin remains one of the most widely used therapies for anti-coagulation today. In 2008, more than 100 deaths in the US were attributed to inadequate purification processes that resulted in heparin contaminated with oversulfated products. According to the Extracorporeal Life Support Organization's Registry 2016 report, 37% of patients on ECMO were neonates with respiratory failure, among whom CDH remained one of the major diagnoses (R. R. Thiagarajan et al., "Extracorporeal Life Support Organization Registry International Report 2016," ASAIO J., vol. 63, no. 1, pp. 60-67, 2017). Systemic heparinization is a major contributor of complications in these patients as the occurrence of central nervous system, surgical, pulmonary, and cannula hemorrhage continue to pose significant risk to the patient and treatment challenges. Results from this study raised further concern with regard to the clinical use of heparin in neonates with respiratory failure as it could impair lung growth via interfering with VEGF signaling. In recent years, due to the risk of heparin-induced thrombocytopenia and heparin resistance, direct thombin inhibitors (DTI), i.e. argatroban and bivalirudin, have emerged as a safe alternative for heparin in ECMO anticoagulation. Thus, because of the potential inhibitory effects of heparin on lung growth, the need to transition to anticoagulation alternatives that are not anti-angiogenic should merit more serious consideration.

**EXAMPLE 7. Intranasal delivery of VEGF enhances compensatory lung growth in mice**

Experiments were performed to determine the efficacy of administering exogenous murine VEGF via nasal delivery compared to systemic administration, specifically intraperitoneal (ip) injection.

**Methods**

*Experimental Groups and Surgical Procedure*

Eight to ten week old C57BL/6 male mice (Jackson Laboratories, Bar Harbor, ME) were randomized into control or VEGF experimental groups. Prior to left pneumonectomy, VEGF groups received 0.5 mg/kg of recombinant murine VEGF-164 (GenScript, Piscataway, NJ) (SEQ ID NO: 1) either via intraperitoneal (ip) injection (N= 4) or nasal instillation (N = 4). Mice in control group for ip injection (N= 4) received saline in place of drug and mice in control group for nasal instillation (N= 4) received saline in place of drug. For nasal instillation, mice were sedated with isoflurane until a regular respiratory rate of 50-70 breaths/min was achieved. The mouse was held upright and a droplet of concentrated VEGF164 (200 μg/mL) was pipetted onto its nose at a volume dose of 2.5 μL/g.

Animals were anesthetized with 120-400 mg/kg of Avertin (Sigma, St. Louis, MO) via intraperitoneal injection and orotracheally intubated. After the left chest was prepared with ethanol and iodine solution, a skin incision was made along the left anterior axillary line. The left lung was accessed via a thoracotomy incision at the fifth intercostal space and the hilum was ligated with a 4-0 silk suture (Ethicon, Somerville, NJ). Thoracotomy and skin incisions were closed with running PDS® (Ethicon, Somerville, NJ), followed by resuscitation with 3 mL of subcutaneous warm normal saline. Buprenorphine was administered post-operatively twice daily via subcutaneous injection for 72 hours. Following a left PNX, mice were randomized into either the control or VEGF group. The VEGF group received 0.5 mg/kg/day of VEGF 164 via nasal instillation while control mice received an isovolumetric volume of normal saline. All procedures were carried out according to the National Institutes of Health Guide for the Care and Use of Laboratory Animals and approved by the Institutional Animal Care and Use Committee at Boston Children's Hospital.

*Assessment of Plasma VEGF Levels*

To quantify the amount of systemic absorption of VEGF with each route of administration, blood collection was performed via retro-orbital puncture 5 minutes after VEGF administration for the baseline timepoint, and repeated at 30, 120, and 240 minutes. Blood was collected in EDTA-containing collection tubes and plasma was separated by centrifugation at 2000 g and 4 °C for 15 minutes. Plasma VEGF levels were measured with an enzyme-linked immunosorbent assay (ELISA) (R&D Systems, Minneapolis, MN) according to the manufacturer's protocol.

*Organ Harvest, Mass and Volume Measurement*

Animals were euthanized on POD 4 upon disconnection from the Flexivent® system. Animals were weighed and the remaining right lung was removed en-bloc with the tracheobronchial tree. Specimens for morphometric analysis were inflated with 10% formalin at 30 cmH2O via the previously intubated 18-gauge needle and total lung volume was measured with the water displacement method (N= 10 mice in each group). Of note, 26 mice (13 in each group) underwent functional studies, yet lung volume measurement was only performed in 20 animals (10 in each group). The remaining lung specimens were inadvertently injured during organ harvest, which precluded formalin inflation. No lung specimens were excluded for any other reason. The trachea was then ligated with a 4-0 silk suture to maintain alveolar distension and the specimen was placed in 10% formalin overnight at 4 °C. All specimens were subsequently embedded in paraffin for histologic analyses. Lung volume and organ mass measurements were normalized for body weight. Lung specimens for RNA/protein analysis were immediately flash frozen in liquid nitrogen for future tissue analysis. Lung volume was similarly measured on POD 10 (N = 5 in each group).

*Morphometric Analyses*

Quantitative microscopy was performed on hematoxylin and eosin (H&E) stained lung sections based on the principles of stereology. Five lung specimens from each group harvested on POD 4 were randomly selected for morphometric analyses. One 5-micron section from each specimen was examined at 200X magnification using the principle of systemic uniform random sampling: the first lung field on each section was selected randomly, followed by systemic sampling of every other field to achieve a total of 40-50 lung fields per section. For each field, a 42-point test lattice with predefined grid line length was used to facilitate point and intersection counting. Point counting allowed for estimation of parenchymal volume, alveolar volume, alveolar to parenchymal volume ratio, and septal volume while intersection counting facilitated the measurement of septal surface area and mean septal thickness. Parenchyma was defined as the gas-exchange compartment of the lung, which included alveoli, terminal ducts, and alveolar septa, while airways, vessels, and pleura were classified as non-parenchyma. All volume and area measurements were normalized for body weight. Under the assumptions that alveolar units assume a spherical shape and are randomly distributed throughout the lung parenchyma, alveolar density was mathematically calculated from counting the number of alveolar transections on randomly chosen lung fields using the method as described in a previous Example that is described in Weibel ER, Gomez DM. A principle for counting tissue structures on random sections. J. Appl. Physiol. 17, 343-8 (1962).

*Quantitative Real-Time Polymerase Chain Reaction (qPCR)*

Total RNA was extracted from lung tissues with the RNeasy Mini Kit (Qiagen, Hilden, Germany) according to the manufacturer's protocol. RNA concentration was determined with the NanoDrop 8000 Spectrophotometer (Thermo Fisher Scientific, Waltham, MA). Amplification reactions were performed with the StepOne Real-Time PCR Systems (Applied Biosystems, Foster City, CA) using pre-designed and validated TaqMan® primers for VEGF receptor (VEGFR) 1, VEGFR2, neuropilin (NRP) 1, VEGF-A, epidermal growth factor (EGF), and EGF receptor (EGFR) (Applied Biosystems, Foster City, CA). All target genes were normalized to the housekeeping gene GAPDH and their mRNA expression level was calculated using the $2^{\Delta\Delta Ct}$ method. Three biological replicates from each control group and four from each VEGF group were used for the reactions.

*Western Blot and Enzyme-Linked Immunosorbent Assay (ELISA)*

Lung tissue samples from animals euthanized on POD 4 were immediately suspended in radio-immunoprecipitation assay (RIPA) buffer (Boston Bio Products, Ashland, MA) containing protease and phosphatase inhibitors (Thermo Fisher Scientific, Waltham, MA). Tissue samples were then homogenized in a Bullet Blender (Next Advance Inc., Averill Park, NY) and centrifuged for 15 minutes at 4 °C and 14,000 rpm. The supernatants were collected and protein concentration was measured with the Bradford colorimetric assay (Bio-Rad Laboratories Inc, Hercules, CA). Forty micrograms of protein from each sample were mixed with 4x Laemmli buffer (Boston Bio Products, Ashland, MA), heated at 95oC for 5 minutes, and separated on a 4-12% Bis-Tris polyacrylamide gel (Thermo Fisher Scientific, Waltham, MA). The proteins were then transferred to a nitrocellulose membrane and blocked in a TBST solution (50 mM Tris-HCl - pH 7.4, 150 mM NaCl, and 0.1% Tween 20) containing 5% non-fat dry milk (Bio-Rad Laboratories Inc, Hercules, CA) for 1 hour. Incubation with primary antibodies diluted in blocking buffer was done overnight at 4 °C. Primary antibodies included anti-P-VEGFR2, -VEGFR2, -P-EGFR, -EGFR (Cell Signaling Technology, Danvers, MA), - HB-EGF (R&D Systems, Minneapolis, MN), and β-Actin (Sigma-Aldrich, St. Louis, MO). On the following day, the membrane was washed with TBST for 10 minutes x3 before incubation in secondary antibodies, horseradish peroxidase conjugated goat anti-rabbit, anti-mouse (Santa Cruz Biotechnology, Dallas, TX), or anti-rat IgG (R&D Systems, Minneapolis, MN) antibodies for 1 hour at room temperature. The membrane was again washed with TBST for 10 minutes x3. The membrane was developed with enhanced chemiluminescence (ECL) reagents (Thermo Fisher Scientific, Waltham, MA) and the signal was visualized using ChemiDoc Touch (BioRad, Hercules, CA). Three biological replicates from each control group and four from each VEGF group were analyzed. followed by signal visualization with the enhanced chemiluminescence (ECL) reagents (Thermo Fisher Scientific, Waltham, MA).

Tissue levels of pulmonary VEGF on POD 4 were determined with ELISA (R&D Systems, Minneapolis, MN) according to the manufacturer's protocol. Three biological replicates were used for each experimental group and VEGF concentration was normalized against total protein concentration.

*Immunohistochemistry (IHC)*

Immunohistochemistry (IHC) was performed on formalin-fixed, paraffinembedded lung sections dried in an oven at 58 °C. All sections were deparaffinized with xylene and progressively rehydrated in various concentrations of ethanol. Heat-induced epitope retrieval was achieved with a citrate-based unmasking solution (Vector Laboratories, Burlingame, CA) at 120 °C in a pressurized chamber (Decloaking Chamber™, Biocare Medical, Pacheco, CA). Slides were washed with PBST (phosphate-buffered saline with 0.5% Triton-X) for 10 minutes x3, followed by 30 minutes of incubation in blocking solution (PBST containing 1% bovine-serum albumin). Incubation with primary antibodies was done overnight at 4 °C. Primary antibodies were prepared in blocking solution and included rat anti-Ki67 (Invitrogen, Carlsbad, CA) and rabbit anti-ERG (Abcam, Cambridge, MA) antibodies. ERG is a nuclear marker of endothelial cells and was used to facilitate cell counting. On the next day, slides were again washed with PBST for 30 minutes x3, followed by incubation in secondary antibodies, Alexa Fluorconjugated donkey anti-rat (Abcam, Cambridge, MA) and anti-rabbit (Invitrogen, Carlsbad, CA) IgG antibodies. DAPI counterstaining was performed for 5 minutes. Slides were washed again with PBST, dried, and mounted.

Sections of control and VEGF-treated lungs harvested on POD 2 (N = 5 in each group) and on POD 4 (N = 4 in each control group and N = 5 in each VEGF group) were examined with a confocal microscope (LSM 800, Zeiss, Jena, Germany) at 10X and 20X magnification. For each specimen, cell counting was performed on four random fields sampled across the entire right lung at 10X magnification. Endothelial cells were quantified with ImageJ by counting ERG-positive cells. Proliferating endothelial cells, marked by double staining with ERG and Ki67, were counted manually. Percent proliferating endothelial cells was calculated by normalizing the number of proliferating endothelial cells against total endothelial cells.

*Statistical Analysis*

The area under the curve (AUC) was calculated from the curves generated from plasma levels of VEGF at different time points following intranasal or ip administration of exogenous murine VEGF or normal saline. Comparison of AUC between the two delivery methods was achieved with a two-sided Student's t-test. Lung volume adjusted for body weight was analyzed with a two-way analysis of variance (ANOVA) using Sidek correction for multiple comparisons. Tests of significance for pulmonary function tests, morphometric analyses, qPCR, ELISA, and immunoblot between the control and VEGF groups were performed with two-sided Student's t-test. A paired t-test was used to analyze pre- and post-treadmill parameters on physical activity assessments. Comparisons of cell density at various time points on proliferation assays was achieved with multiple t-tests and Sidak-Bonferroni correction for multiple comparisons. For HB-EGF neutralization assays, a one-way ANOVA with Dunnett correction for multiple comparisons was used to compare cell density of different conditions to +VEGF/-Ab. A P value of < 0.05 was considered statistically significant. Results are presented as mean ± standard error (SE). All analyses were performed with GraphPAD Prism v.7.

*Proliferation Assay*

Human lung microvascular endothelial cells (HMVEC-L) and human bronchial epithelial cells (HBEC) (ATCC, Manasses, VA) were cultured under aseptic technique at 37 °C. First, HBEC were grown to 50% confluence on a 24-well plate, followed by 24-hour starvation in airway epithelial cell basal medium (ATCC, Manassas, Virginia) with human serum albumin 500 μg/mL, linoleic acid 0.6 μM, lecithin 0.6 μg/ mL, and L-glutamine 6 mM. Half of the wells were then treated with VEGF 165 at 10 ng/mL while the other half received no treatment. Cell viability was assessed at baseline and every 24 hours for 72 hours with the WST-8 cell counting kit (Sigma Aldrich, St. Louis, MO). The assay was done in quadruplicates. Subsequently, co-culture of HMVEC-L and HBEC was performed with Transwell® permeable inserts (Corning, Corning, NY) containing 0.4 μm pores. HMVEC-L were plated on the inserts and expanded until confluence before being starved for 24 hours in endothelial basal medium 2 (EBM-2) (Lonza, Allendale, NJ) + 0.5% fetal bovine serum (FBS). Meanwhile, HBEC were expanded to 50% confluence and starved. On the day of treatment, half of HMVEC-L-covered inserts were treated with VEGF 165 at 10 ng/mL. The other half was the control and received no treatment. The inserts were subsequently transferred to the 24-well plate containing HBEC. Cell viability was assessed at baseline and every 24 hours for 72 hours. The assay was done in quadruplicates.

*Angiogenesis and Protease Array*

Human pulmonary microvascular endothelial cells (HMVEC-L) (Lonza, Allendale, NJ) were lysed on ice with Laemmli buffer after 5 minutes of treatment with human VEGF165 (GenScript, Piscataway, NJ) at 10 ng/mL and activation of VEGFR2 was confirmed with immunoblot for P-VEGFR2. Once activation was confirmed, HMVEC-L were treated without (control) or with VEGF165 for 8 hours. Protein content of conditioned media from control and VEGF-treated HMVEC-L were analyzed with a Proteome ProfilerTM Angiogenesis Array (R&D Systems, Minneapolis, MN) while cell lysate was characterized with a Protease Array (R&D Systems) according to the manufacturer's protocol. Briefly, nitrocellulose membranes embedded with capture antibodies were blocked for 1 hour prior to the addition of conditioned medium or cell lysate samples. Samples were incubated with an antibody cocktail for 1 hour at room temperature before being applied on the membranes for overnight incubation at 4°C. After conjugation with streptavidin-HRP, signals were developed with a hydrogen peroxide and luminol-based reagent mix and quantified using ImageJ (NIH, Bethesda, MD).

*HB-EGF Neutralization Assay*

The proliferation assay was repeated with the addition of an HB-EGF neutralization antibody (R&D Systems). VEGF (10 ng/mL) and HB-EGF neutralizing antibody were separately added to chambers containing HMVEC-L and HBEC, respectively. Five conditions were tested: +VEGF/-antibody (Ab), -VEGF/+Ab 5 μg/mL, +VEGF/+Ab 0.5 μg/ mL, +VEGF/+Ab 5 μg/ mL, and +VEGF/+Ab 50 μg/ mL. Cell viability was assessed after 24 hours of treatment. The assay was done in biological quadruplicate. To confirm the neutralizing effect of HB-EGF antibody, HBEC were lysed with Laemmli buffer after 24 hours of treatment and the degree of EGFR activation was assessed with immunoblot of P-EGFR and EGFR (Cell Signaling Technology). Two treatment conditions were compared, +VEGF/-Ab and +VEGF/+Ab 5 μg/mL. The experiment was done in quadruplicates.

**Results**

To compare the degree of VEGF absorption between ip injection and nasal instillation, plasma VEGF concentration was measured at different time points after administration. While there was a low and steady level of VEGF detected in the plasma after nasal instillation, ip injection resulted in a spike of VEGF concentration at 30 minutes, followed by a rapid decline (FIG. 28A). Overall, the total AUC with ip injection was almost 70 times higher than that of nasal instillation (3529 $\pm$ 830 vs 51 $\pm$ 16 min.ng/mL, P = 0.003).

FIGS. 28A-28H show the results of lung volume and functional assessments. Nasal instillation resulted in less systemic absorption of VEGF compared to intraperitoneal injection as measured by enzyme-linked immunosorbent assay (ELISA) (FIG. 28A). VEGF-treated mice display higher post-euthanasia lung volume on post-operative day (POD) 4 and 10 (FIG. 28B). There is a trend toward increased total lung capacity (FIG. 28C) and pulmonary compliance (FIG. 28D) with VEGF treatment on POD 4. Although not reaching statistical significance, mice in the VEGF group performed better on post-treadmill rest time (FIG. 28E), walking distance (FIG. 28F), basic movement count (FIG. 28G), and fine movement count (FIG. 28H) than the control mice on POD 10.

To test the effect of topically delivered VEGF in a model of CLG, mice underwent left PNX and were randomized to receive either saline or VEGF via nasal instillation daily. Post-euthanasia body weight-normalized lung volume in the VEGF group was significantly higher than that of the control mice on both POD 4 (5.49 $\pm$0.11 vs 4.91$\pm$0.07 $\times$ 10-2 mL/g, P = 0.001) and POD 10 (6.68 $\pm$ 0.22 vs 6.11 $\pm$ 0.11 $\times$ 10-2 mL/g, P = 0.01) (FIG. 28B). Pulmonary function measured on POD 4 before euthanasia also showed a trend towards improvement in the VEGF group. VEGF-treated mice displayed improved total lung capacity (2.84 $\pm$ 0.04 vs 2.73 $\pm$ 0.03 $\times$ 10-2 mL/g, P = 0.05) (FIG. 28C) and pulmonary compliance (2.65 $\pm$ 0.09 vs 2.45 $\pm$ 0.06 $\times$ 10-3 mL/g.cmH2O, P = 0.07) (FIG. 28D). On POD 10, five randomly selected mice in each group underwent physical activity measurements before and after a compulsory treadmill exercise regimen. Although not reaching statistical significance, the VEGF group performed better across all parameters in the post-treadmill period, including longer walking distance, reduced rest time, and higher basic and fine movement counts (FIGS. 28E-28H). Overall, topical VEGF enhanced lung growth and potentially improved pulmonary function after left pneumonectomy.

Morphometric analyses were performed with a point and intersection counting technique. On POD 4, VEGF-treated lungs demonstrated increased normalized parenchymal volume (4.71 $\pm$ 0.28 vs 3.74 $\pm$ 0.18 $\times$ 10-2 mL/g, P = 0.02) (FIG. 29A), septal surface area (19.2 $\pm$ 0.8 vs 15.6 $\pm$ 0.8 cm2/g, P = 0.01) (FIG. 29C), and total alveolar count (3.61 $\pm$ 0.16 vs 2.61 $\pm$ 0.20 $\times$ 107, P = 0.005) (FIG. 29E). There was also a trend toward increased alveolar volume in the VEGF group (2.77 $\pm$ 0.22 vs 2.23 $\pm$ 0.11 $\times$ 10-2 mL/g, P = 0.06) (FIG. 29B). Of note, there was no difference in mean septal thickness between the two groups (FIG. 29D), indicating an absence of pulmonary edema.

FIGS. 29A-29E show the results of morphometric analyses. Lungs of VEGF-treated mice demonstrate increased parenchymal volume (FIG. 29A) and a trend toward higher alveolar volume (FIG. 29B). Septal surface area (FIG. 29C) was higher in the VEGF group but there was no difference in mean septal thickness (FIG. 29D). VEGF-treated mice had increased total alveolar count (FIG. 29E).

In order to assess the effect of VEGF on lung endothelial cells, nuclear staining of lung endothelial cells was performed to facilitate counting (FIGS. 30A-B). FIGS. 30A-B show images of immunostained lung endothelial cells. Lung endothelial cells were labeled with anti-ERG antibody (green) and proliferating cells are marked with anti-Ki67 antibody (red). Double-stained Ki67-positive endothelial cells appear yellow (arrows). Representative sections of control and VEGF-treated lungs harvested on POD 2 (FIG. 30A) and 4 (FIG. 30B) are shown at 20X magnification.

Topical VEGF treatment significantly increased percent proliferating endothelial cells on both POD 2 (0.54 $\pm$ 0.10 vs 0.16 $\pm$ 0.04, P = 0.009) and POD 4 (0.94 $\pm$ 0.16 vs 0.20 $\pm$ 0.04, P = 0.005) (FIG. 30C). Proliferating endothelial cells assumed a clustering pattern. However, these clusters were distributed equally throughout the right lung and there was no predilection for a central or peripheral region. Topical VEGF treatment significantly increases endothelial proliferation on both POD 2 and POD 4 (FIG. 30C).

In order to determine the effects of daily treatment on VEGF expression, a VEGF ELISA was utilized to quantify the levels of VEGF in lung tissues harvested on POD 4. There was a 2.1-fold increase in VEGF levels in the VEGF-treated lungs as compared to the control group (P = 0.048) (FIG. 31A). However, on qPCR analyses, there was no difference in the levels of VEGF mRNA transcripts (FIG. 31B). Therefore, the difference in VEGF levels between the two groups on ELISA was likely a result of their experimental treatments instead of an endogenous increase in expression of the protein.

FIGS. 31A-31E show the results of protein expression and gene expression analysis from lung tissue. VEGF levels were measured by ELISA from lung tissue isolated from mice on POD4 (FIG. 31A). ELISA results reveal increased VEGF levels in VEGF-treated lungs (FIG. 31A) on POD 4 compared to lungs of mice treated with saline (Control). However, quantitative polymerase chain reactions (qPCR) show no difference in mRNA transcript levels of VEGF, VEGFR1, or VEGFR2 between the two groups (FIG. 31B). Immunoblot demonstrates an increase in the levels of P-VEGFR2, VEGFR2, P-EGFR, EGFR, and heparin-binding EGF-like growth factor (HB-EGF) with VEGF treatment (FIGS. 31C-31D).

Analyses of lung tissues on POD 4 also revealed an increase in activated VEGFR2, P-VEGFR2, in the VEGF group (P = 0.03) (FIGS. 31C-31D). Interestingly, there was also an increase in total VEGFR2 expression with VEGF treatment (P = 0.02). Therefore, there was no difference in P-VEGFR2/VEGFR2 ratio. Since there was no difference in VEGFR2 transcript levels on qPCR (FIG. 31B), the increase in VEGFR2 expression on immunoblot could be a result of decreased receptor degradation. Similarly, VEGF treatment resulted in an increase in the levels of both P-EGFR (P = 0.03) and EGFR (P = 0.02) with no changes in P-EGFR/EGFR ratio. As HB-EGF shedding has been shown to be dependent on VEGFR2 activation, its presence was examined in the lungs and found to be significantly increased with VEGF treatment (P = 0.02) (FIG 31C-D). This data suggests that HB-EGF mediate angiogenic stimulation and epithelial cell proliferation in the context of lung growth.

To investigate if VEGF-dependent effects on the lung are mediated in part by HB-EGF, a series of *in vitro* co-culture assays were performed with human lung microvascular endothelial cells (HMVEC-L) and human bronchial epithelial cells (HBEC). To confirm that HMVEC-L cells alone are responsive to VEGF 165, unphosphorylated and phosphorylated VEGFR2 levels were measured in HMVEC-L following incubation with VEG165. As expected levels of unphosphorylated VEGFR2 were upregulated in HMVEC-L cells conditional on incubation with VEG165 (FIG 32A). Western blotting analysis revealed that HBEGF levels, specifically levels of soluble HBEGF not bound to membrane fragments, were upregulated in HMVEC-L cells conditional on incubation with VEG165 (FIG 32B). Additional western blotting analysis revealed that multiple angiogenic protein products - including Cathepsin B, Cathepsin V, matrix metalloprotease (MMP)-7, CD10, and Kallikrein-13 - were upregulated in HMVEC-L cells conditional on incubation with VEG165 (FIG 32B). Notably, Cathepsin B and MMP-7 are known to promote the release of HB-EGF from its transmembrane precursor, a process called ectodomain shedding. Thus, VEG165 stimulation of HMVEC-L cells confers upregulation of select proteases that are likely to increase release of membrane bound HG-EGF.

FIGS. 32A-32C show protein content results from assays performed with human lung microvascular endothelial cells (HMVEC-L). VEGF165 at 10 ng/mL activated HMVEC-L production of unphosphorylated VEGFR2 protein (FIG. 32A); VEGF165 is labeled as "VEGF." HMVEC-L treated with VEGF 165 show upregulation of proteases such as Cathepsin B and V, matrix metalloprotease (MMP)-7, CD10, and kallikrein 13 (FIG. 32B) as well as a more than 6-fold increase in HB-EGF concentration in the conditioned medium (FIG. 32C).

As a control, prior to embarking on HMVEC-L and HBEC co-culture assays (FIG 32E), cell density of HBEC cells cultured alone showed no difference over time in presence of VEGF 165. FIG 32D shows cell density of HBEC cells over time in presence or absence of VEGF 165.

To determine if proliferation of HBEC increases when co-cultured with HMVEC-L treated with VEGF 165, cell density of HBEC was measured over time from HBEC cells grown in the co-culture apparatus (FIG 32E). The proliferation of HBEC increased in first 24 hours conditional on HMVEC-L being treated with VEGF 154 (FIG. 32F).

FIG. 32E shows a cartoon representation of the HMVEC2 and HVEC co-culture system. The insert for the dish contained a porous membrane that restricted movement of HMVEC-2 cells, but did not restrict diffusion of VEG165 that was initially placed in medium held by the insert. FIG. 32F shows cell density of HBEC cells over time in presence or absence of HMVEC-L cells treated or not treated with VEGF 165.

To verify that HMVEC-L cells treated with VEGF165 are competent to confer increased proliferation of HBEC cells, a competition experiment was performed with different amounts of HB-EGF neutralizing; a cartoon representation of the competition experiment is shown in FIG. 32G and quantified results of HBEC cell density in different conditions tested are shown in FIG. 32H. Notably, addition of increasing amounts of HB-EGF neutralizing antibody to medium of HBEC cells co-cultured with HMVEC-L cells treated with VEGF165 is sufficient to decrease HBEC proliferation. These results are consistent with VEGF-dependent effects on lung growth being mediated by HB-EGF. To further verify this, levels of EGFR - a protein that correlates with active growth dependent on HB-EGF - decreased in HBEC cells exposed to HB-EGF neutralizing antibody and co-cultured with HMVEC-L cells treated with VEGF165 (FIGS. 32I and 32J).

Collectively, the results demonstrate that HB-EGF is an important paracrine factor that links angiogenic stimulation to epithelial proliferation and may mediate the effects of VEGF on enhancing growth of bronchial epithelial cells.

FIG 32G shows a cartoon representation of HMVEC-L and HBEC co-culture competition assays performed with differing amounts of VEG165 present in the medium located in the insert and with different amounts of HB-EGF neutralizing antibody (HB-EGF antibody) present in the medium of the well. FIG. 32H shows the quantified results of the HMVEC-L and HBEC co-culture competition assays depicted in FIG 32G.

In this example, experiments were performed to replicate these results with topical administration of VEGF. Consideration was given to aerosol inhalation, intratracheal instillation, and nasal instillation. Although aerosol inhalation has been shown to result in a more uniform pulmonary distribution, dosage delivery is variable and limited. Intratracheal instillation requires deep sedation and intratracheal intubation, which is traumatic to the animal if performed on a daily basis. Although nasal instillation presents certain limitations, such as heterogenous intrapulmonary distribution and central nervous system absorption, it appeared to be the optimal approach due to the benefits of controlled dosage and light sedation requirement.

Compared to systemic injection, nasal instillation significantly decreased the amount of VEGF absorbed into the bloodstream. However, targeted intrapulmonary administration of VEGF still resulted in improvement in lung volume and alveolar count. Moreover, the increase in endothelial proliferation observed on both POD 2 and 4 provided supporting evidence for the effectiveness of nasal instillation. The clustering appearance of these proliferating endothelial cells may be a result of the heterogenous distribution associated with nasal instillation. However, it should be noted that these clusters were evenly distributed across the entire right lung and there was no predilection for the peribronchiolar region. The improvement in pulmonary distribution could be a result of higher administered volume used in this example (50 μL/20 g).

Furthermore, the increase in lung volume was supported by an improvement in total lung capacity and pulmonary compliance on pulmonary mechanical studies. Since pulmonary compliance is a measure of volume change against applied pressure and total lung capacity is a direct measure of lung volume in live animals, these parameters have been shown to correlate with lung growth and improve during the process of CLG. Despite the lack of statistical significance, there was also an improvement in walking distance, rest time, and basic and fine movements in VEGF-treated mice on POD 10, especially in the post-treadmill measurements. Since exercise intolerance correlates with pulmonary arterial hypertension (PAH) and VEGF can induce nitric oxide (NO) production, the effects of VEGF on exercise capacity seen in this example were possibly a product of PAH alleviation. It is also possible that more pronounced and significant differences in exercise tolerance could be seen with more intense exercise regimens or further post-operative time points.

While the mechanism of how VEGF contributes to microvascular endothelial cell proliferation is widely characterized, how VEGF leads to bronchial epithelial cell proliferation is not well understood. In the lung, VEGF has been shown to increase both endothelial and epithelial cell proliferation of lung explants in vitro. This disclosure found HB-EGF to be a key factor that was upregulated by VEGF treatment in both the in vivo CLG model and in in vitro assays. VEGF had no direct effects on HBEC growth, yet co-culture with VEGF-stimulated HMVEC-L resulted in increased HBEC proliferation. HB-EGF was up-regulated in the conditioned medium of VEGF-treated HMVEC-L and neutralization of HB-EGF abolished the mitogenic effects of VEGF on HBEC. These results suggest that HB-EGF is a paracrine factor that relays angiogenic signaling for epithelial cell proliferation.

HB-EGF is a ligand for EGFR and its upregulation has been shown as a key event in the tissue repair and regeneration of many organs ranging from the skin and cornea to liver and gastrointestinal tract. It is synthesized as a transmembrane protein called proHB-EGF, which undergoes further proteolytic cleavage to release the mature, soluble HB-EGF, a process called ectodomain shedding. The concurrent increase in multiple proteases, such as cathepsin B and MMP-7 in VEGF-treated HMVEC-L further supported the occurrence of ectodomain shedding as a mechanism for HB-EGF upregulation in this study. HB-EGF shedding is a key event after activation of VEGFR2 and inhibition of this process significantly impaired CLG. This disclosure shows HB-EGF may play a key mechanistic role in mediating the effects of VEGF on CLG given its upregulation with VEGF treatment and mitogenic properties on lung epithelial cells. However, epithelial cell proliferation is only one of the key steps involved in alveologenesis and additional factors likely contribute to this process.

Dysregulation of the VEGF pathway has been implicated in many neonatal lung diseases from CDH to bronchopulmonary dysplasia (BPD). Both diseases are characterized by a deficiency in alveolar units either through an arrest in pulmonary development (CDH) or destruction of parenchyma from mechanical ventilation and a hyperoxic environment (BPD). Additionally, the need for mechanical respiratory support in almost all children born with CDH can further deteriorate their pulmonary functions despite the use of lung-protective ventilator strategies.

## Claims

1. A composition comprising VEGF-A for use in a method of promoting lung growth in a subject, the method comprising:

identifying a subject in need of promoting lung growth; and
administering to the subject an effective amount of the composition, wherein the composition is administered through respiratory tract by inhalation, the subject is a newborn, a child, or an adult, and
wherein the subject has pulmonary hypoplasia or congenital diaphragmatic hernia (CDH).

**2.** The composition for use of claim 1, wherein the VEGF-A is rhVEGF-165.

**3.** The composition for use of claim 1 or 2, wherein percent-predicted lung volume (PPLV) in the subject is less than 15%.

**4.** The composition for use of any one of claims 1-3, wherein the subject is monitored for hypotension during or after administering the composition to the subj ect.

**5.** The composition for use of any one of claims 1-4, wherein one or more agents selected from the group consisting of midodrine, norepinephrine, norepinephrine bitartrate, phenylephrine, droxidopa, ephedrine, and N-Nitroarginine methyl ester are administered to the subject before, during, or after administering the composition to the subject.

**6.** A composition comprising VEGF-A for use in a method of increasing parenchymal volume, alveolar volume, or septal surface area, or alveolar density and number in a subject, the method comprising:

administering to the subject an effective amount of the composition, wherein the composition is administered through respiratory tract by inhalation, the subject is a newborn, a child, or an adult, and
wherein the subject has pulmonary hypoplasia.

**7.** The composition for use of claim 6, wherein the VEGF-A is rhVEGF-165.

**8.** A composition comprising VEGF-A for use in a method of promoting lung growth in a subject, the method comprising:

administering to the subject an effective amount of the composition via intravenous administration; and,
subsequently, administering to the subject an effective amount of a second composition comprising VEGF-A via the respiratory tract by inhalation,
wherein the subject has pulmonary hypoplasia or congenital diaphragmatic hernia (CDH).

**9.** The composition for use of claim 8, wherein the VEGF-A in the composition is rhVEGF-165.

**10.** The composition for use of claim 8 or 9, wherein the VEGF-A in the second composition is rhVEGF-165.

**11.** The composition for use of claim 8 or 9, wherein the second composition comprises HB-EGF.

**12.** The composition for use of claims 1-9, wherein the composition is administered to a 0-1 month old baby, a 1-3 month old baby, a 3-6 month old baby, a 6-9 month old baby, or a 9-12 month old baby.

## Patentansprüche

**1.** Zusammensetzung, die einen VEGF-A zur Verwendung bei einem Verfahren zur Förderung des Lungenwachstums in einem Individuum umfasst, wobei das Verfahren Folgendes umfasst:

das Identifizieren eines Individuums, das eine Förderung des Lungenwachstums benötigt; und
das Verabreichen einer wirksamen Menge der Zusammensetzung an das Individuum, wobei die Zusammensetzung mittels Inhalation über die Atemwege verabreicht wird, das Individuum ein Neugeborenes, ein Kind oder ein Erwachsener ist, und
wobei das Individuum eine Lungenhypoplasie oder kongenitale Diaphragmahernie (CDH) aufweist.

**2.** Zusammensetzung zur Verwendung nach Anspruch 1, wobei es sich beim VEGF-A um rhVEGF-165 handelt.

**3.** Zusammensetzung zur Verwendung nach Anspruch 1 oder 2, wobei das erwartete prozentuale Lungenvolumen (PPLV) im Individuum weniger als 15 % beträgt.

**4.** Zusammensetzung zur Verwendung nach einem der Ansprüche 1-3, wobei das Individuum während oder nach der Verabreichung der Zusammensetzung an das Individuum auf Hypotonie überwacht wird.

**5.** Zusammensetzung zur Verwendung nach einem der Ansprüche 1-4, wobei ein oder mehrere Mittel, das bzw. die ausgewählt ist bzw. sind aus der Gruppe bestehend aus Midodrin, Norepinephrin, Norepinephrinbitartrat, Phenylephrin, Droxidopa, Ephedrin und N-Nitroargininmethylester, vor, während oder nach der Verabreichung der Zusammensetzung an das Individuum an das Individuum verabreicht wird bzw. werden.

**6.** Zusammensetzung, die VEGF-A umfasst, zur Verwendung bei einem Verfahren zur Erhöhung des Parenchymvolumens, Alveolarvolumens oder der septalen Oberfläche oder der Dichte und Zahl der Alveolen in einem Individuum, wobei das Verfahren Folgendes umfasst:

das Verabreichen einer wirksamen Menge der Zusammensetzung an das Individuum, wobei die Zusammensetzung mittels Inhalation über die Atemwege verabreicht wird, das Individuum ein Neugeborenes, ein Kind oder ein Erwachsener ist, und
wobei das Individuum eine Lungenhypoplasie aufweist.

**7.** Zusammensetzung zur Verwendung nach Anspruch 6, wobei es sich beim VEGF-A um rhVEGF-165 handelt.

**8.** Zusammensetzung, die einen VEGF-A zur Verwendung bei einem Verfahren zur Förderung des Lungenwachstums in einem Individuum umfasst, wobei das Verfahren Folgendes umfasst:

das Verabreichen einer wirksamen Menge der Zusammensetzung an das Individuum über eine intravenöse Verabreichung und
anschließend das Verabreichen einer wirksamen Menge einer zweiten VEGF-A umfassenden Zusammensetzung mittels Inhalation über die Atemwege an das Individuum,
wobei das Individuum eine Lungenhypoplasie oder kongenitale Diaphragmahernie (CDH) aufweist.

**9.** Zusammensetzung zur Verwendung nach Anspruch 8, wobei es sich beim VEGF-A in der Zusammensetzung um rhVEGF-165 handelt.

**10.** Zusammensetzung zur Verwendung nach Anspruch 8 oder 9, wobei es sich beim VEGF-A in der zweiten Zusammensetzung um rhVEGF-165 handelt.

**11.** Zusammensetzung zur Verwendung nach Anspruch 8 oder Anspruch 9, wobei die zweite Zusammensetzung HB-EGF umfasst.

**12.** Zusammensetzung zur Verwendung nach den Ansprüchen 1-9, wobei die Zusammensetzung an ein 0-1 Monat altes Baby, ein 1-3 Monate altes Baby, ein 3-6 Monate altes Baby, ein 6-9 Monate altes Baby oder ein 9-12 Monate altes Baby verabreicht wird.

## Revendications

**1.** Composition comprenant VEGF-A pour une utilisation dans un procédé de favorisation de la croissance pulmonaire chez un sujet, le procédé comprenant :

l'identification d'un sujet ayant besoin de favorisation de la croissance pulmonaire ; et
l'administration au sujet d'une quantité efficace de la composition, la composition étant administrée par inhalation par les voies respiratoires, le sujet étant un nouveau-né, un enfant ou un adulte, et
dans laquelle le sujet présente une hypoplasie pulmonaire ou une hernie diaphragmatique congénitale (CDH).

**2.** Composition pour une utilisation selon la revendication 1, dans laquelle le VEGF-A est rhVEGF-165.

**3.** Composition pour une utilisation selon la revendication 1 ou 2, dans laquelle le pourcentage de volume pulmonaire prédit (PPLV) chez le sujet est inférieur à 15 %.

**4.** Composition pour une utilisation selon l'une quelconque des revendications 1-3, dans laquelle le sujet fait l'objet d'une surveillance pour détecter une hypotension pendant ou après l'administration de la composition au sujet.

5. Composition pour une utilisation selon l'une quelconque des revendications 1-4, dans laquelle un ou plusieurs agents sélectionnés dans le groupe constitué par midodrine, noradrénaline, bitartrate de noradrénaline, phényléphrine, droxidopa, éphédrine et ester méthylique de N-nitroarginine sont administrés au sujet avant, pendant ou après l'administration de la composition au sujet.

6. Composition comprenant VEGF-A pour une utilisation dans un procédé d'augmentation du volume parenchymateux, du volume alvéolaire ou de la surface septale, ou de la densité et du nombre d'alvéoles chez un sujet, le procédé comprenant :

   l'administration au sujet d'une quantité efficace de la composition, la composition étant administrée par inhalation par les voies respiratoires, le sujet étant un nouveau-né, un enfant ou un adulte, et
   dans laquelle le sujet présente une hypoplasie pulmonaire.

7. Composition pour une utilisation selon la revendication 6, dans laquelle le VEGF-A est rhVEGF-165.

8. Composition comprenant VEGF-A pour une utilisation dans un procédé de favorisation de la croissance pulmonaire chez un sujet, le procédé comprenant :

   l'administration au sujet d'une quantité efficace de la composition via une administration intraveineuse ; et,
   par la suite, l'administration au sujet d'une quantité efficace d'une deuxième composition comprenant VEGF-A via les voies respiratoires par inhalation,
   dans laquelle le sujet présente une hypoplasie pulmonaire ou une hernie diaphragmatique congénitale (CDH).

9. Composition pour une utilisation selon la revendication 8, dans laquelle le VEGF-A dans la composition est rhVEGF-165.

10. Composition pour une utilisation selon la revendication 8 ou 9, dans laquelle le VEGF-A dans la deuxième composition est rhVEGF-165.

11. Composition pour une utilisation selon la revendication 8 ou 9, dans laquelle la deuxième composition comprend HB-EGF.

12. Composition pour une utilisation selon les revendications 1-9, la composition étant administrée à un bébé de 0-1 mois, un bébé de 1-3 mois, un bébé de 3-6 mois, un bébé de 6-9 mois ou un bébé de 9-12 mois.

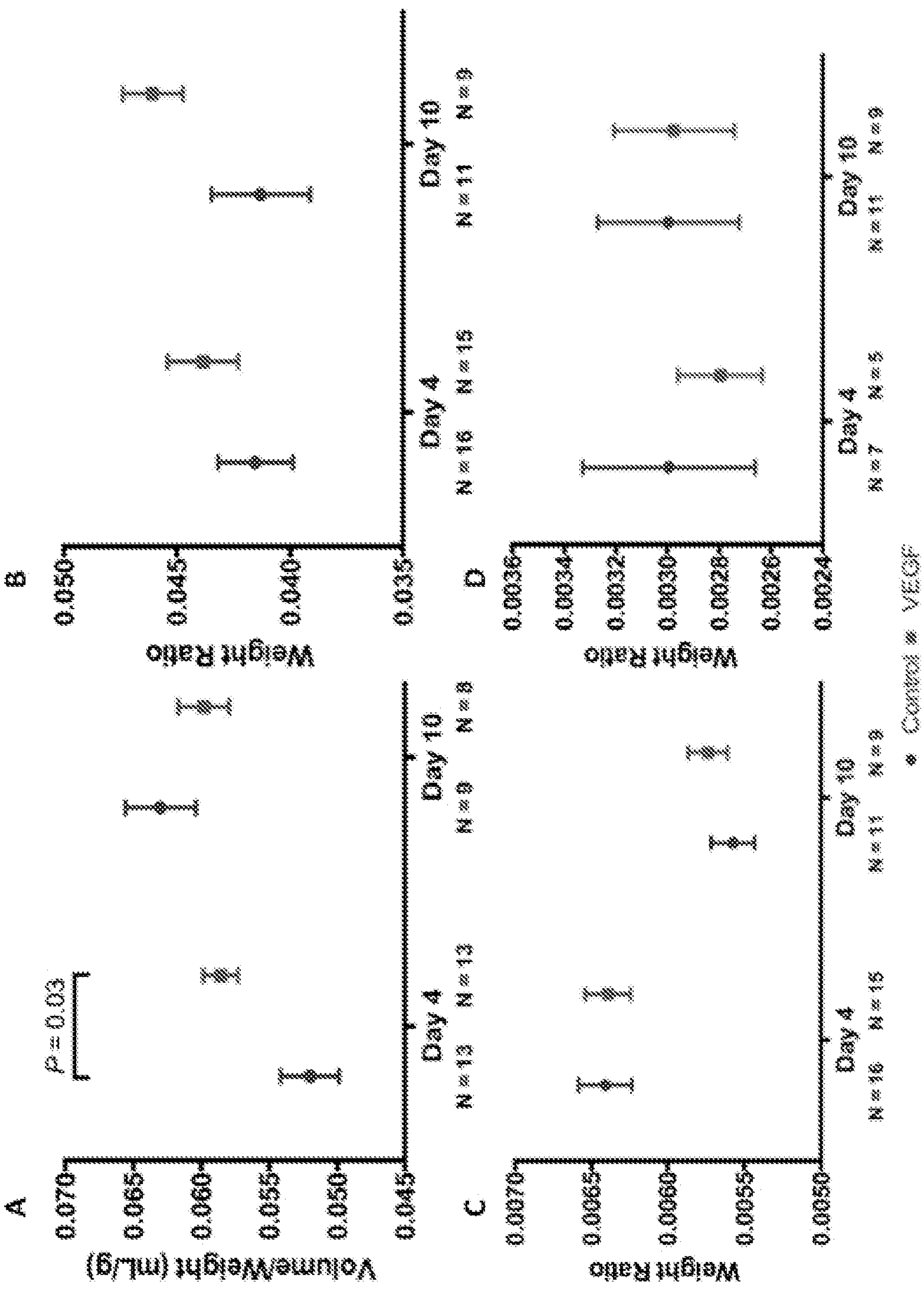
A
Volume/Weight (mL/g)
0.070
0.065
0.060
0.055
0.050
0.045
P = 0.03
Day 4
Day 10
N = 13
N = 13
N = 9
N = 8
B
Weight Ratio
0.050
0.045
0.040
0.035
Day 4
Day 10
N = 16
N = 15
N = 11
N = 9
C
Weight Ratio
0.0070
0.0065
0.0060
0.0055
0.0050
Day 4
Day 10
N = 16
N = 15
N = 11
N = 9
D
Weight Ratio
0.0036
0.0034
0.0032
0.0030
0.0028
0.0026
0.0024
Day 4
Day 10
N = 7
N = 5
N = 11
N = 9
Control
VEGF

FIGS. 1A-1D

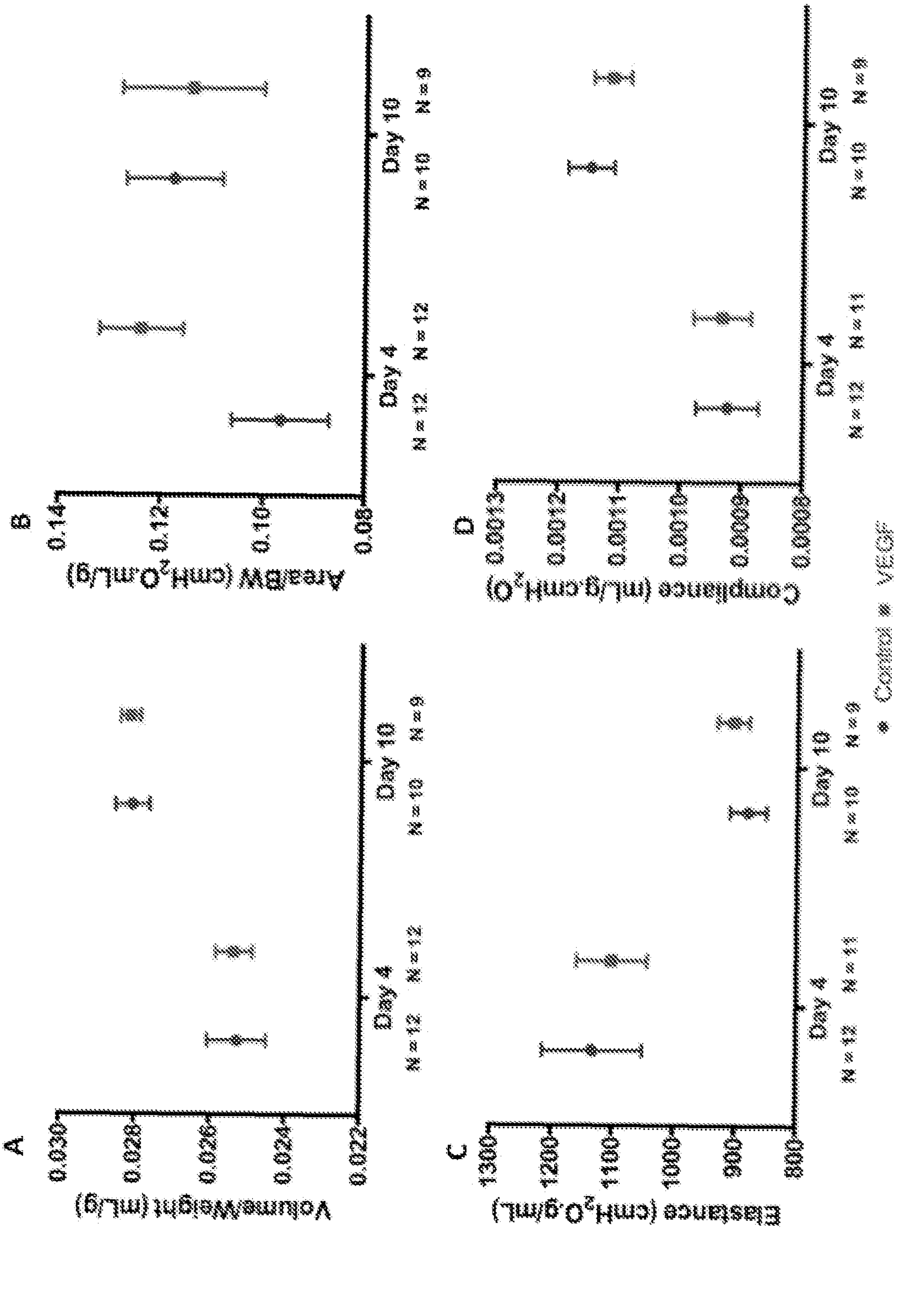
A
Volume/Weight (mL/g)
0.030
0.028
0.026
0.024
0.022
Day 4
Day 10
N = 12 N = 12
N = 10 N = 9
B
Area/BW (cmH2O.mL/g)
0.14
0.12
0.10
0.08
C
Elastance (cmH2O.g/mL)
1300
1200
1100
1000
900
800
N = 12 N = 11
N = 10 N = 9
D
Compliance (mL/g.cmH2O)
0.0013
0.0012
0.0011
0.0010
0.0009
0.0008
Control
VEGF

FIGS. 2A-2D

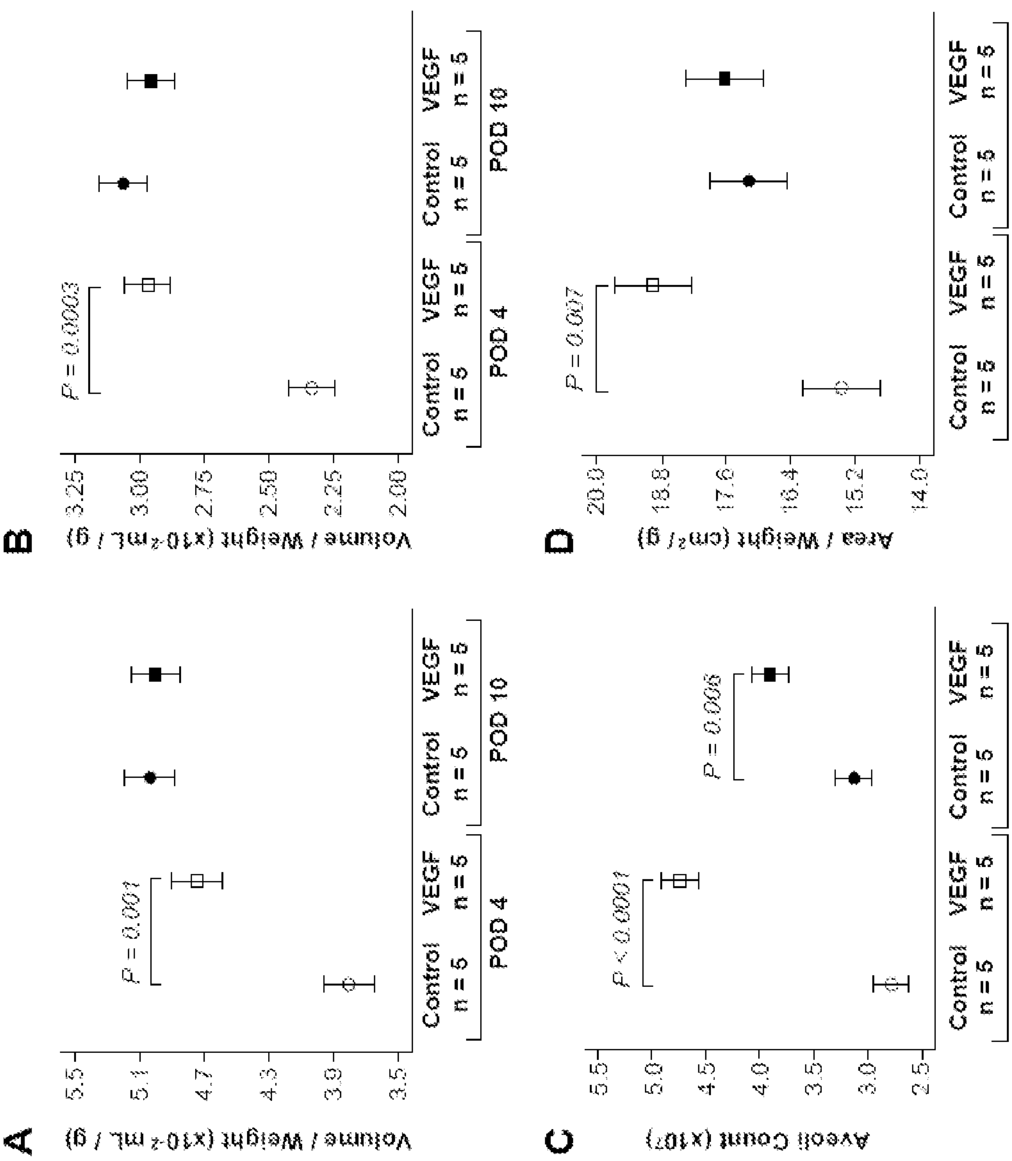
A
B
C
D
Volume / Weight (x10-2 mL / g)
Alveoli Count (x10^7)
Area / Weight (cm^2 / g)
P = 0.001
P = 0.0003
P < 0.0001
P = 0.006
P = 0.007
Control
VEGF
n = 5
POD 4
POD 10

FIGS. 3A-3D

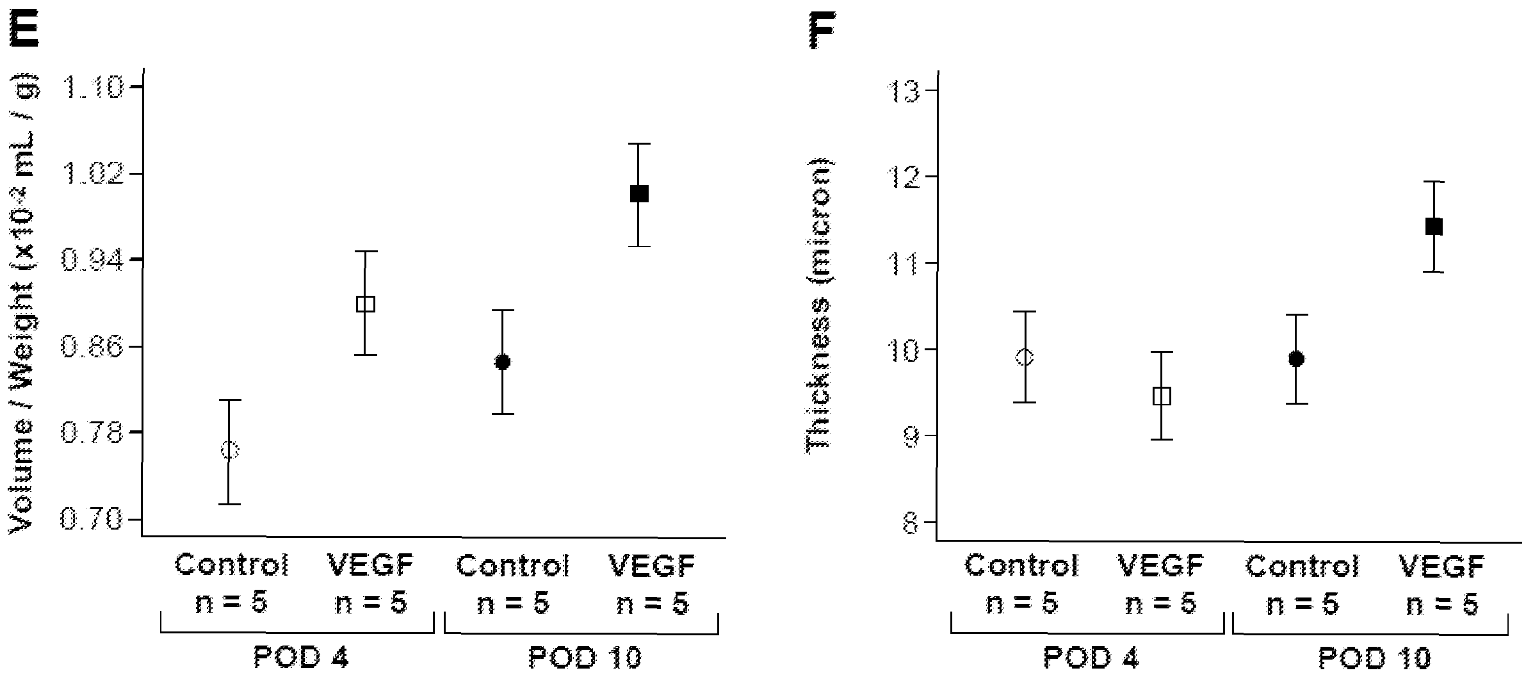
E
Volume / Weight (x10⁻² mL / g)
1.10
1.02
0.94
0.86
0.78
0.70
Control
n = 5
VEGF
n = 5
Control
n = 5
VEGF
n = 5
POD 4
POD 10
F
Thickness (micron)
13
12
11
10
9
8
Control
n = 5
VEGF
n = 5
Control
n = 5
VEGF
n = 5
POD 4
POD 10

FIGS. 3E-3F

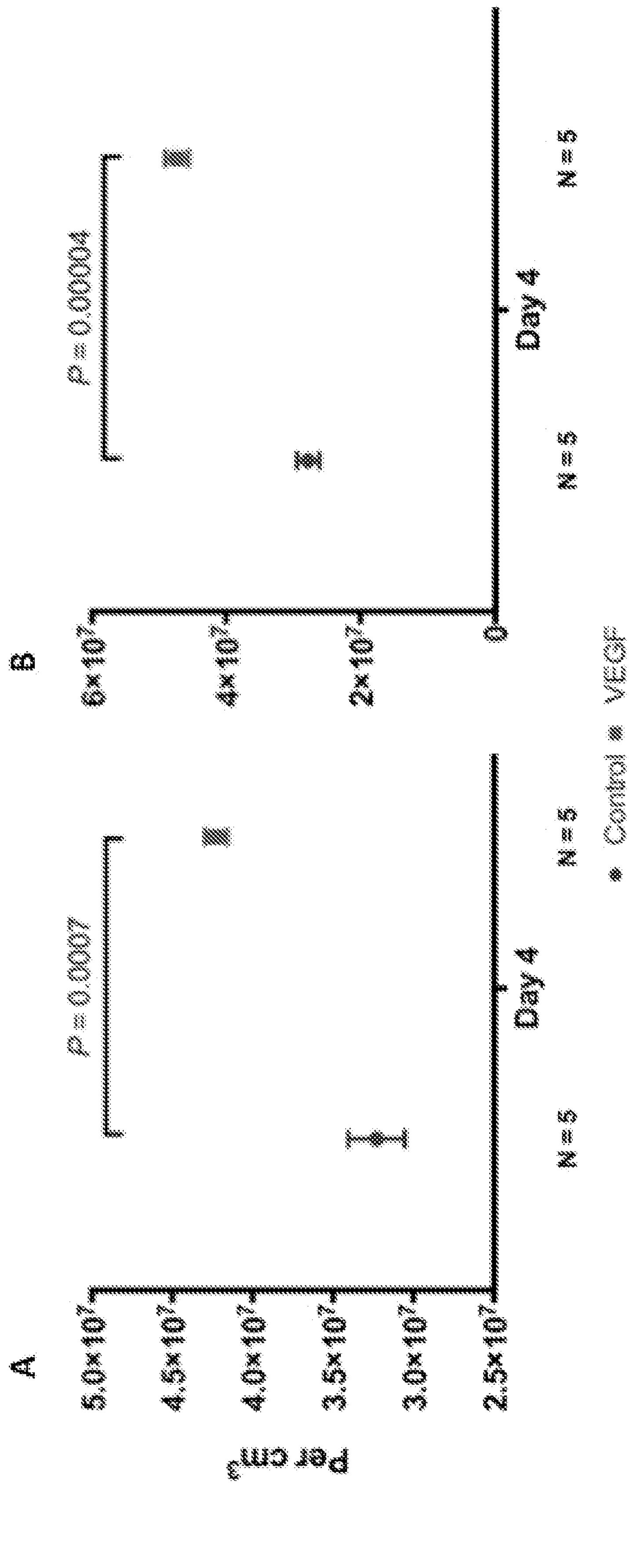
A
B
Per cm³
5.0×10⁷
4.5×10⁷
4.0×10⁷
3.5×10⁷
3.0×10⁷
2.5×10⁷
6×10⁷
4×10⁷
2×10⁷
0
P = 0.0007
P = 0.00004
N = 5
N = 5
N = 5
N = 5
Day 4
Day 4
Control
VEGF

FIGS. 4A-4B

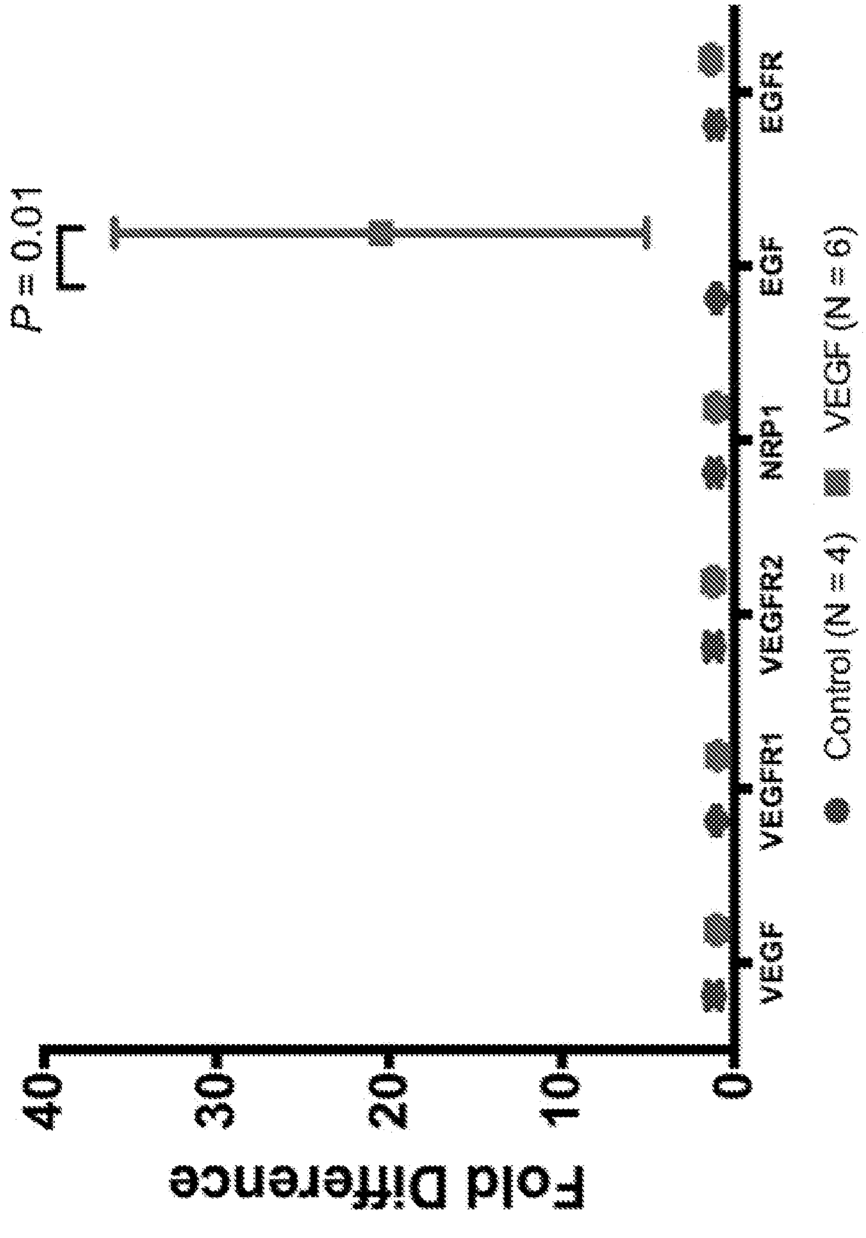
Fold Difference
0
10
20
30
40
VEGF
VEGFR1
VEGFR2
NRP1
EGF
EGFR
P = 0.01
Control (N = 4)
VEGF (N = 6)

FIG. 5

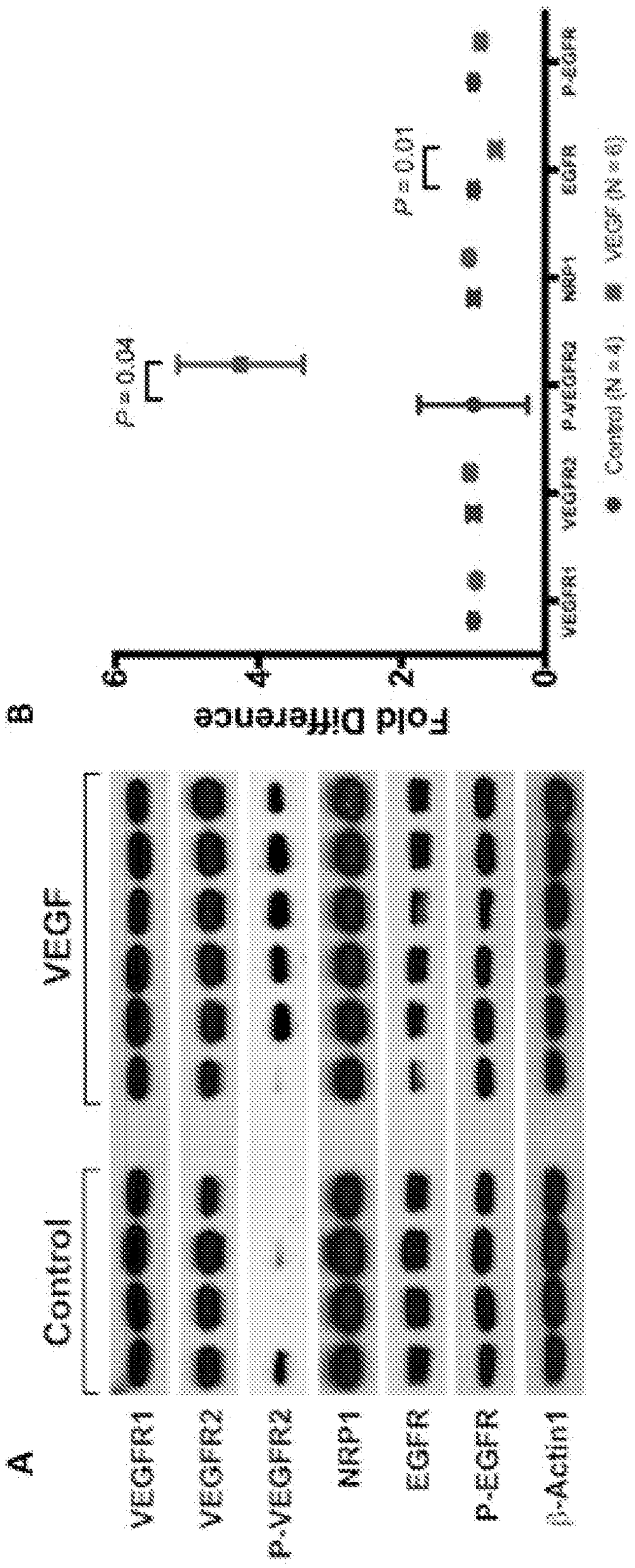
A
VEGFR1
VEGFR2
P-VEGFR2
NRP1
EGFR
P-EGFR
β-Actin1
Control
VEGF
B
Fold Difference
0
2
4
6
P = 0.04
P = 0.01
VEGFR1
VEGFR2
P-VEGFR2
NRP1
EGFR
P-EGFR
Control (N = 4)
VEGF (N = 6)

FIGS. 6A-6B

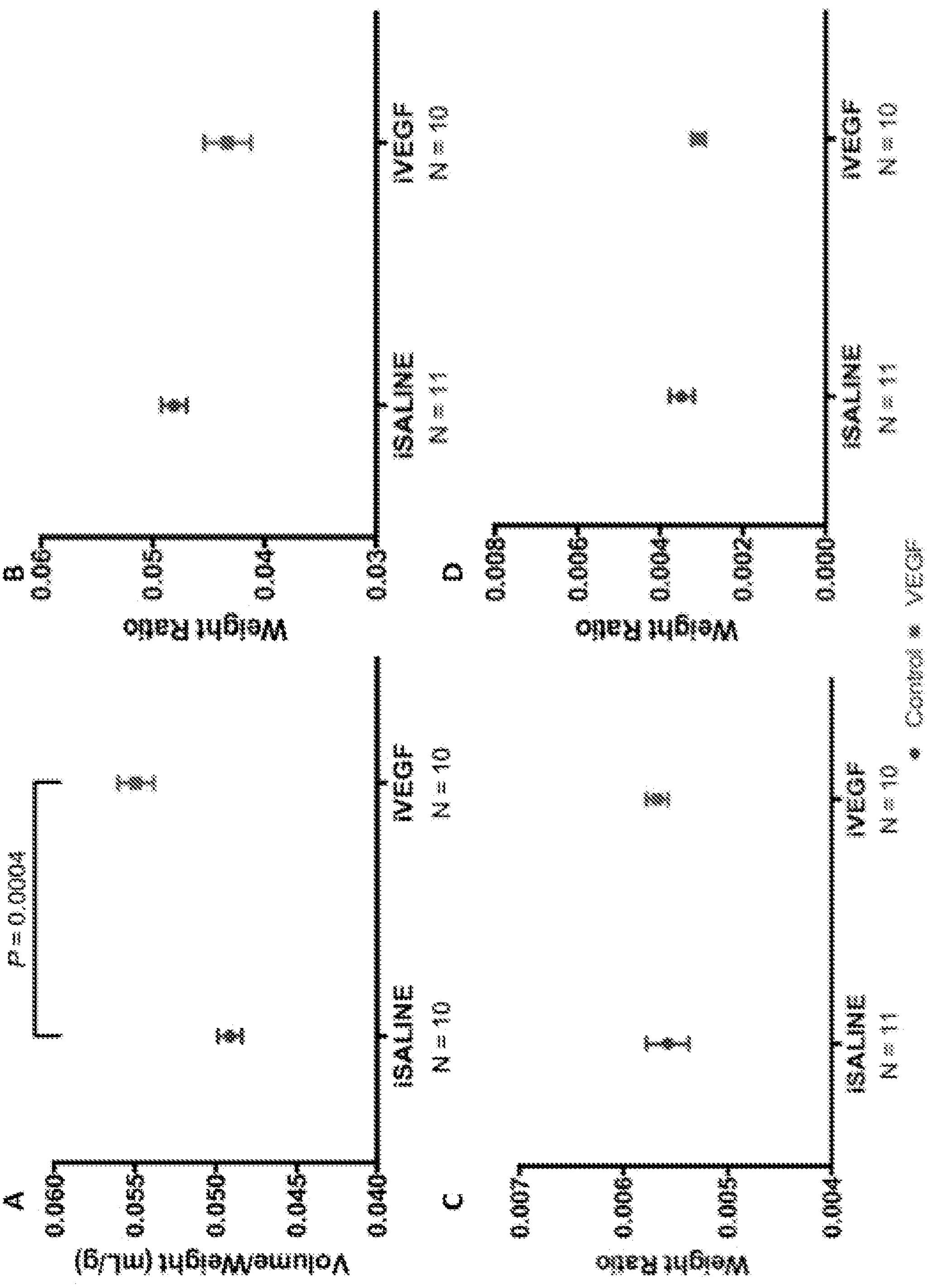
A
Volume/Weight (mL/g)
0.060
0.055
0.050
0.045
0.040
P = 0.0004
iSALINE
N = 10
iVEGF
N = 10
B
Weight Ratio
0.06
0.05
0.04
0.03
iSALINE
N = 11
iVEGF
N = 10
C
Weight Ratio
0.007
0.006
0.005
0.004
iSALINE
N = 11
iVEGF
N = 10
D
Weight Ratio
0.008
0.006
0.004
0.002
0.000
iSALINE
N = 11
iVEGF
N = 10
Control
VEGF

FIGS. 7A-7D

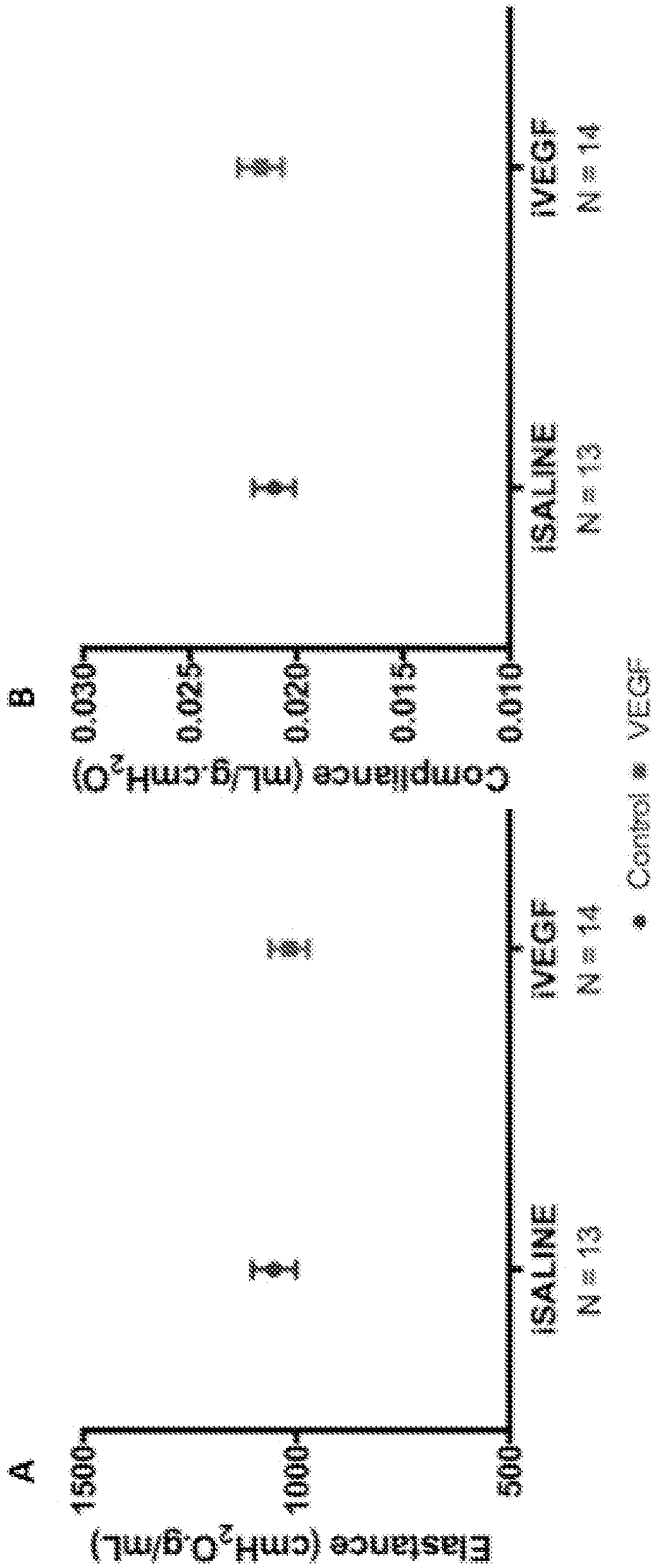
A
Elastance (cmH$_2$O.g/mL)
1500
1000
500
SALINE
N = 13
VEGF
N = 14
B
Compliance (mL/g.cmH$_2$O)
0.030
0.025
0.020
0.015
0.010
SALINE
N = 13
VEGF
N = 14
Control
VEGF

FIGS. 8A-8B

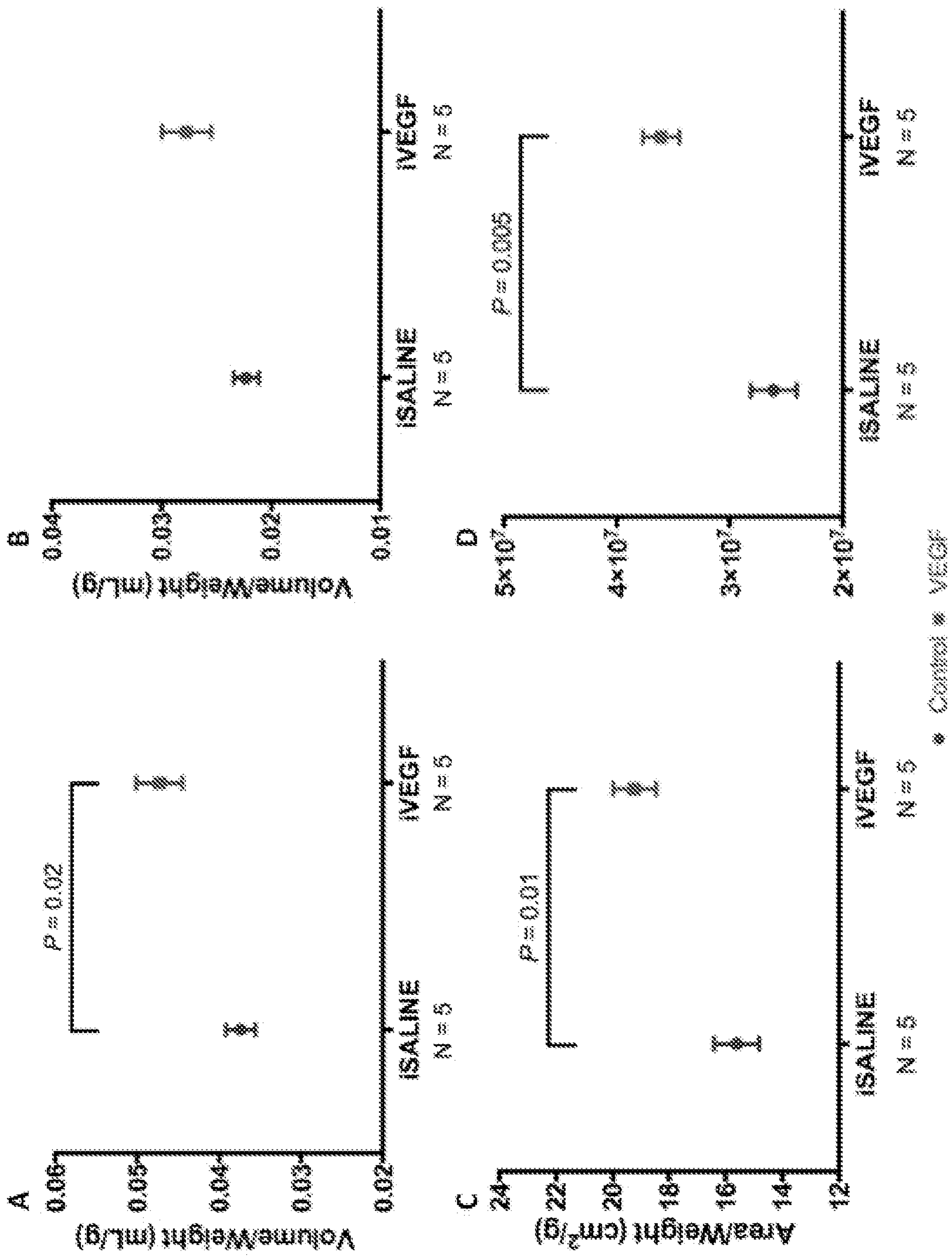
A
Volume/Weight (mL/g)
0.06
0.05
0.04
0.03
0.02
P = 0.02
ISALINE
N = 5
IVEGF
N = 5
B
Volume/Weight (mL/g)
0.04
0.03
0.02
0.01
ISALINE
N = 5
IVEGF
N = 5
C
Area/Weight (cm²/g)
24
22
20
18
16
14
12
P = 0.01
ISALINE
N = 5
IVEGF
N = 5
D
5×10⁷
4×10⁷
3×10⁷
2×10⁷
P = 0.005
ISALINE
N = 5
IVEGF
N = 5
Control
VEGF

FIGS. 9A-9D

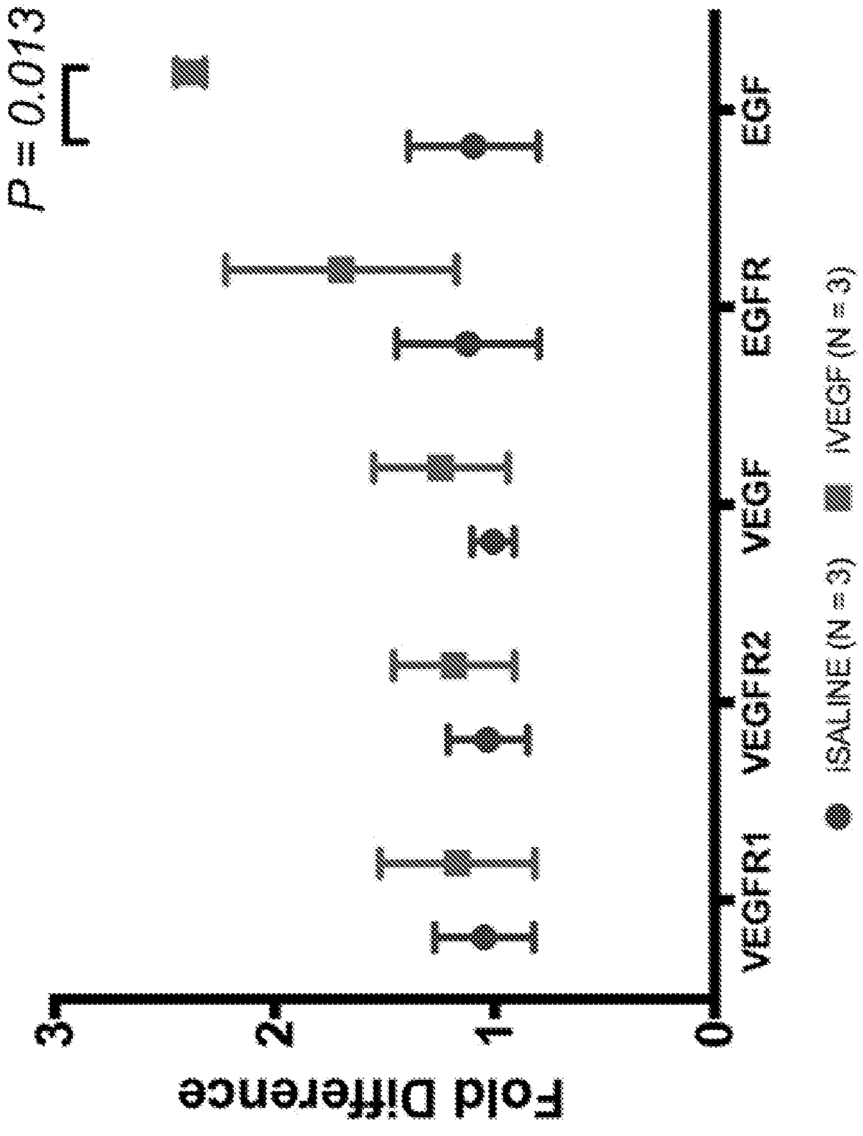
P = 0.013
Fold Difference
0
1
2
3
VEGFR1
VEGFR2
VEGF
EGFR
EGF
iSALINE (N = 3)
iVEGF (N = 3)

FIG. 10

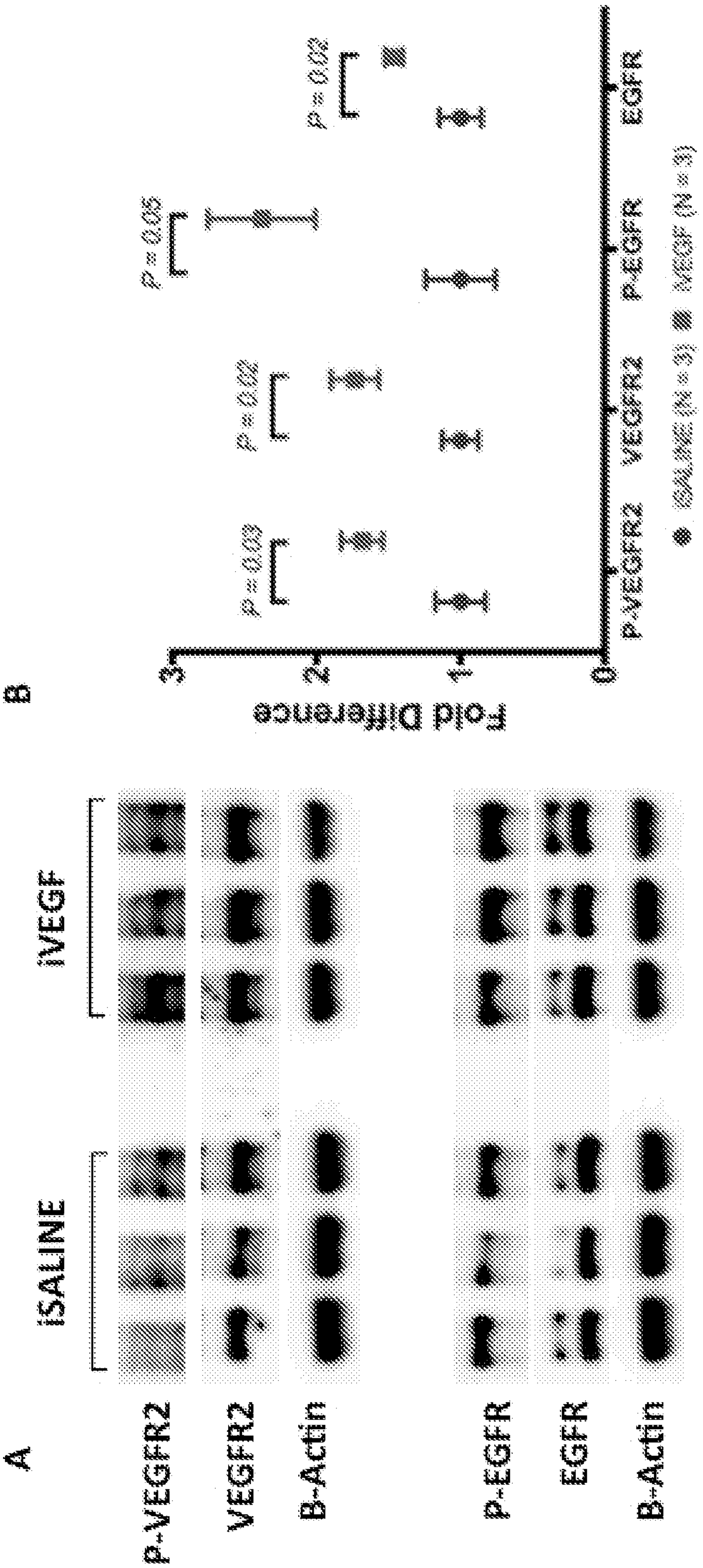
A
P-VEGFR2
VEGFR2
B-Actin
P-EGFR
EGFR
B-Actin
iSALINE
iVEGF
B
Fold Difference
0
1
2
3
P-VEGFR2
VEGFR2
P-EGFR
EGFR
P = 0.03
P = 0.02
P = 0.05
P = 0.02
iSALINE (N = 3)
iVEGF (N = 3)

FIGS. 11A-11B

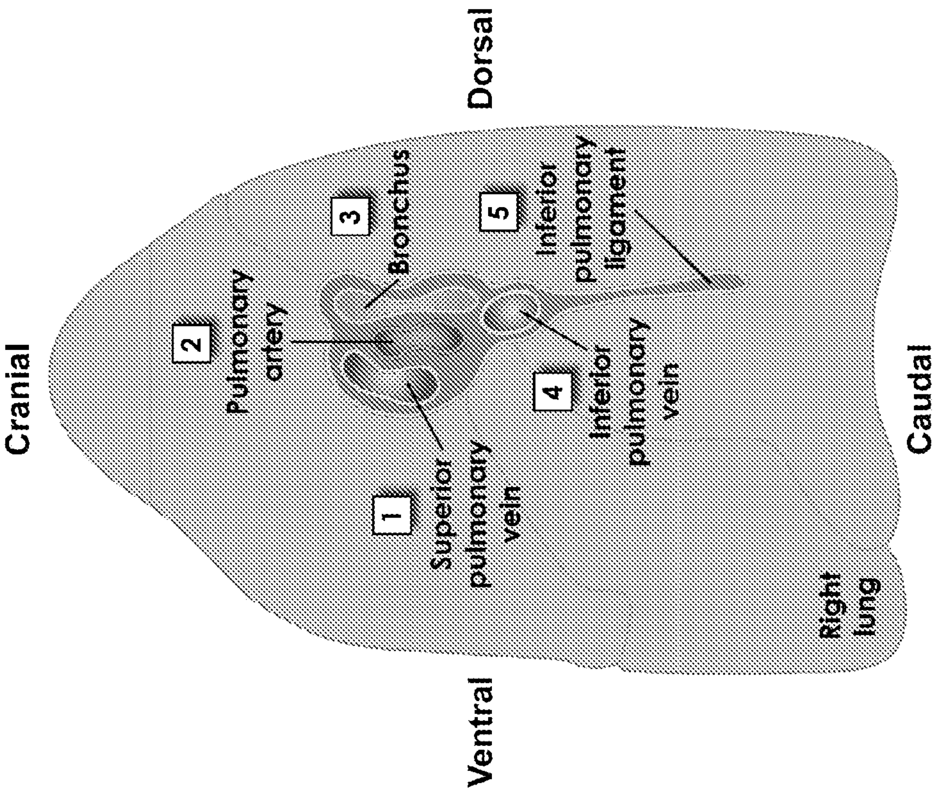
Cranial
Dorsal
Ventral
Caudal
1
Superior pulmonary vein
2
Pulmonary artery
3
Bronchus
4
Inferior pulmonary vein
5
Inferior pulmonary ligament
Right lung

FIG. 12

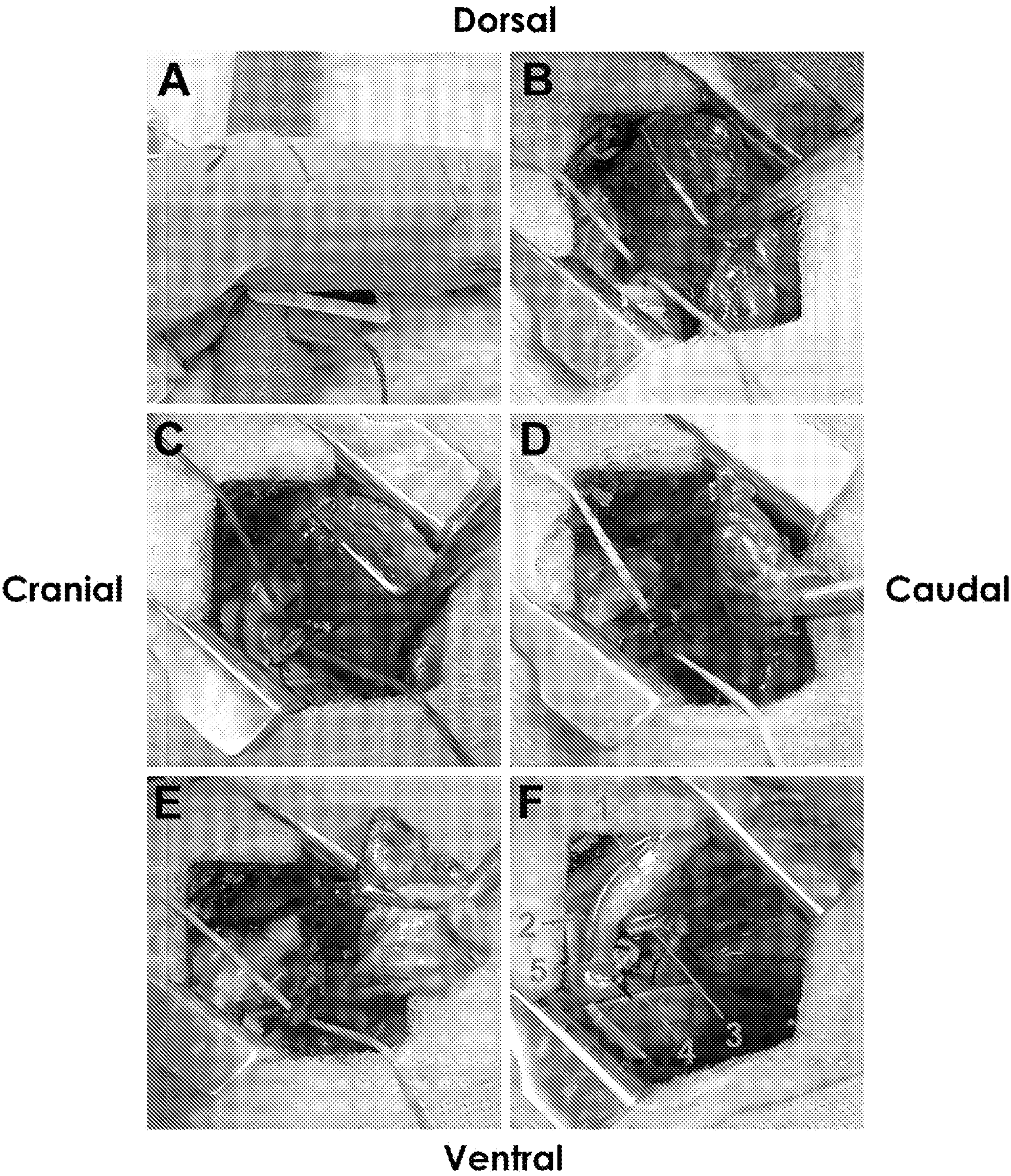


FIGS. 13A-13F

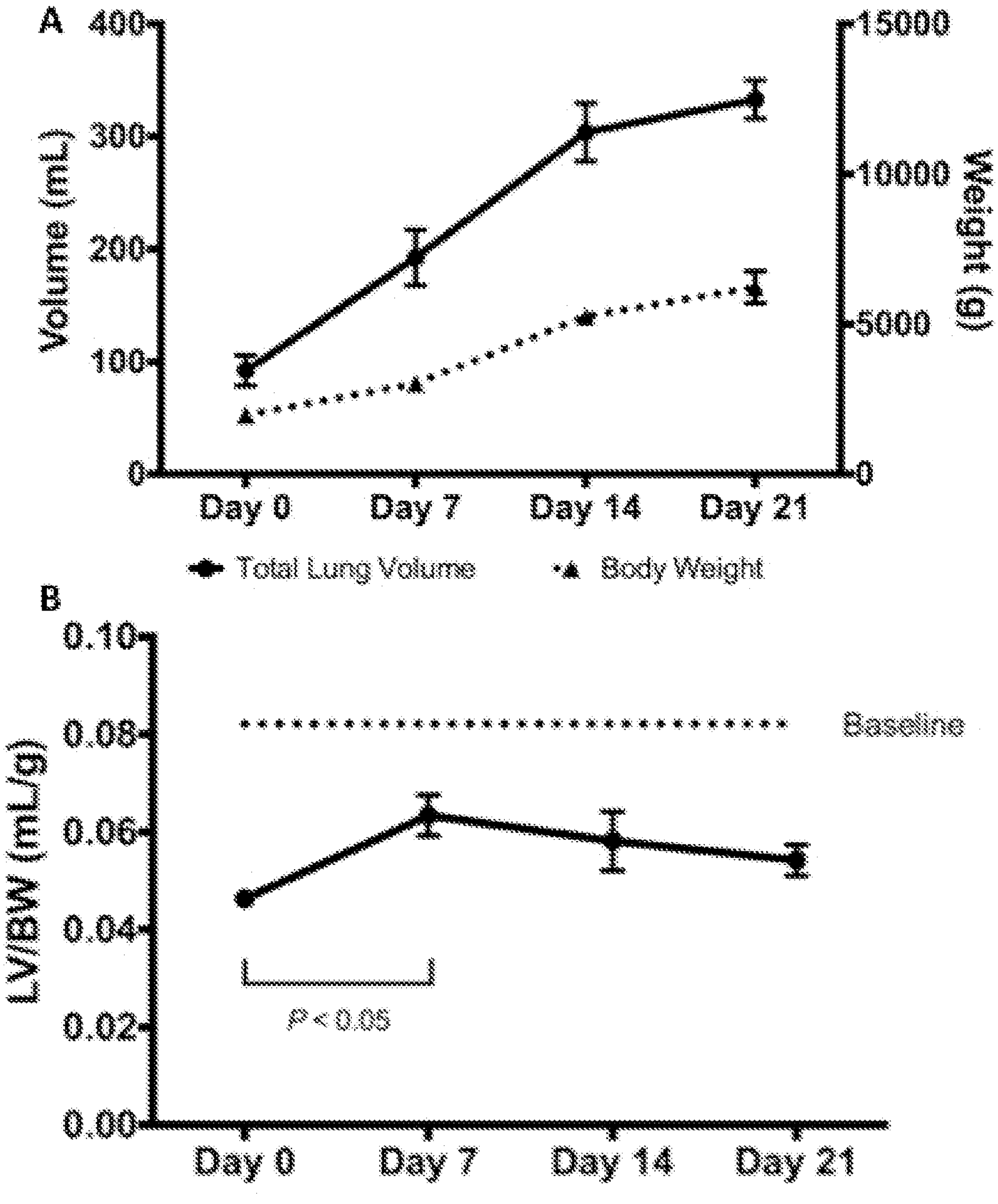
A
Volume (mL)
400
300
200
100
0
15000
10000
5000
0
Weight (g)
Day 0
Day 7
Day 14
Day 21
Total Lung Volume
Body Weight
B
LV/BW (mL/g)
0.10
0.08
0.06
0.04
0.02
0.00
Baseline
P < 0.05
Day 0
Day 7
Day 14
Day 21

FIGS. 14A-14B

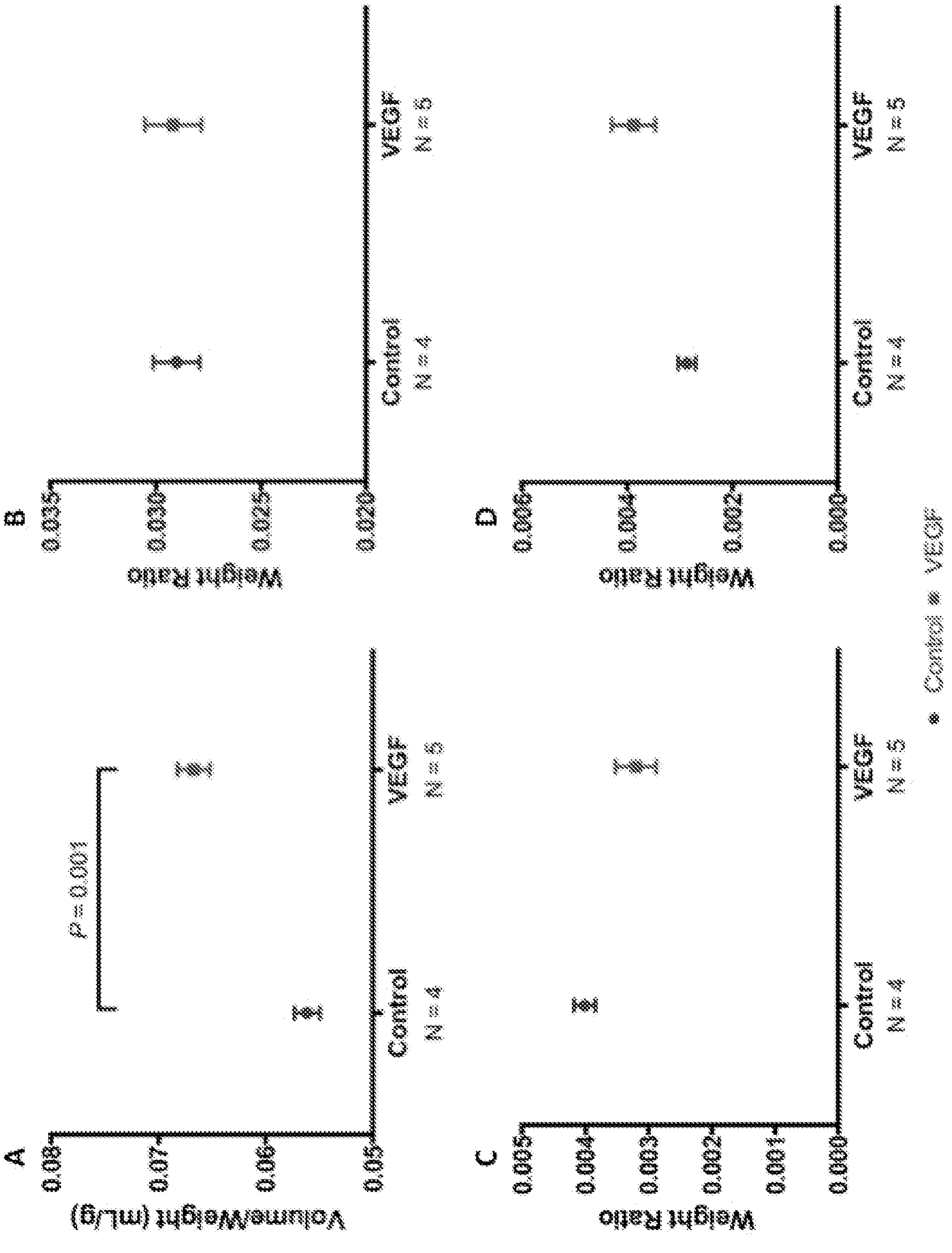
A
Volume/Weight (mL/g)
0.08
0.07
0.06
0.05
P = 0.001
Control
N = 4
VEGF
N = 5
B
Weight Ratio
0.035
0.030
0.025
0.020
Control
N = 4
VEGF
N = 5
C
Weight Ratio
0.005
0.004
0.003
0.002
0.001
0.000
Control
N = 4
VEGF
N = 5
D
Weight Ratio
0.006
0.004
0.002
0.000
Control
N = 4
VEGF
N = 5
Control
VEGF

FIGS. 15A-15D

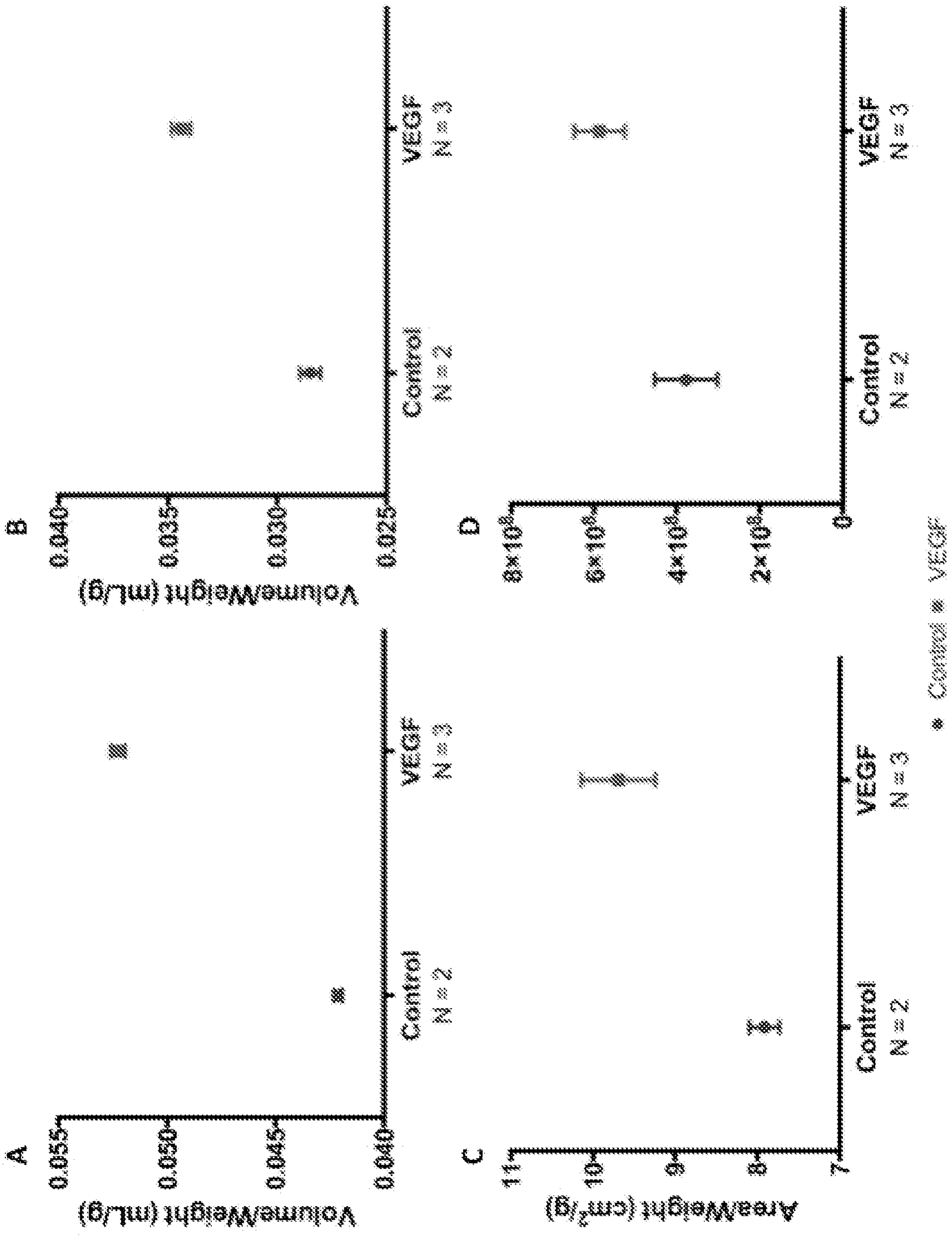
A
Volume/Weight (mL/g)
0.055
0.050
0.045
0.040
Control
N = 2
VEGF
N = 3
B
Volume/Weight (mL/g)
0.040
0.035
0.030
0.025
Control
N = 2
VEGF
N = 3
C
Area/Weight (cm²/g)
11
10
9
8
7
Control
N = 2
VEGF
N = 3
D
8×10⁸
6×10⁸
4×10⁸
2×10⁸
0
Control
N = 2
VEGF
N = 3
Control
VEGF

FIGS. 16A-16D

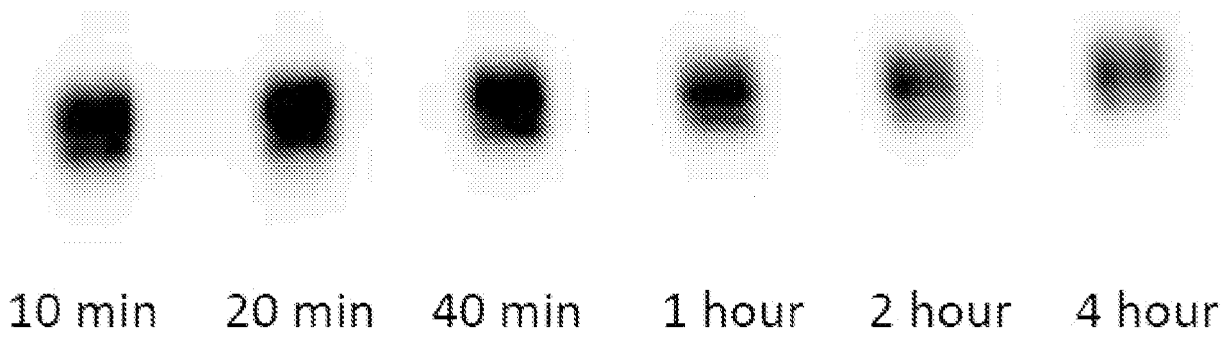


FIG. 17A

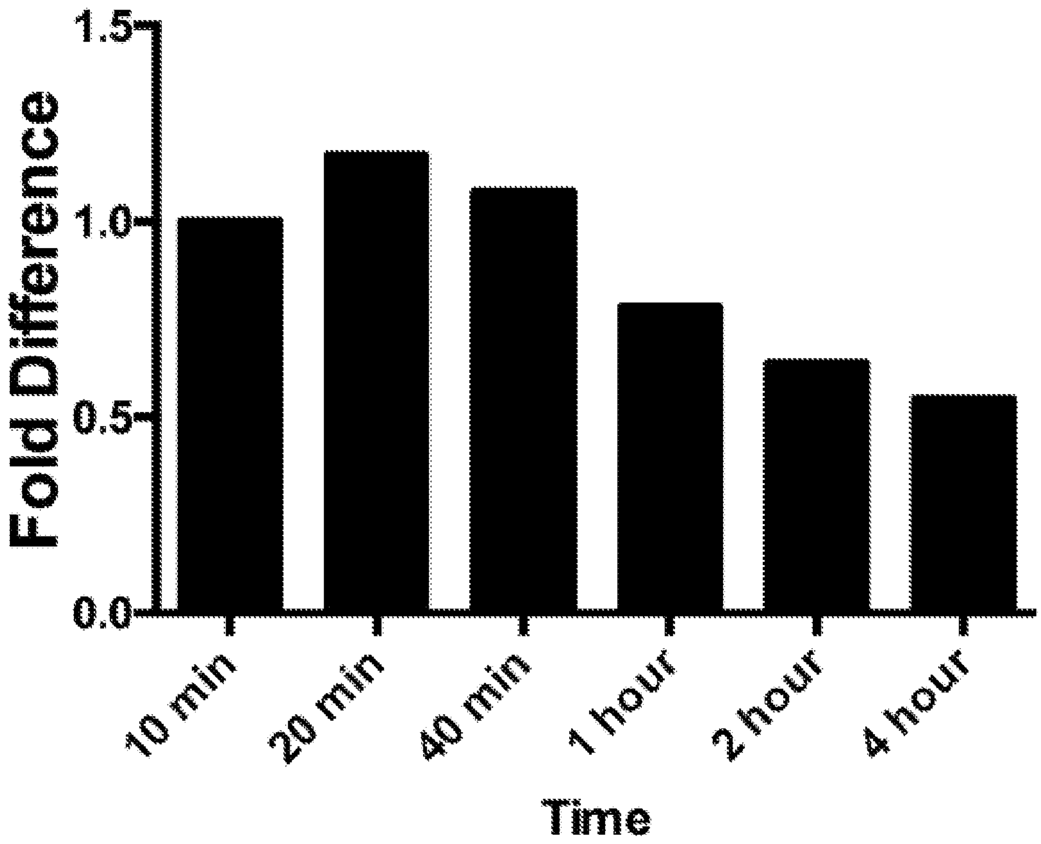


FIG. 17B

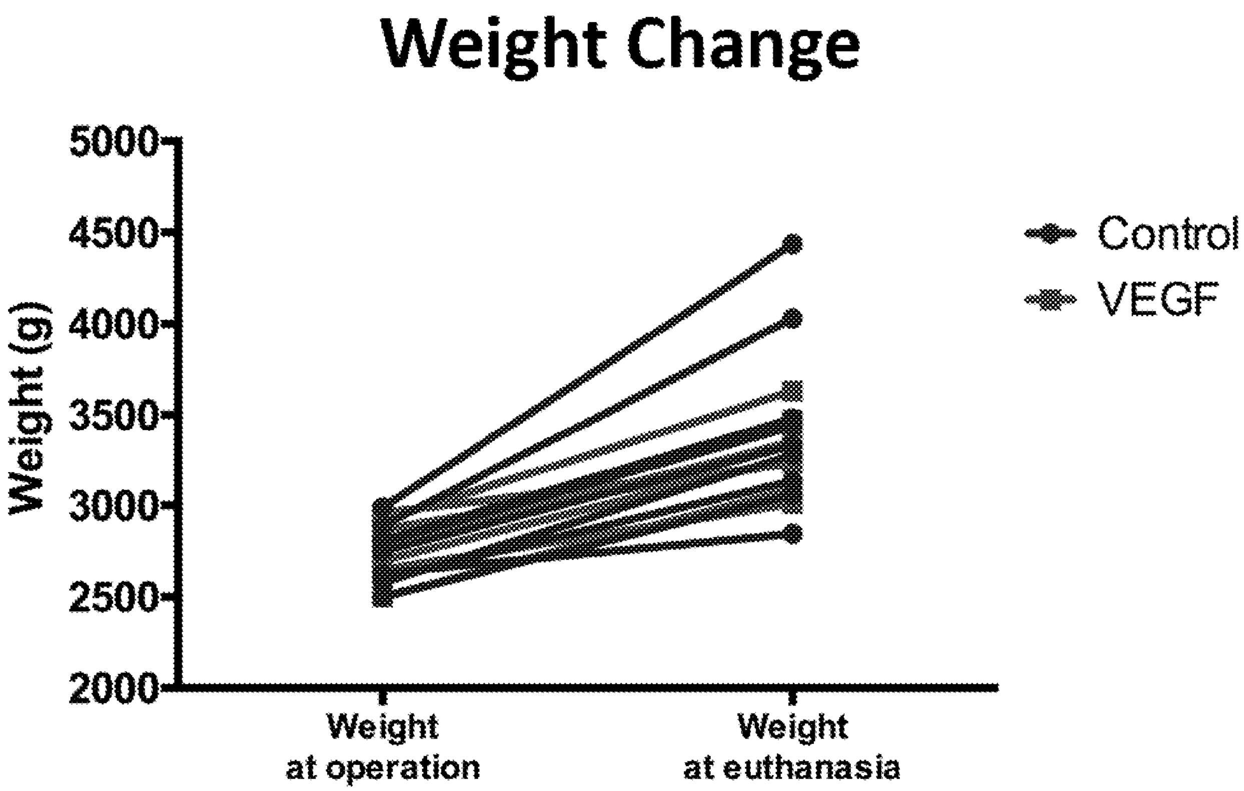
Weight Change
5000
4500
4000
3500
3000
2500
2000
Weight (g)
Weight
at operation
Weight
at euthanasia
Control
VEGF

FIG. 18

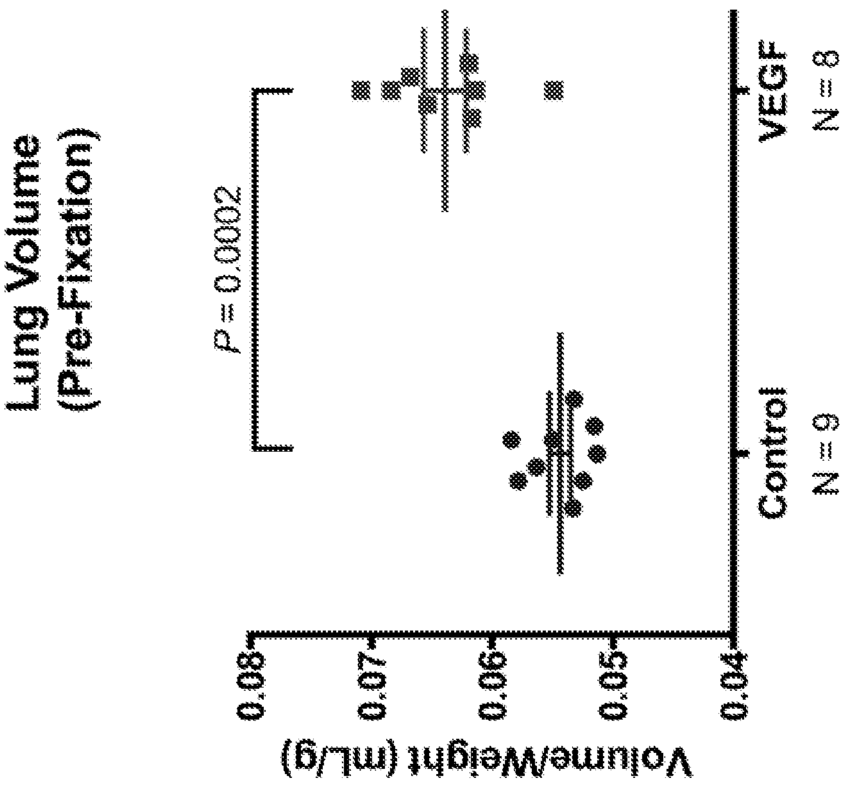


FIG. 19A

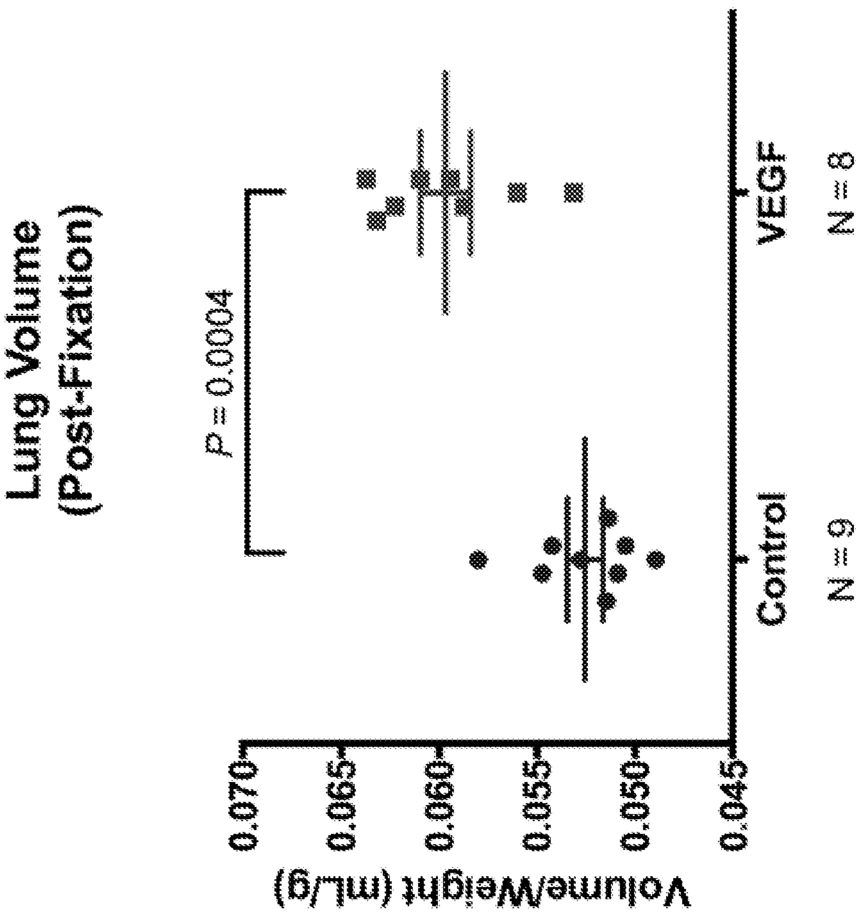


FIG. 19B

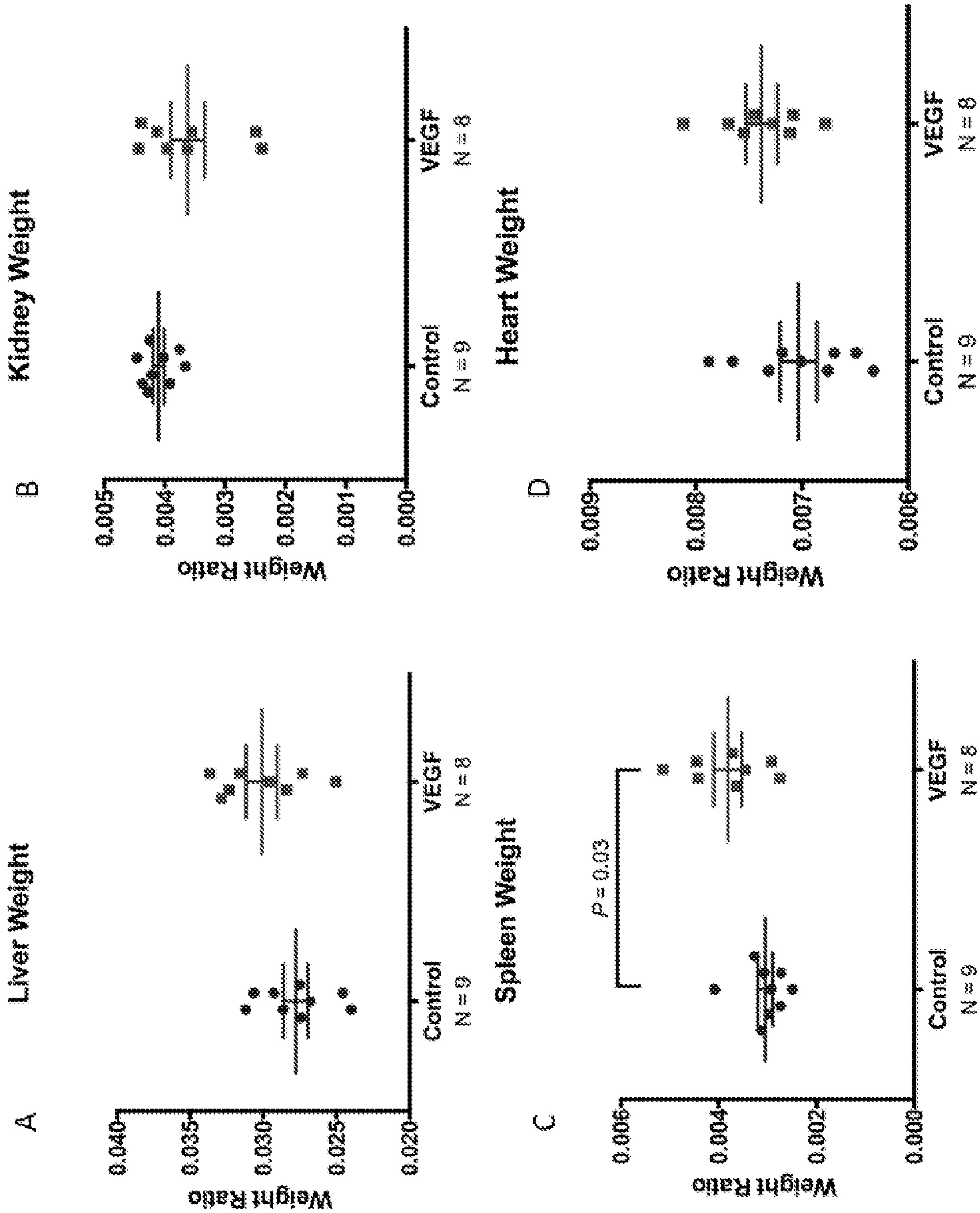
A
Liver Weight
Weight Ratio
0.040
0.035
0.030
0.025
0.020
Control
N = 9
VEGF
N = 8
B
Kidney Weight
Weight Ratio
0.005
0.004
0.003
0.002
0.001
0.000
Control
N = 9
VEGF
N = 8
C
Spleen Weight
Weight Ratio
0.006
0.004
0.002
0.000
P = 0.03
Control
N = 9
VEGF
N = 8
D
Heart Weight
Weight Ratio
0.009
0.008
0.007
0.006
Control
N = 9
VEGF
N = 8

FIGS. 20A-20D

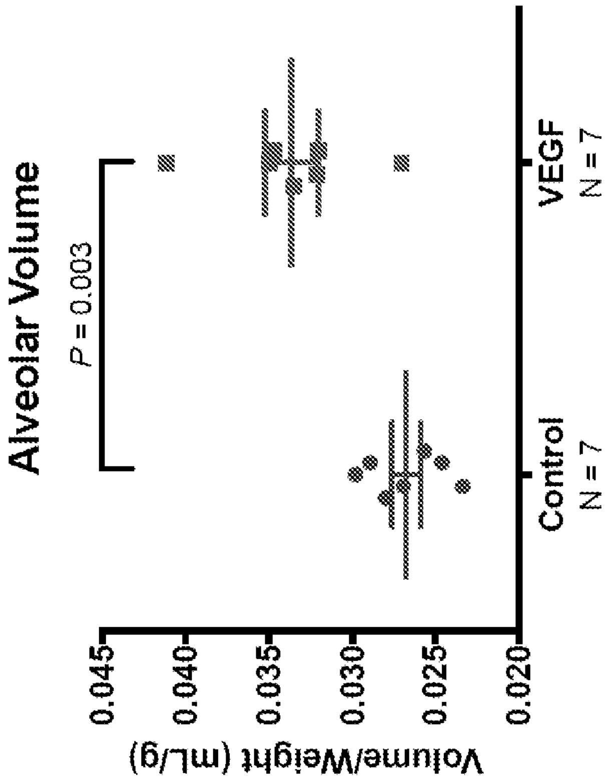


FIG. 21B

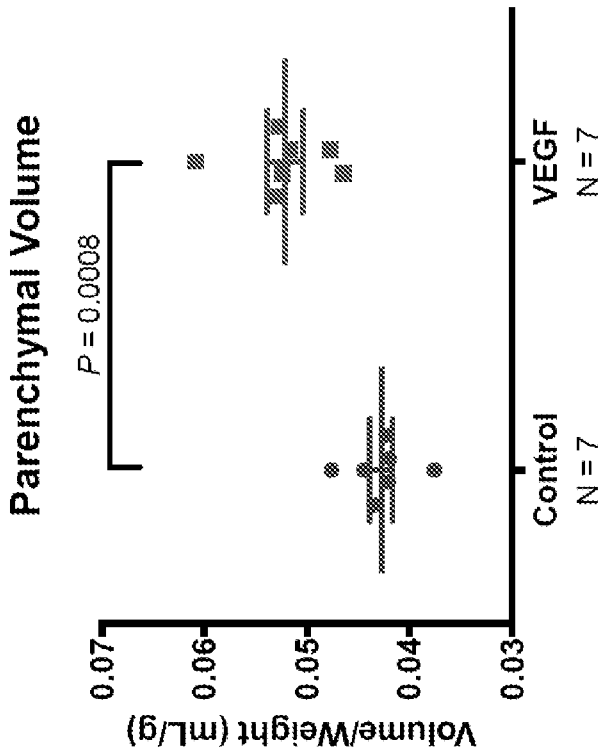


FIG. 21A

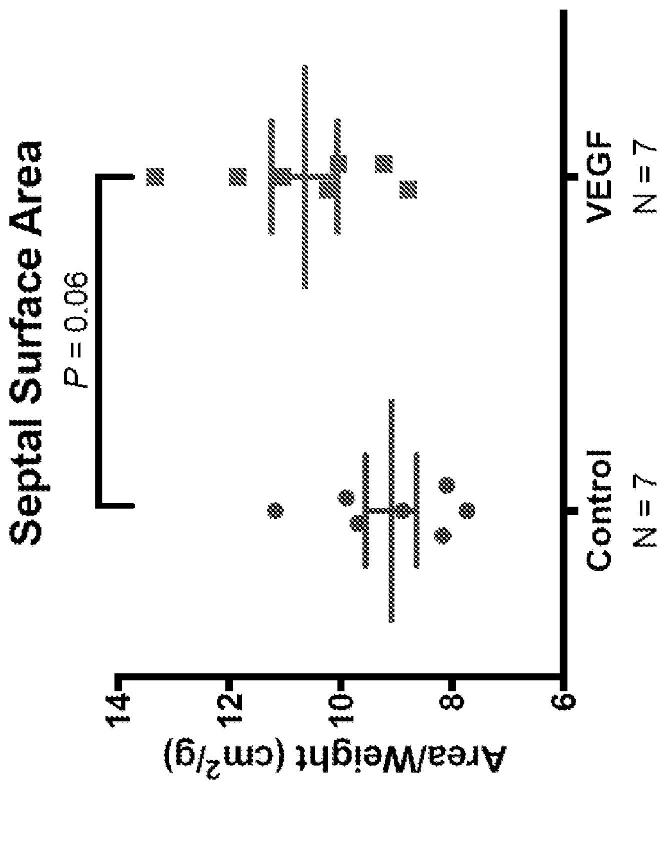


FIG. 21C

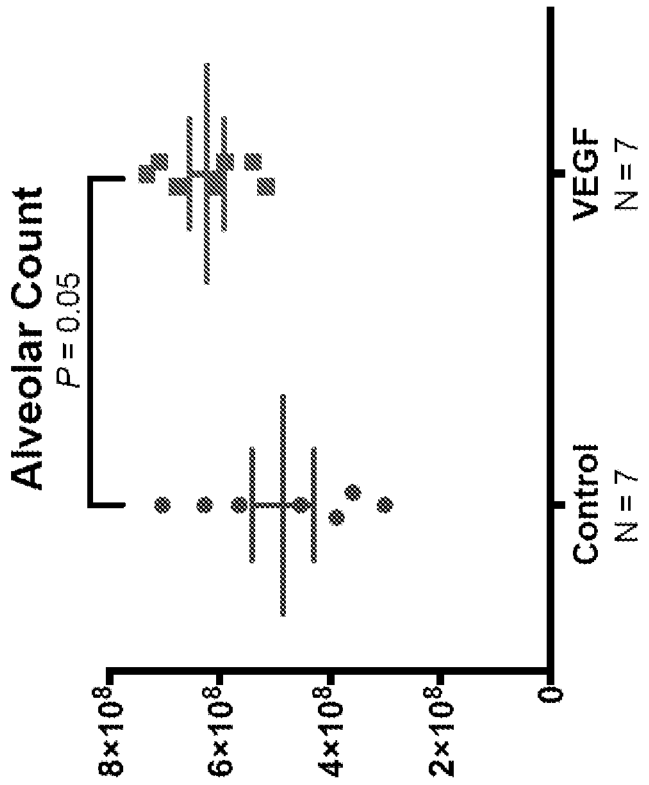


FIG. 21D

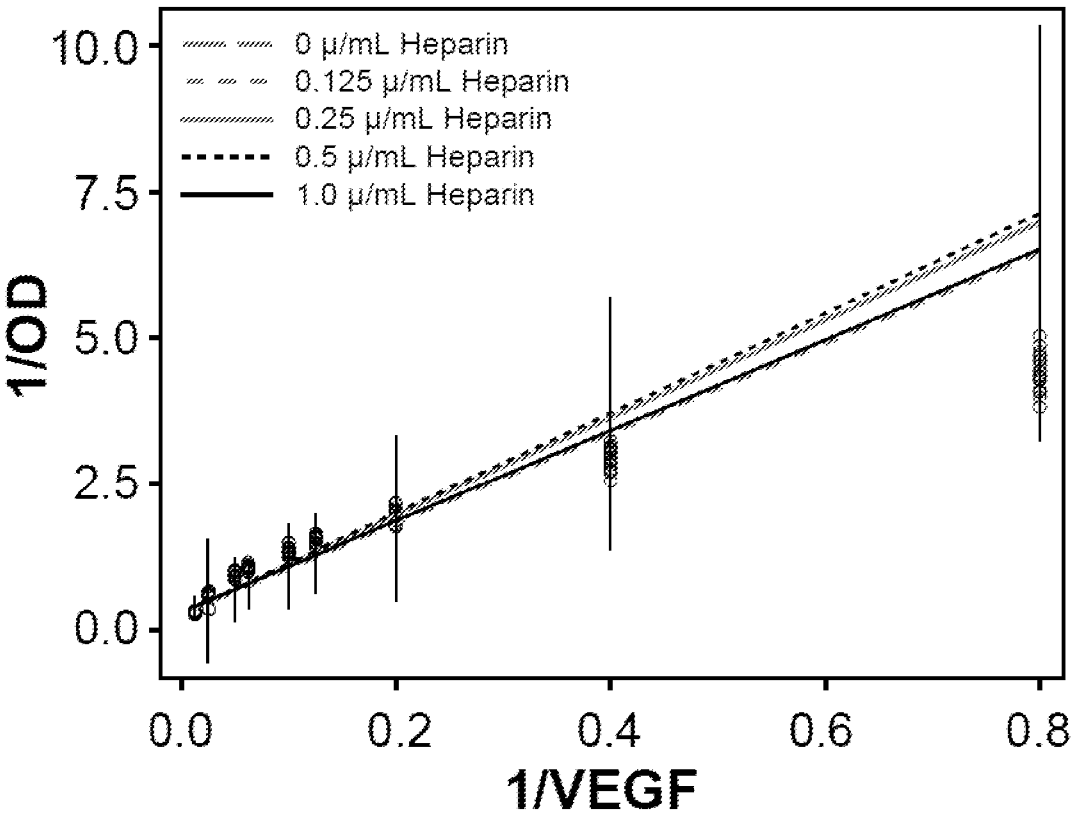


| Heparin Concentration | Kd (±SE) (ng/mL) |
|---|---|
| 0 µ/mL | 8.67 (± 0.59) |
| 0.125 µ/mL | 7.98 (± 0.59) |
| 0.25 µ/mL | 8.63 (± 0.59) |
| 0.5 µ/mL | 8.84 (± 0.59) |
| 1.0 µ/mL | 8.06 (± 0.59) |

FIG. 22A

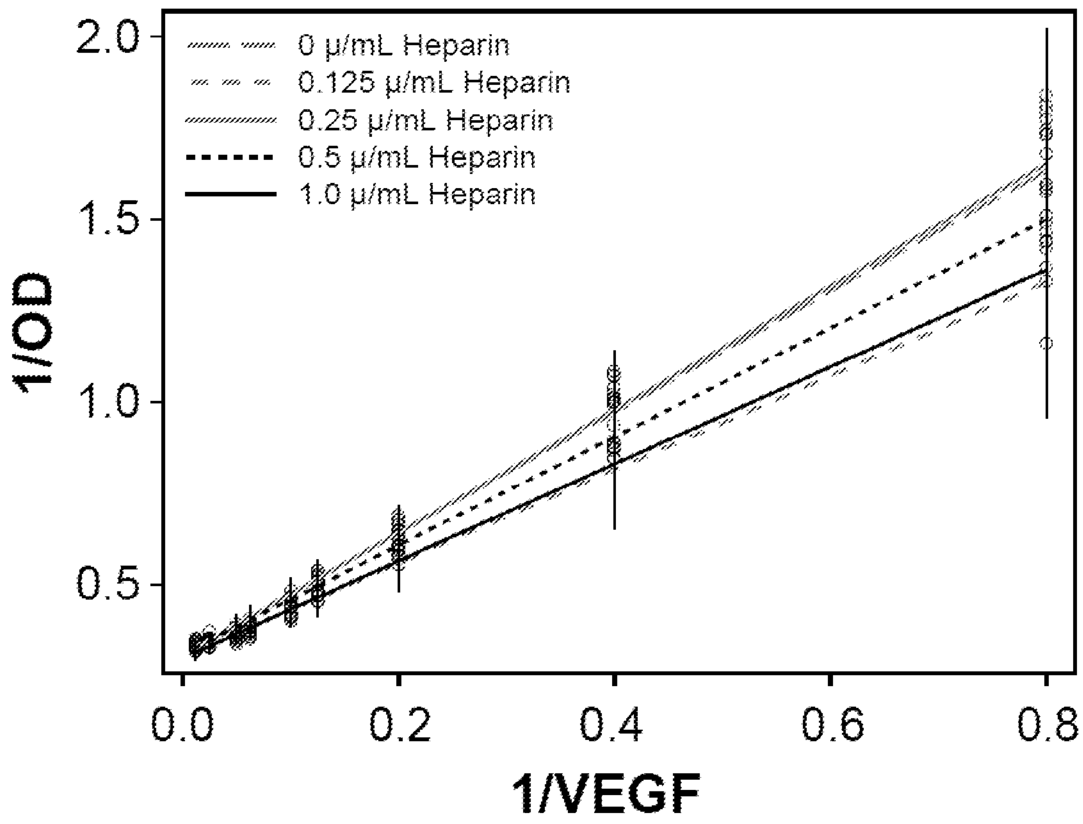


| Heparin Concentration | Kd (±SE) (ng/ml) |
|---|---|
| 0 μ/mL | 1.96 (± 0.05) |
| 0.125 μ/mL | 1.58 (± 0.05) |
| 0.25 μ/mL | 1.99 (± 0.05) |
| 0.5 μ/mL | 1.79 (± 0.05) |
| 1.0 μ/mL | 1.63 (± 0.05) |

| Comparison | P |
|---|---|
| Heparin (U/mL): 0 vs 0.125 | <.0001 |
| Heparin (U/mL): 0 vs 0.25 | 0.6888 |
| Heparin (U/mL): 0 vs 0.5 | 0.0544 |
| Heparin (U/mL): 0 vs 1 | <.0001 |
| Heparin (U/mL): 0.125 vs 0.25 | <.0001 |
| Heparin (U/mL): 0.125 vs 0.5 | 0.0243 |

FIG. 22B

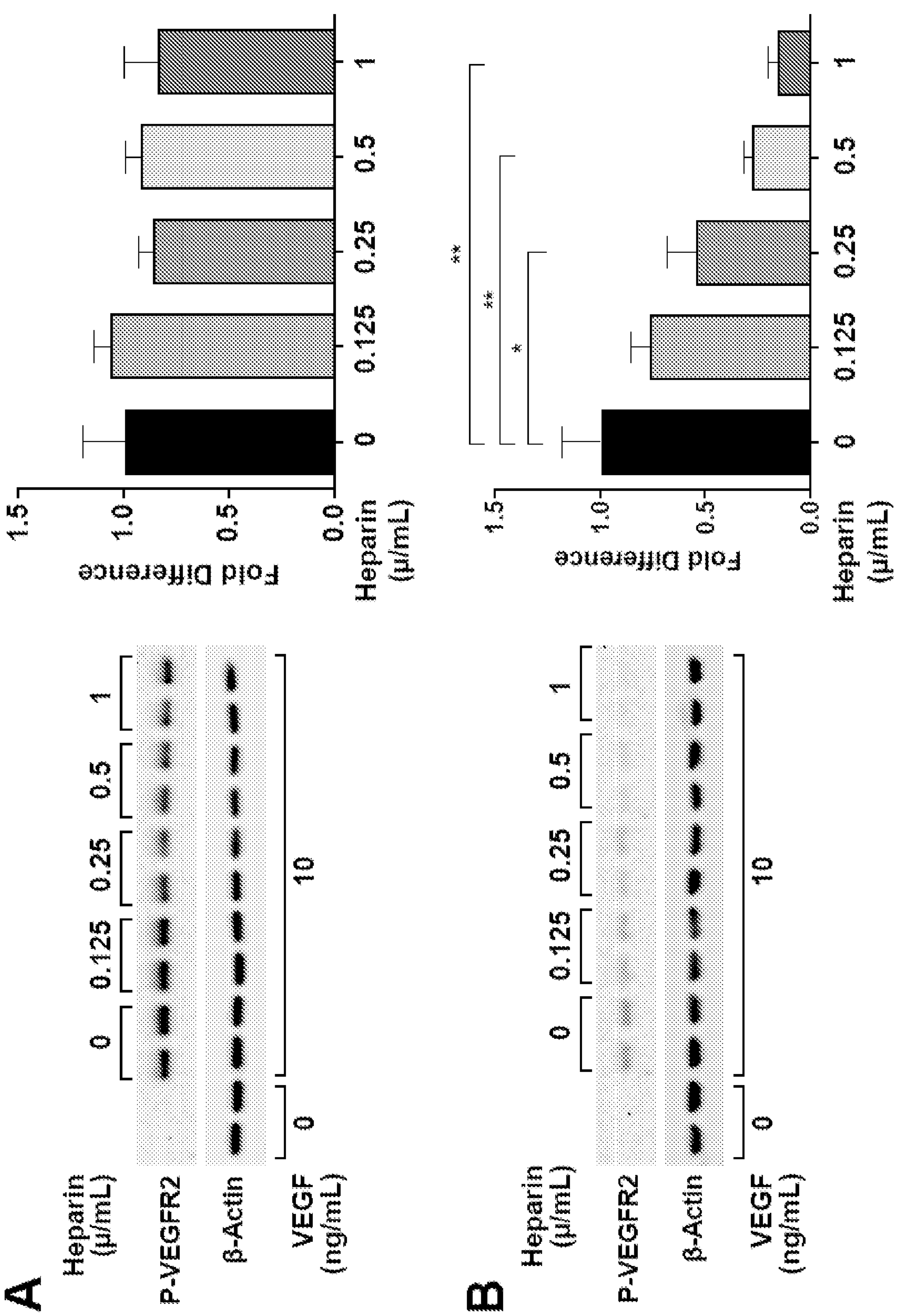
A
B
Heparin (µ/mL)
P-VEGFR2
β-Actin
VEGF (ng/mL)
0
0.125
0.25
0.5
1
10
Fold Difference
0.0
0.5
1.0
1.5
*
**
**

FIGS. 23A-23B

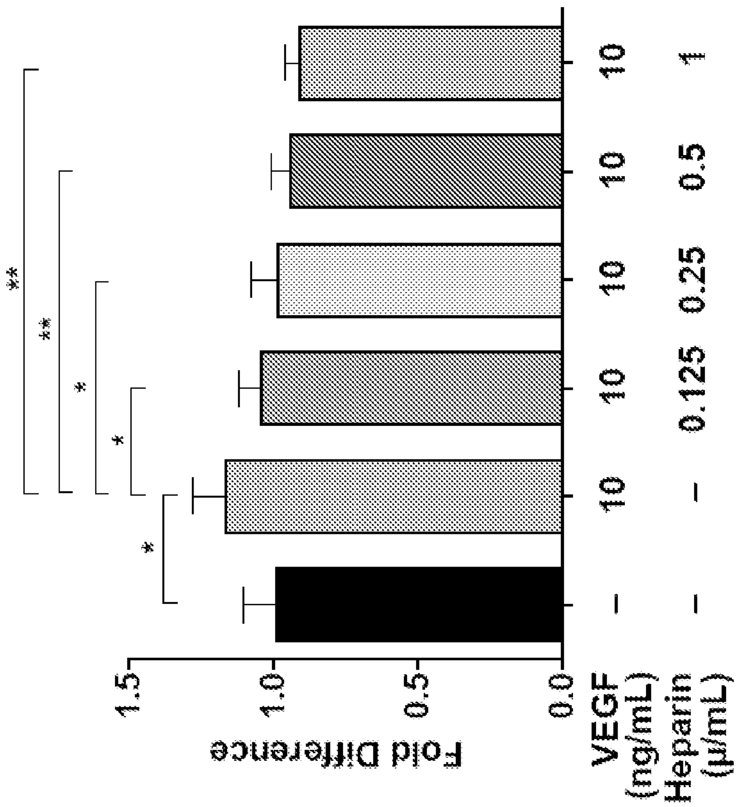


FIG. 23D

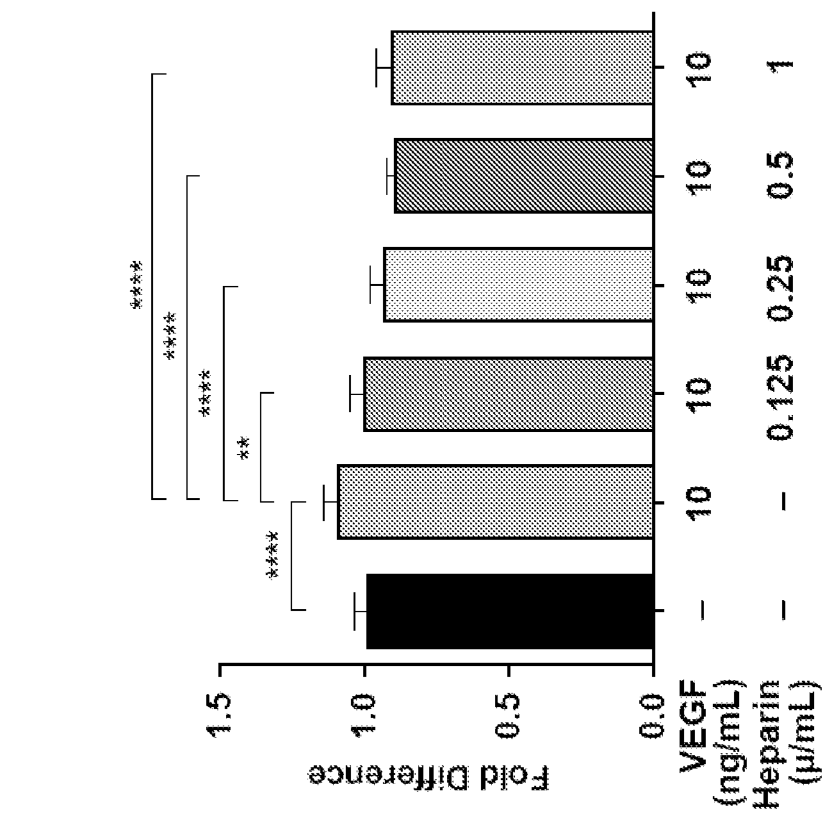


FIG. 23C`

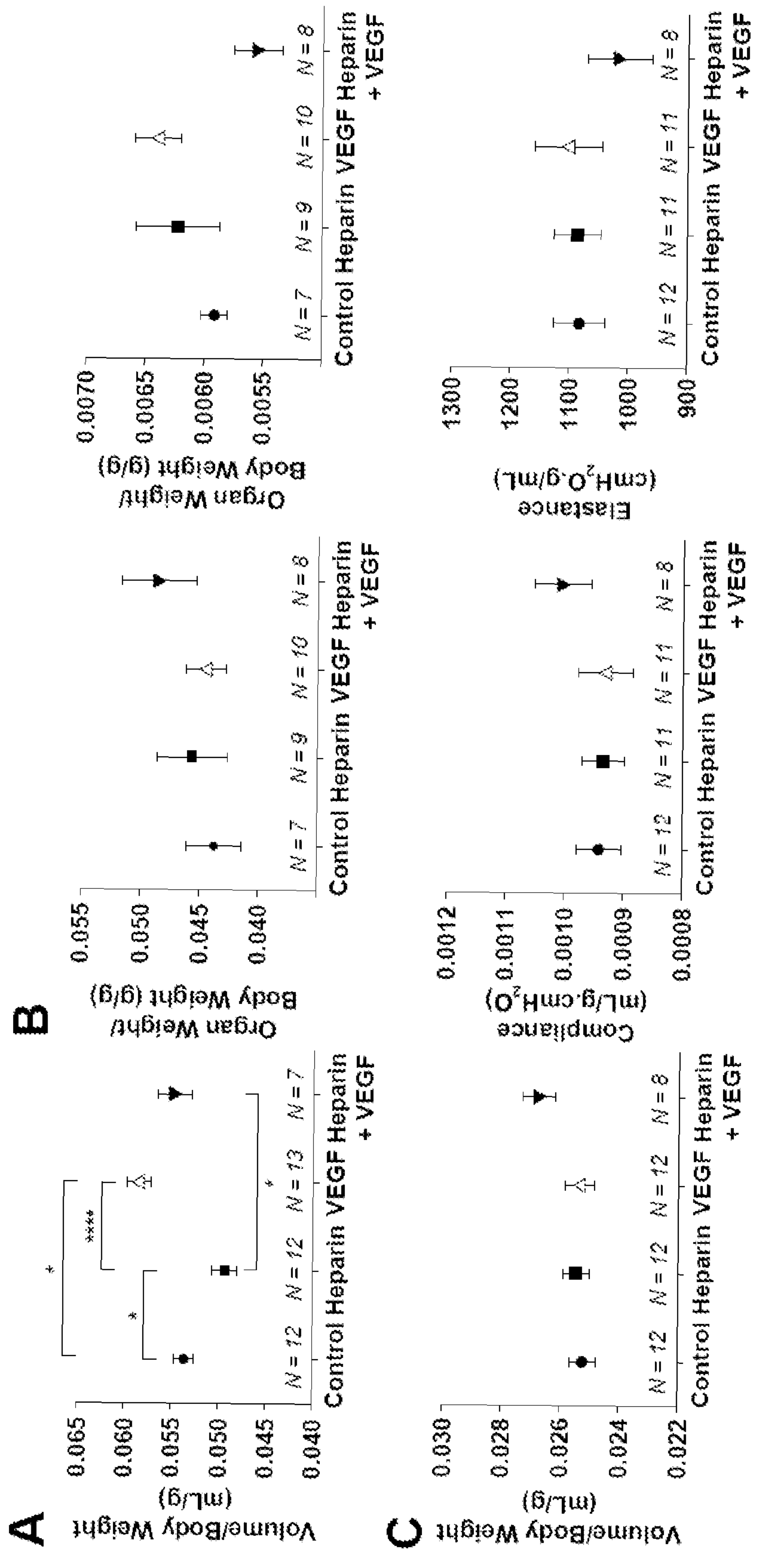
A
Volume/Body Weight (mL/g)
0.065
0.060
0.055
0.050
0.045
0.040
*
*
****
*
N = 12
N = 12
N = 13
N = 7
Control Heparin VEGF Heparin + VEGF
B
Organ Weight/ Body Weight (g/g)
0.055
0.050
0.045
0.040
N = 7
N = 9
N = 10
N = 8
Control Heparin VEGF Heparin + VEGF
Organ Weight/ Body Weight (g/g)
0.0070
0.0065
0.0060
0.0055
N = 7
N = 9
N = 10
N = 8
Control Heparin VEGF Heparin + VEGF
C
Volume/Body Weight (mL/g)
0.030
0.028
0.026
0.024
0.022
N = 12
N = 12
N = 12
N = 8
Control Heparin VEGF Heparin + VEGF
Compliance (mL/g.cmH2O)
0.0012
0.0011
0.0010
0.0009
0.0008
N = 12
N = 11
N = 11
N = 8
Control Heparin VEGF Heparin + VEGF
Elastance (cmH2O.g/mL)
1300
1200
1100
1000
900
N = 12
N = 11
N = 11
N = 8
Control Heparin VEGF Heparin + VEGF

FIGS. 24A-24C

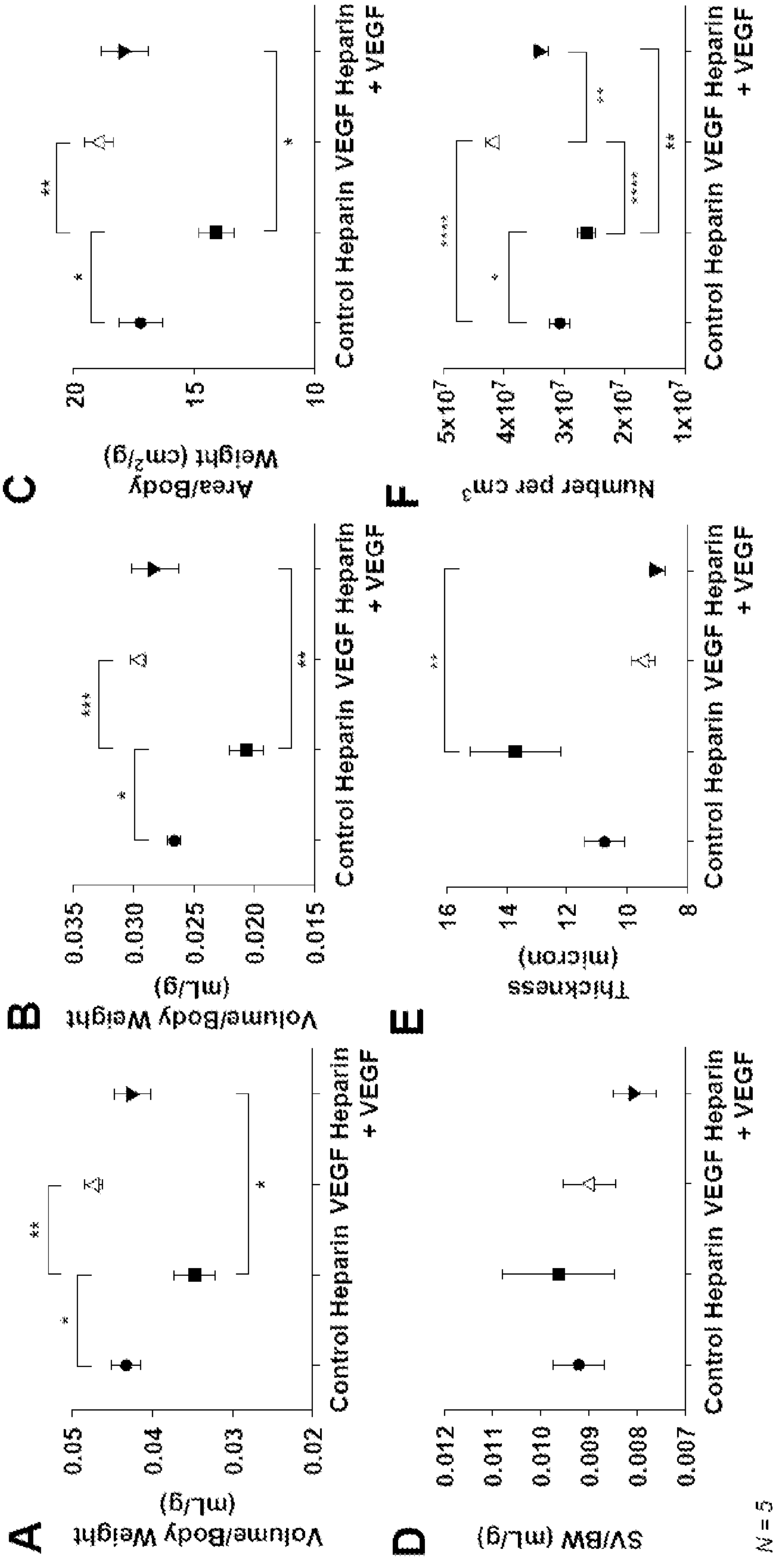
A
Volume/Body Weight (mL/g)
B
Volume/Body Weight (mL/g)
C
Area/Body Weight (cm²/g)
D
SV/BW (mL/g)
E
Thickness (micron)
F
Number per cm³
Control Heparin VEGF Heparin + VEGF
N = 5

FIGS. 25A-25F

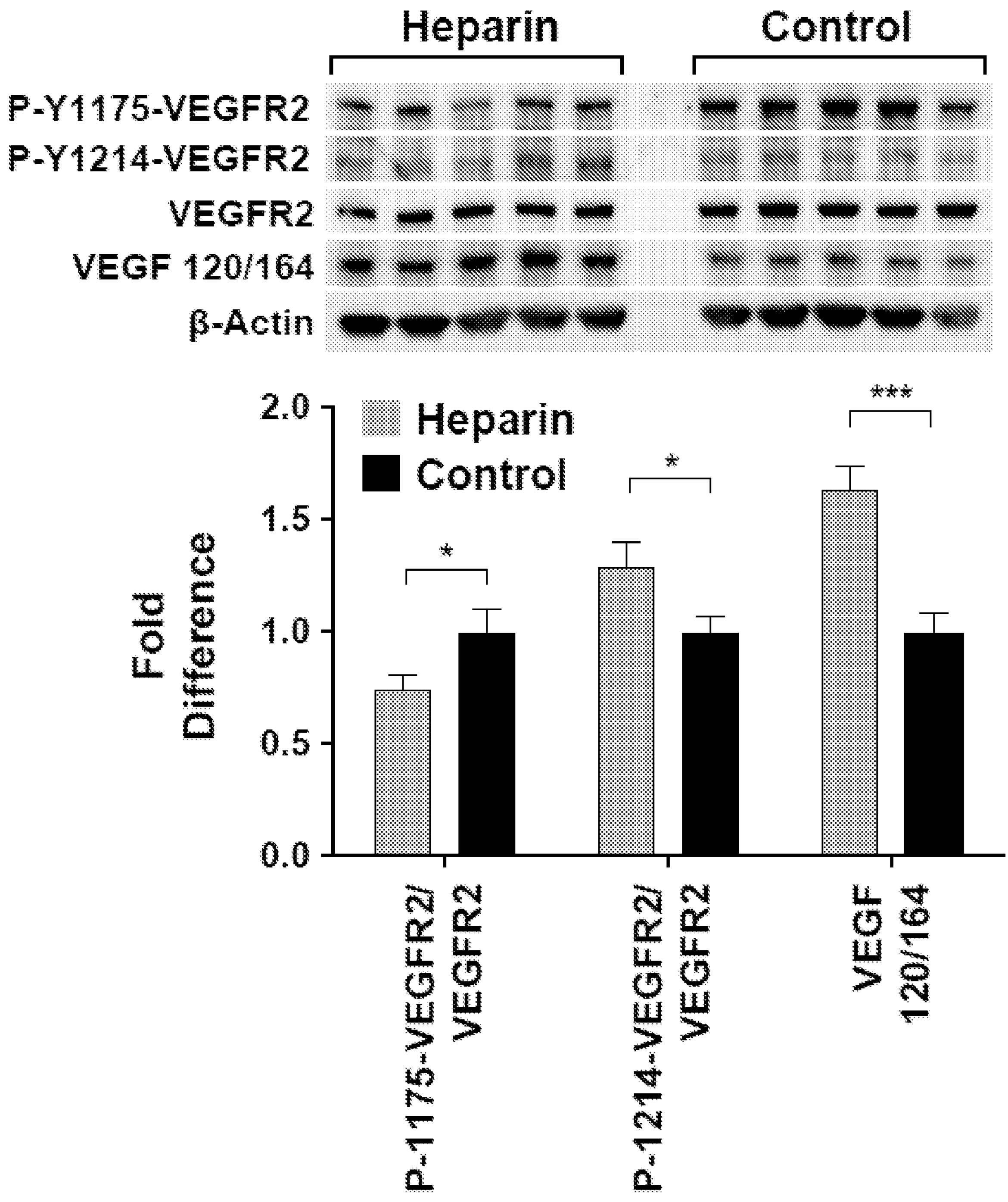
Heparin
Control
P-Y1175-VEGFR2
P-Y1214-VEGFR2
VEGFR2
VEGF 120/164
β-Actin
2.0
1.5
1.0
0.5
0.0
Heparin
Control
Fold Difference
*
*
***
P-1175-VEGFR2/ VEGFR2
P-1214-VEGFR2/ VEGFR2
VEGF 120/164

FIG. 26A

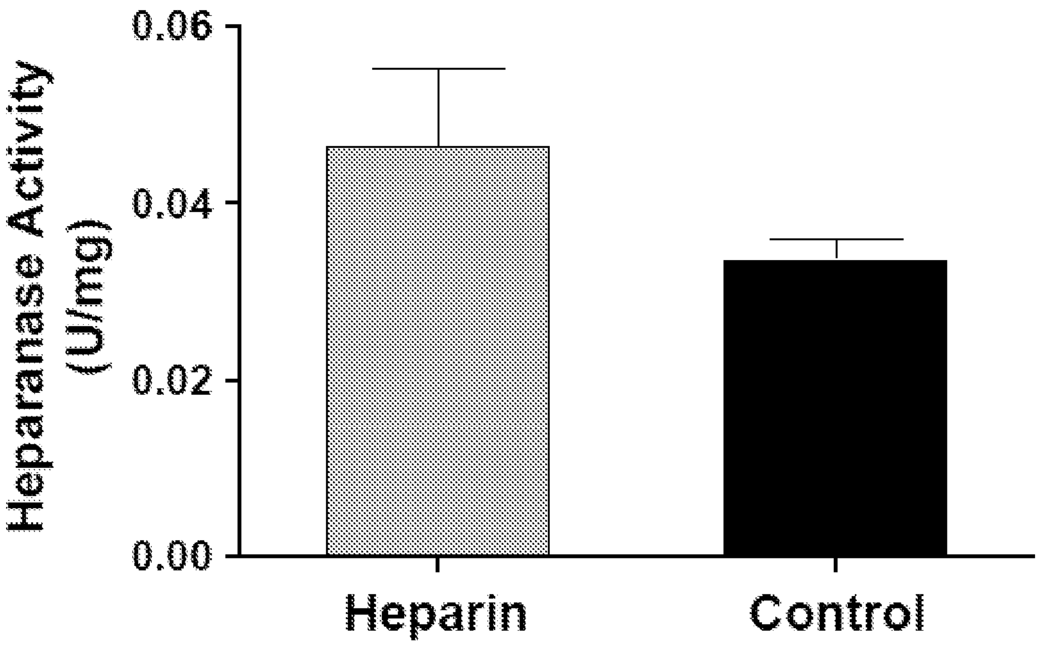


FIG. 26B

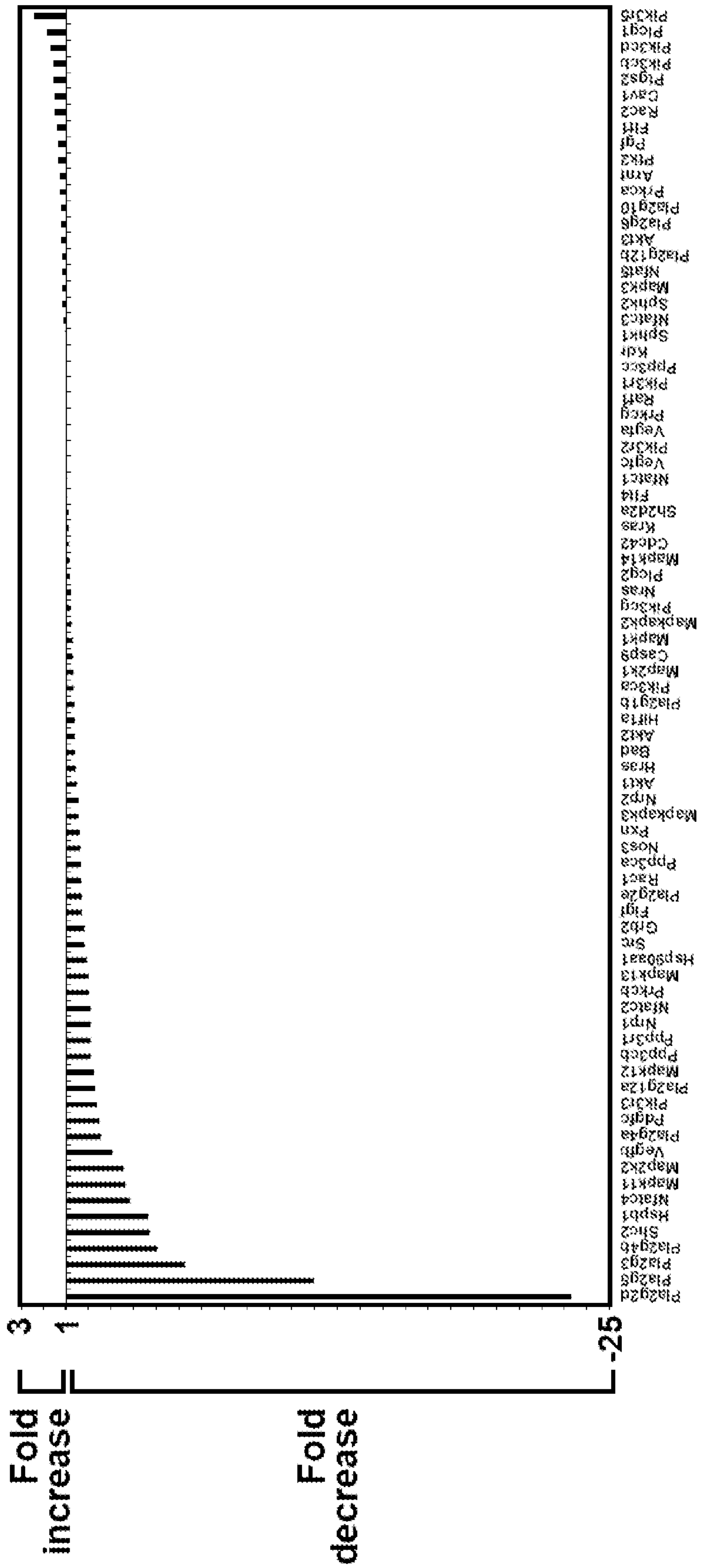
Fold increase
Fold decrease
3
1
-25
Pla2g2d
Pla2g5
Pla2g3
Pla2g4b
Shc2
Hspb1
Nfatc4
Mapk11
Map2k2
Vegfb
Pla2g4a
Pdgfc
Pik3r3
Pla2g12a
Mapk12
Ppp3cb
Ppp3r1
Nrp1
Nfatc2
Prkcb
Mapk13
Hsp90aa1
Src
Grb2
Figf
Pla2g2e
Rac1
Ppp3ca
Nos3
Pxn
Mapkapk3
Nrp2
Akt1
Hras
Bad
Akt2
Hif1a
Pla2g1b
Pik3ca
Map2k1
Casp9
Mapk1
Mapkapk2
Pik3cg
Nras
Plcg2
Mapk14
Cdc42
Kras
Sh2d2a
Flt4
Nfatc1
Vegfc
Pik3r2
Vegfa
Prkcg
Raf1
Pik3r1
Ppp3cc
Kdr
Sphk1
Nfatc3
Sphk2
Mapk3
Nfat5
Pla2g12b
Akt3
Pla2g6
Pla2g10
Prkca
Arnt
Ptk2
Pgf
Flt1
Rac2
Cav1
Ptgs2
Pik3cb
Pik3cd
Plcg1
Pik3r5

FIG. 27

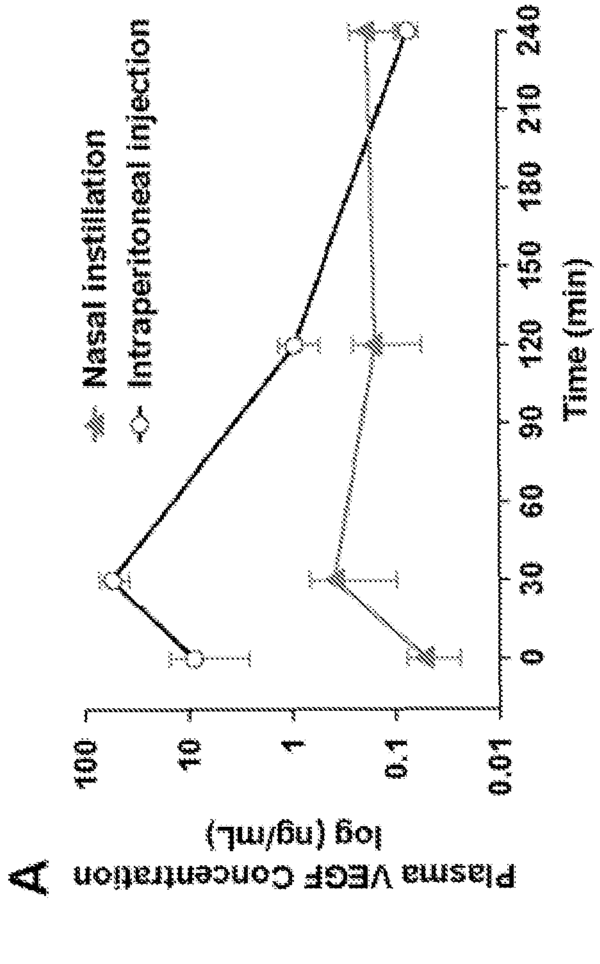


FIG. 28A

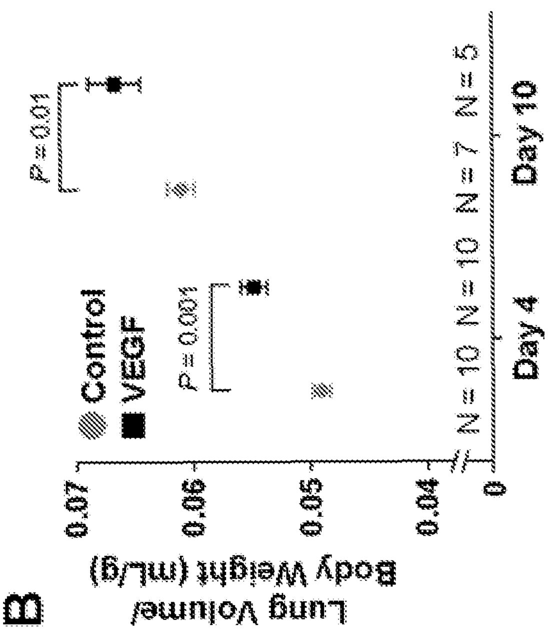


FIG. 28B

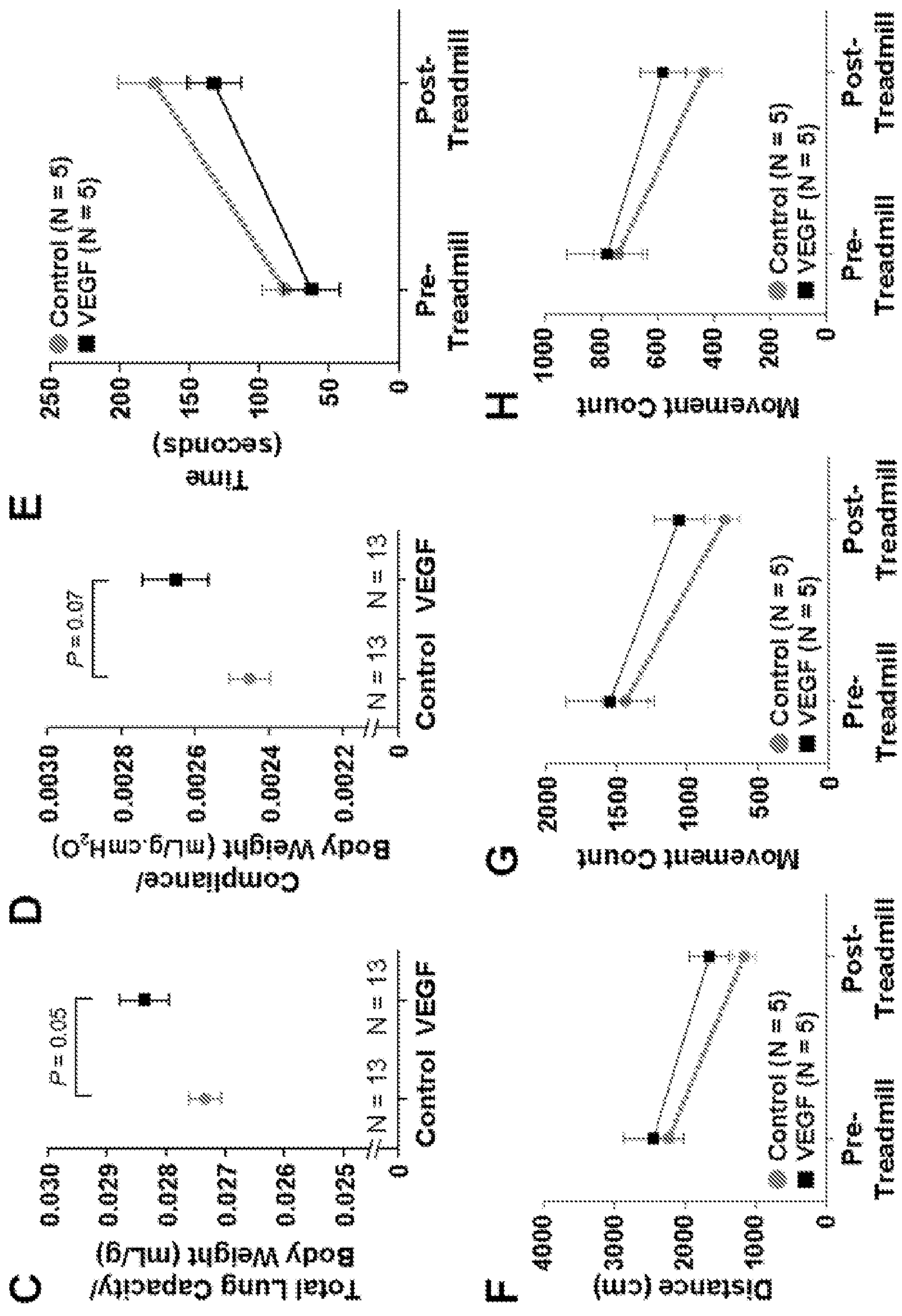
C
Total Lung Capacity/
Body Weight (mL/g)
0.030
0.029
0.028
0.027
0.026
0.025
0
P = 0.05
N = 13
N = 13
Control
VEGF
D
Compliance/
Body Weight (mL/g.cmH2O)
0.0030
0.0028
0.0026
0.0024
0.0022
0
P = 0.07
N = 13
N = 13
Control
VEGF
E
Time
(seconds)
250
200
150
100
50
0
Control (N = 5)
VEGF (N = 5)
Pre-
Treadmill
Post-
Treadmill
F
Distance (cm)
4000
3000
2000
1000
0
Control (N = 5)
VEGF (N = 5)
Pre-
Treadmill
Post-
Treadmill
G
Movement Count
2000
1500
1000
500
0
Control (N = 5)
VEGF (N = 5)
Pre-
Treadmill
Post-
Treadmill
H
Movement Count
1000
800
600
400
200
0
Control (N = 5)
VEGF (N = 5)
Pre-
Treadmill
Post-
Treadmill

FIGS. 28C-28H

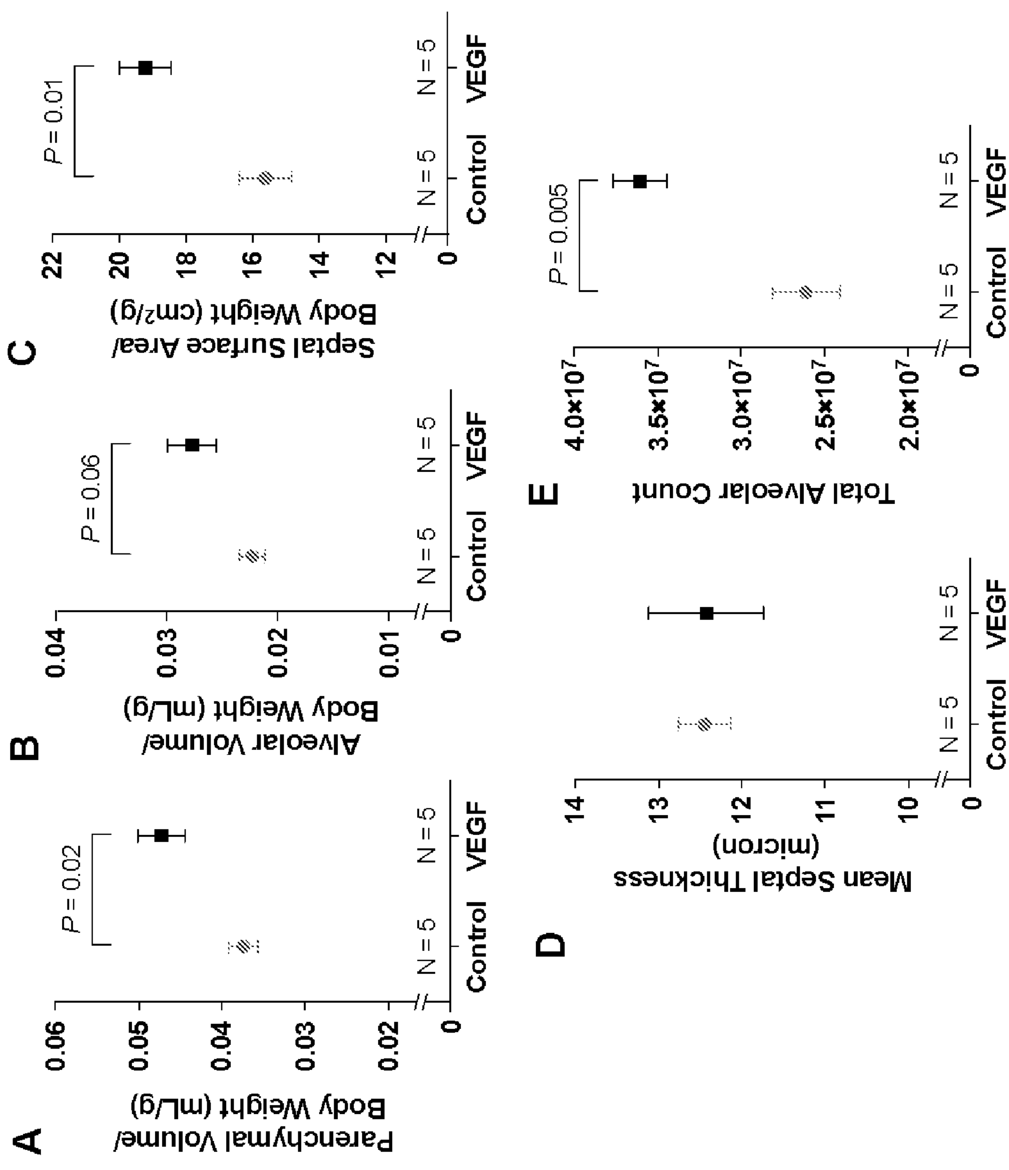
A
Parenchymal Volume/
Body Weight (mL/g)
0.06
0.05
0.04
0.03
0.02
0
P = 0.02
N = 5
N = 5
Control
VEGF
B
Alveolar Volume/
Body Weight (mL/g)
0.04
0.03
0.02
0.01
0
P = 0.06
N = 5
N = 5
Control
VEGF
C
Septal Surface Area/
Body Weight (cm²/g)
22
20
18
16
14
12
0
P = 0.01
N = 5
N = 5
Control
VEGF
D
Mean Septal Thickness
(micron)
14
13
12
11
10
0
N = 5
N = 5
Control
VEGF
E
Total Alveolar Count
4.0×10⁷
3.5×10⁷
3.0×10⁷
2.5×10⁷
2.0×10⁷
0
P = 0.005
N = 5
N = 5
Control
VEGF

FIGS. 29A-29E

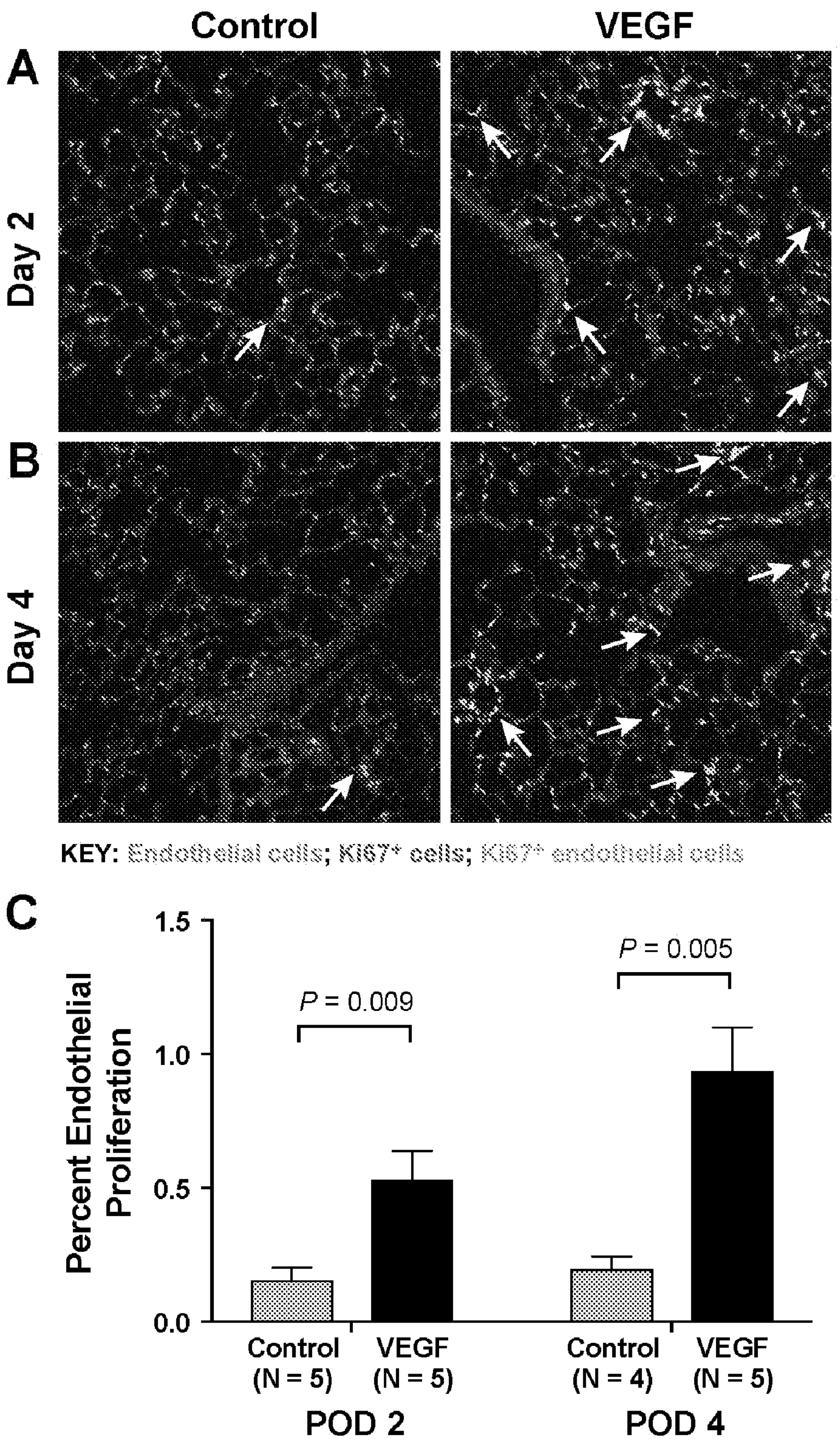
Control
VEGF
A
Day 2
B
Day 4
KEY: Endothelial cells; Ki67+ cells; Ki67+ endothelial cells
C
Percent Endothelial Proliferation
1.5
1.0
0.5
0.0
P = 0.009
P = 0.005
Control (N = 5)
VEGF (N = 5)
Control (N = 4)
VEGF (N = 5)
POD 2
POD 4

FIGS. 30A-30C

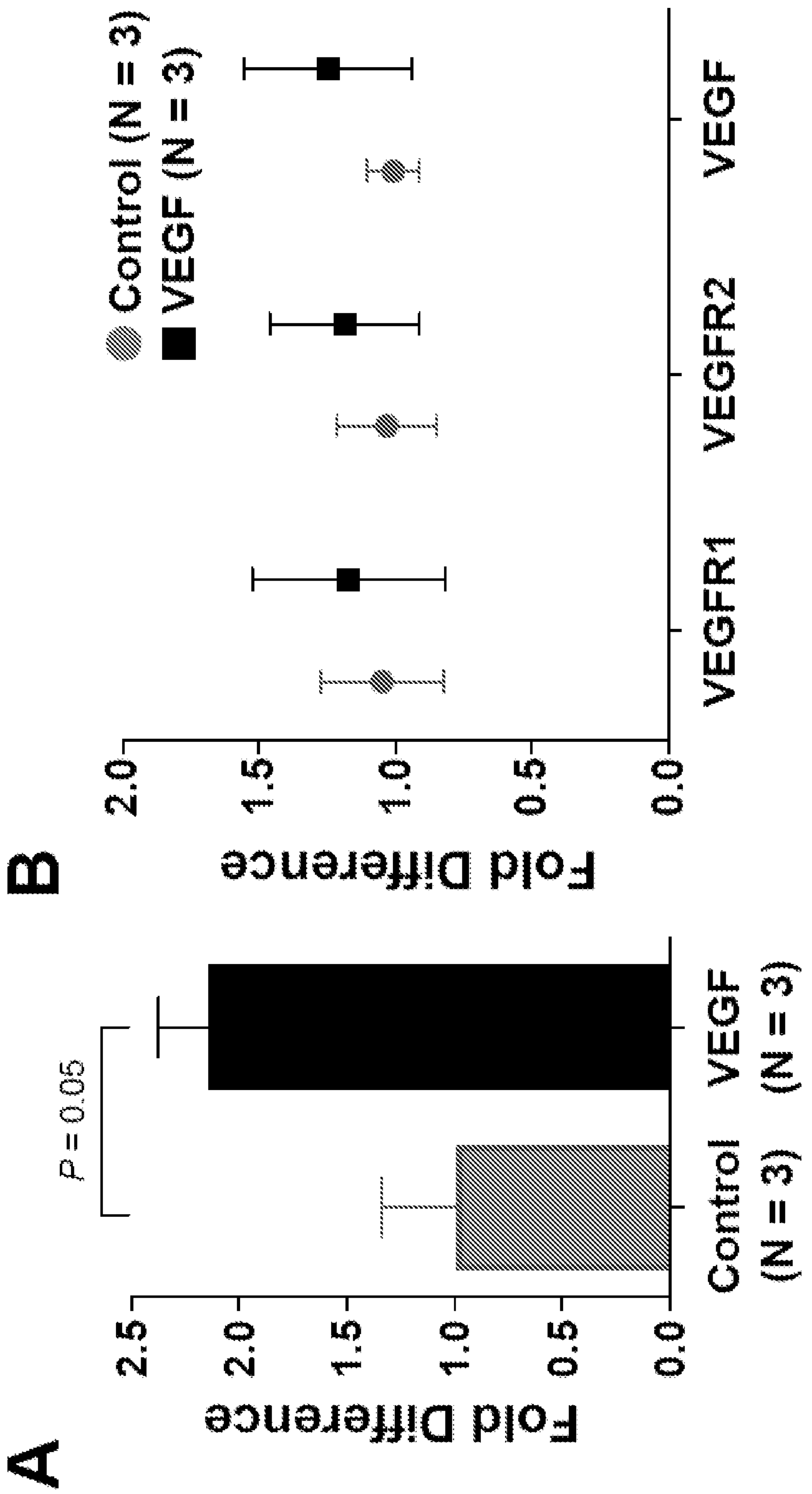
A
Fold Difference
2.5
2.0
1.5
1.0
0.5
0.0
P = 0.05
Control (N = 3)
VEGF (N = 3)
B
Fold Difference
2.0
1.5
1.0
0.5
0.0
Control (N = 3)
VEGF (N = 3)
VEGFR1
VEGFR2
VEGF

FIGS. 31A-31B

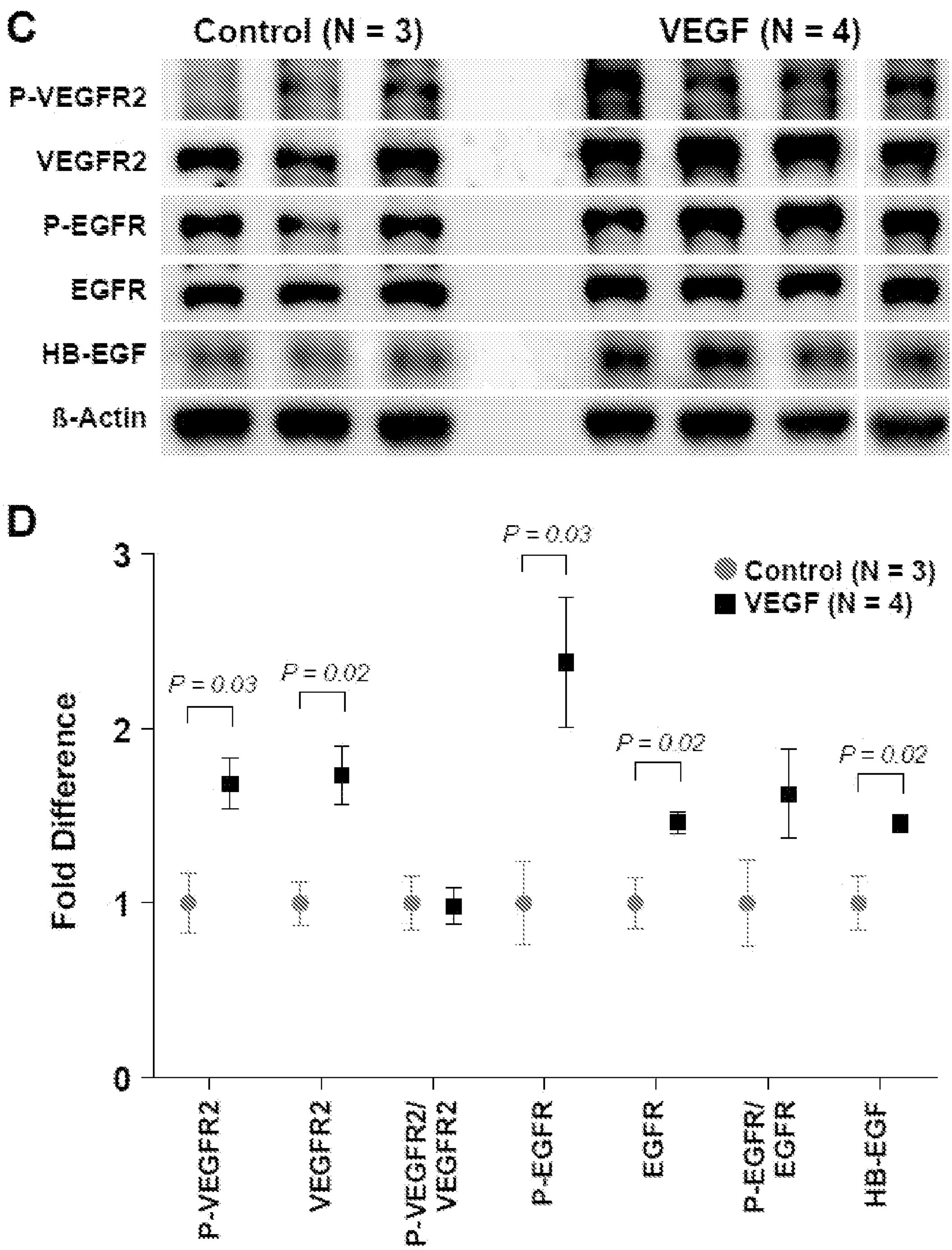
C
Control (N = 3)
VEGF (N = 4)
P-VEGFR2
VEGFR2
P-EGFR
EGFR
HB-EGF
ß-Actin
D
Fold Difference
0
1
2
3
Control (N = 3)
VEGF (N = 4)
P = 0.03
P = 0.02
P = 0.03
P = 0.02
P = 0.02
P-VEGFR2
VEGFR2
P-VEGFR2/ VEGFR2
P-EGFR
EGFR
P-EGFR/ EGFR
HB-EGF

FIGS. 31C-31D

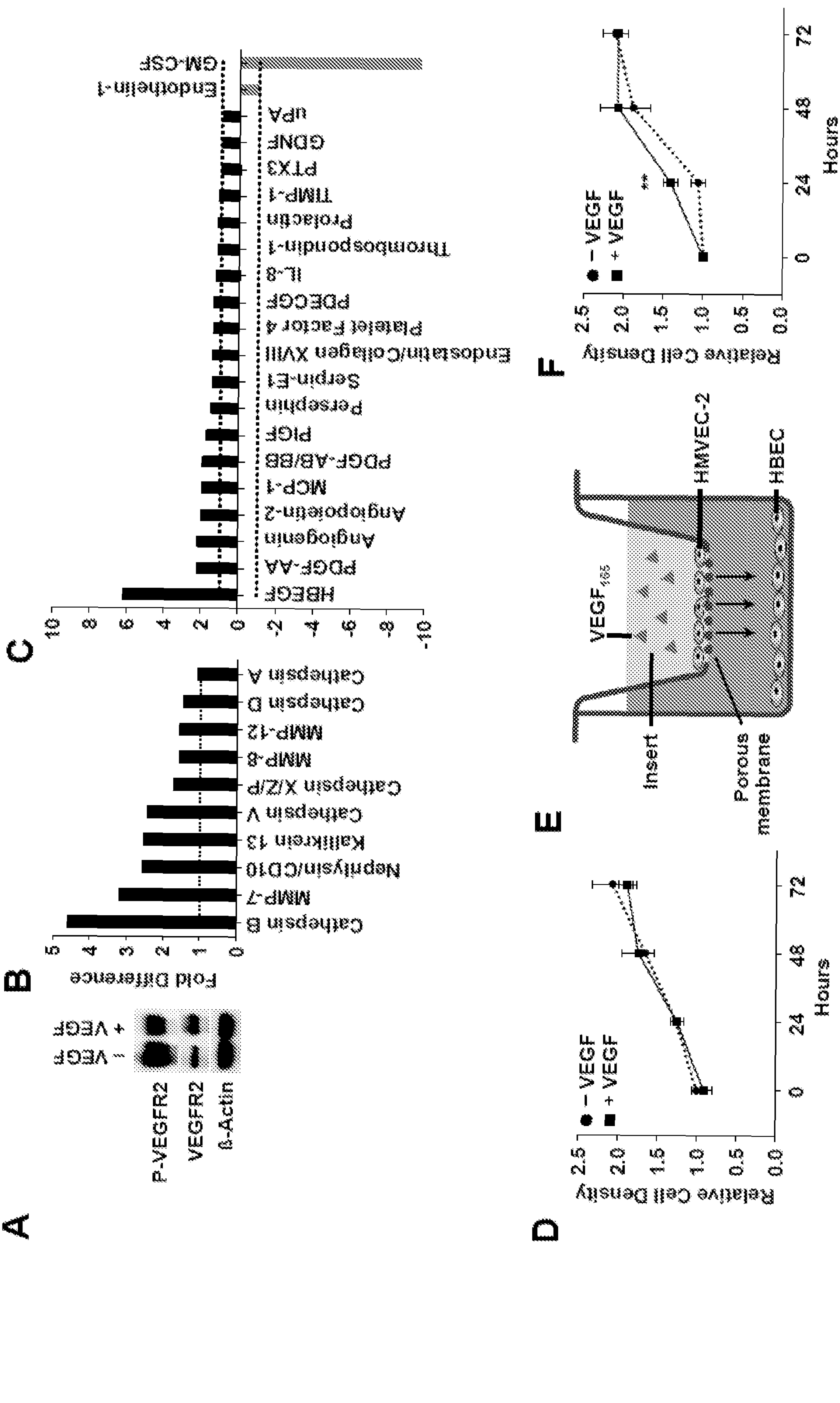
A
P-VEGFR2
VEGFR2
β-Actin
− VEGF
+ VEGF
B
Fold Difference
Cathepsin B
MMP-7
Neprilysin/CD10
Kallikrein 13
Cathepsin V
Cathepsin X/Z/P
MMP-8
MMP-12
Cathepsin D
Cathepsin A
C
HBEGF
PDGF-AA
Angiogenin
Angiopoietin-2
MCP-1
PDGF-AB/BB
PlGF
Persephin
Serpin-E1
Endostatin/Collagen XVIII
Platelet Factor 4
PDECGF
IL-8
Thrombospondin-1
Prolactin
TIMP-1
PTX3
GDNF
uPA
Endothelin-1
GM-CSF
D
Relative Cell Density
Hours
E
Insert
Porous membrane
VEGF165
HMVEC-2
HBEC
F
Relative Cell Density
Hours

FIGS. 32A-32F

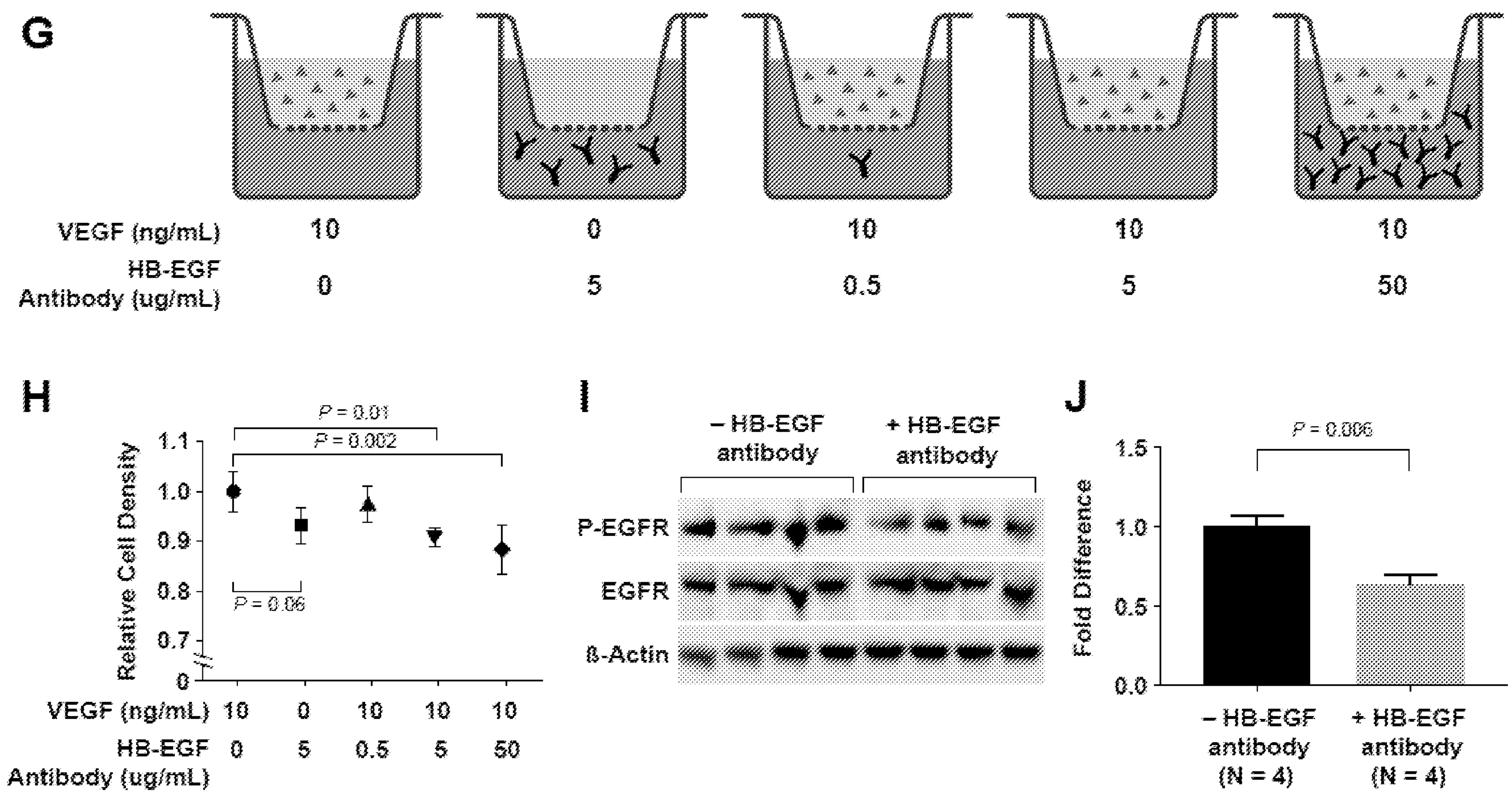
G
VEGF (ng/mL)
10
0
10
10
10
HB-EGF Antibody (ug/mL)
0
5
0.5
5
50
H
Relative Cell Density
1.1
1.0
0.9
0.8
0.7
0
P = 0.01
P = 0.002
P = 0.06
VEGF (ng/mL) 10 0 10 10 10
HB-EGF Antibody (ug/mL) 0 5 0.5 5 50
I
– HB-EGF antibody
+ HB-EGF antibody
P-EGFR
EGFR
ß-Actin
J
Fold Difference
1.5
1.0
0.5
0.0
P = 0.006
– HB-EGF antibody (N = 4)
+ HB-EGF antibody (N = 4)

FIGS. 32G-32J

FIG. 33

**VEGF-164 (VEGF164_Mouse)(SEQ ID NO: 1)**

```
MAPTTEGEQK SHEVIKFMDV YQRSYCRPIE TLVDIFQEYP
DEIEYIFKPS CVPLMRCAGC CNDEALECVP TSESNITMQI
MRIKPHQSQH IGEMSFLQHS RCECRPKKDR TKPEKHCEPC
SERRKHLFVQ DPQTCKCSCK NTDSRCKARQ LELNERTCRC
DKPRR
```

**VEGF-165 (SEQ ID NO: 2)**
**(UniProtKB/TrEMBL: P15692-4)**

```
        10         20         30         40         50
MNFLLSWVHW SLALLLYLHH AKWSQAAPMA EGGGQNHHEV VKFMDVYQRS
        60         70         80         90        100
YCHPIETLVD IFQEYPDEIE YIFKPSCVPL MRCGGCCNDE GLECVPTEES
       110        120        130        140        150
NITMQIMRIK PHQGQHIGEM SFLQHNKCEC RPKKDRARQE NPCGPCSERR
       160        170        180        190
KHLFVQDPQT CKCSCKNTDS RCKARQLELN ERTCRCDKPR R
```

**VEGF-A (Human canonical sequence, VEGF206)(SEQ ID NO: 3)**
**(UniProtKB/TrEMBL: P15692-1)**

```
        10         20         30         40         50
MNFLLSWVHW SLALLLYLHH AKWSQAAPMA EGGGQNHHEV VKFMDVYQRS
        60         70         80         90        100
YCHPIETLVD IFQEYPDEIE YIFKPSCVPL MRCGGCCNDE GLECVPTEES
       110        120        130        140        150
NITMQIMRIK PHQGQHIGEM SFLQHNKCEC RPKKDRARQE KKSVRGKGKG
       160        170        180        190        200
QKRKRKKSRY KSWSVYVGAR CCLMPWSLPG PHPCGPCSER RKHLFVQDPQ
       210        220        230
TCKCSCKNTD SRCKARQLEL NERTCRCDKP RR
```

**PGF (Human)(SEQ ID NO: 4)**
**(UniProtKB/TrEMBL: P49763-1)**

```
        10         20         30         40         50
MPVMRLFPCF LQLLAGLALP AVPPQQWALS AGNGSSEVEV VPFQEVWGRS
        60         70         80         90        100
```

YCRALERLVD VVSEYPSEVE HMFSPSCVSL LRCTGCCGDE NLHCVPVETA
110 120 130 140 150
NVTMQLLKIR SGDRPSYVEL TFSQHVRCEC RHSPGRQSPD MPGDFRADAP
160 170 180 190 200
SFLPPRRSLP MLFRMEWGCA LTGSQSAVWP SSPVPEEIPR MHPGRNGKKQ
210 220
QRKPLREKMK PERCGDAVPR R

**VEGF-B (Human)(SEQ ID NO: 5)**
**(UniProtKB/TrEMBL: P49765-1)**

10 20 30 40 50
MSPLLRRLLL AALLQLAPAQ APVSQPDAPG HQRKVVSWID VYTRATCQPR
60 70 80 90 100
EVVVPLTVEL MGTVAKQLVP SCVTVQRCGG CCPDDGLECV PTGQHQVRMQ
110 120 130 140 150
ILMIRYPSSQ LGEMSLEEHS QCECRPKKKD SAVKPDRAAT PHHRPQPRSV
160 170 180 190 200
PGWDSAPGAP SPADITHPTP APGPSAHAAP STTSALTPGP AAAAADAAAS

SVAKGGA

**VEGF-C (Human)(SEQ ID NO: 6)**
**(UniProtKB/TrEMBL: P49767-1)**

10 20 30 40 50
MHLLGFFSVA CSLLAAALLP GPREAPAAAA AFESGLDLSD AEPDAGEATA
60 70 80 90 100
YASKDLEEQL RSVSSVDELM TVLYPEYWKM YKCQLRKGGW QHNREQANLN
110 120 130 140 150
SRTEETIKFA AAHYNTEILK SIDNEWRKTQ CMPREVCIDV GKEFGVATNT
160 170 180 190 200
FFKPPCVSVY RCGGCCNSEG LQCMNTSTSY LSKTLFEITV PLSQGPKPVT
210 220 230 240 250
ISFANHTSCR CMSKLDVYRQ VHSIIRRSLP ATLPQCQAAN KTCPTNYMWN
260 270 280 290 300
NHICRCLAQE DFMFSSDAGD DSTDGFHDIC GPNKELDEET CQCVCRAGLR
310 320 330 340 350
PASCGPHKEL DRNSCQCVCK NKLFPSQCGA NREFDENTCQ CVCKRTCPRN
360 370 380 390 400
QPLNPGKCAC ECTESPQKCL LKGKKFHHQT CSCYRRPCTN RQKACEPGFS
410

```
YSEEVCRCVP SYWKRPQMS
```

**VEGF-D (Human)(SEQ ID NO: 7)**
**(UniProtKB/TrEMBL: O43915-1)**

```
        10         20         30         40         50
MYREWVVVNV FMMLYVQLVQ GSSNEHGPVK RSSQSTLERS EQQIRAASSL
        60         70         80         90        100
EELLRITHSE DWKLWRCRLR LKSFTSMDSR SASHRSTRFA ATFYDIETLK
       110        120        130        140        150
VIDEEWQRTQ CSPRETCVEV ASELGKSTNT FFKPPCVNVF RCGGCCNEES
       160        170        180        190        200
LICMNTSTSY ISKQLFEISV PLTSVPELVP VKVANHTGCK CLPTAPRHPY
       210        220        230        240        250
SIIRRSIQIP EEDRCSHSKK LCPIDMLWDS NKCKCVLQEE NPLAGTEDHS
       260        270        280        290        300
HLQEPALCGP HMMFDEDRCE CVCKTPCPKD LIQHPKNCSC FECKESLETC
       310        320        330        340        350
CQKHKLFHPD TCSCEDRCPF HTRPCASGKT ACAKHCRFPK EKRAAQGPHS

RKNP
```

**VEGF189 (Human)(SEQ ID NO: 8)**
**(UniProtKB/TrEMBL: P15692-2)**

```
        10         20         30         40         50
MNFLLSWVHW SLALLLYLHH AKWSQAAPMA EGGGQNHHEV VKFMDVYQRS
        60         70         80         90        100
YCHPIETLVD IFQEYPDEIE YIFKPSCVPL MRCGGCCNDE GLECVPTEES
       110        120        130        140        150
NITMQIMRIK PHQGQHIGEM SFLQHNKCEC RPKKDRARQE KKSVRGKGKG
       160        170        180        190        200
QKRKRKKSRY KSWSVPCGPC SERRKHLFVQ DPQTCKCSCK NTDSRCKARQ
       210
LELNERTCRC DKPRR
```

**VEGF183 (Human)(SEQ ID NO: 9)**
**(UniProtKB/TrEMBL: P15692-3)**

```
        10         20         30         40         50
MNFLLSWVHW SLALLLYLHH AKWSQAAPMA EGGGQNHHEV VKFMDVYQRS
        60         70         80         90        100
YCHPIETLVD IFQEYPDEIE YIFKPSCVPL MRCGGCCNDE GLECVPTEES
```

```
       110        120        130        140        150
NITMQIMRIK PHQGQHIGEM SFLQHNKCEC RPKKDRARQE KKSVRGKGKG
       160        170        180        190        200
QKRKRKKSRP CGPCSERRKH LFVQDPQTCK CSCKNTDSRC KARQLELNER

TCRCDKPRR
```

**VEGF148 (Human)(SEQ ID NO: 10)**
**(UniProtKB/TrEMBL: P15692-5)**

```
        10         20         30         40         50
MNFLLSWVHW SLALLLYLHH AKWSQAAPMA EGGGQNHHEV VKFMDVYQRS
        60         70         80         90        100
YCHPIETLVD IFQEYPDEIE YIFKPSCVPL MRCGGCCNDE GLECVPTEES
       110        120        130        140        150
NITMQIMRIK PHQGQHIGEM SFLQHNKCEC RPKKDRARQE NPCGPCSERR
       160        170
KHLFVQDPQT CKCSCKNTDS RCKM
```

**VEGF145 (Human)(SEQ ID NO: 11)**
**(UniProtKB/TrEMBL: P15692-6)**

```
        10         20         30         40         50
MNFLLSWVHW SLALLLYLHH AKWSQAAPMA EGGGQNHHEV VKFMDVYQRS
        60         70         80         90        100
YCHPIETLVD IFQEYPDEIE YIFKPSCVPL MRCGGCCNDE GLECVPTEES
       110        120        130        140        150
NITMQIMRIK PHQGQHIGEM SFLQHNKCEC RPKKDRARQE KKSVRGKGKG
       160        170
QKRKRKKSRY KSWSVCDKPR R
```

**VEGF165B (Human)(SEQ ID NO: 12)**
**(UniProtKB/TrEMBL: P15692-8)**

```
        10         20         30         40         50
MNFLLSWVHW SLALLLYLHH AKWSQAAPMA EGGGQNHHEV VKFMDVYQRS
        60         70         80         90        100
YCHPIETLVD IFQEYPDEIE YIFKPSCVPL MRCGGCCNDE GLECVPTEES
       110        120        130        140        150
NITMQIMRIK PHQGQHIGEM SFLQHNKCEC RPKKDRARQE NPCGPCSERR
       160        170        180        190
KHLFVQDPQT CKCSCKNTDS RCKARQLELN ERTCRSLTRK D
```

```
VEGF121 (Human)(SEQ ID NO: 13)
(UniProtKB/TrEMBL: P15692-9)
        10         20         30         40         50
MNFLLSWVHW SLALLLYLHH AKWSQAAPMA EGGGQNHHEV VKFMDVYQRS
        60         70         80         90        100
YCHPIETLVD IFQEYPDEIE YIFKPSCVPL MRCGGCCNDE GLECVPTEES
       110        120        130        140
NITMQIMRIK PHQGQHIGEM SFLQHNKCEC RPKKDRARQE KCDKPRR

VEGF111 (Human)(SEQ ID NO: 14)
(UniProtKB/TrEMBL: P15692-10)
        10         20         30         40         50
MNFLLSWVHW SLALLLYLHH AKWSQAAPMA EGGGQNHHEV VKFMDVYQRS
        60         70         80         90        100
YCHPIETLVD IFQEYPDEIE YIFKPSCVPL MRCGGCCNDE GLECVPTEES
       110        120        130
NITMQIMRIK PHQGQHIGEM SFLQHNKCEC RCDKPRR

L-VEGF165 (Human)(SEQ ID NO: 15)
(UniProtKB/TrEMBL: P15692-11)
        10         20         30         40         50
MTDRQTDTAP SPSYHLLPGR RRTVDAAASR GQGPEPAPGG GVEGVGARGV
        60         70         80         90        100
ALKLFVQLLG CSRFGGAVVR AGEAEPSGAA RSASSGREEP QPEEGEEEEE
       110        120        130        140        150
KEEERGPQWR LGARKPGSWT GEAAVCADSA PAARAPQALA RASGRGGRVA
       160        170        180        190        200
RRGAEESGPP HSPSRRGSAS RAGPGRASET MNFLLSWVHW SLALLLYLHH
       210        220        230        240        250
AKWSQAAPMA EGGGQNHHEV VKFMDVYQRS YCHPIETLVD IFQEYPDEIE
       260        270        280        290        300
YIFKPSCVPL MRCGGCCNDE GLECVPTEES NITMQIMRIK PHQGQHIGEM
       310        320        330        340        350
SFLQHNKCEC RPKKDRARQE NPCGPCSERR KHLFVQDPQT CKCSCKNTDS
       360        370
RCKARQLELN ERTCRCDKPR R

L-VEGF121 (Human)(SEQ ID NO: 16)
(UniProtKB/TrEMBL: P15692-12)
        10         20         30         40         50
```

```
MTDRQTDTAP SPSYHLLPGR RRTVDAAASR GQGPEPAPGG GVEGVGARGV
        60         70         80         90        100
ALKLFVQLLG CSRFGGAVVR AGEAEPSGAA RSASSGREEP QPEEGEEEEE
       110        120        130        140        150
KEEERGPQWR LGARKPGSWT GEAAVCADSA PAARAPQALA RASGRGGRVA
       160        170        180        190        200
RRGAEESGPP HSPSRRGSAS RAGPGRASET MNFLLSWVHW SLALLLYLHH
       210        220        230        240        250
AKWSQAAPMA EGGGQNHHEV VKFMDVYQRS YCHPIETLVD IFQEYPDEIE
       260        270        280        290        300
YIFKPSCVPL MRCGGCCNDE GLECVPTEES NITMQIMRIK PHQGQHIGEM
       310        320
SFLQHNKCEC RPKKDRARQE KCDKPRR
```

**L-VEGF189 (Human)(SEQ ID NO: 17)**
**(UniProtKB/TrEMBL: P15692-13)**

```
        10         20         30         40         50
MTDRQTDTAP SPSYHLLPGR RRTVDAAASR GQGPEPAPGG GVEGVGARGV
        60         70         80         90        100
ALKLFVQLLG CSRFGGAVVR AGEAEPSGAA RSASSGREEP QPEEGEEEEE
       110        120        130        140        150
KEEERGPQWR LGARKPGSWT GEAAVCADSA PAARAPQALA RASGRGGRVA
       160        170        180        190        200
RRGAEESGPP HSPSRRGSAS RAGPGRASET MNFLLSWVHW SLALLLYLHH
       210        220        230        240        250
AKWSQAAPMA EGGGQNHHEV VKFMDVYQRS YCHPIETLVD IFQEYPDEIE
       260        270        280        290        300
YIFKPSCVPL MRCGGCCNDE GLECVPTEES NITMQIMRIK PHQGQHIGEM
       310        320        330        340        350
SFLQHNKCEC RPKKDRARQE KKSVRGKGKG QKRKRKKSRY KSWSVPCGPC
       360        370        380        390
SERRKHLFVQ DPQTCKCSCK NTDSRCKARQ LELNERTCRC DKPRR
```

**L-VEGF206 (Human)(SEQ ID NO: 18)**
**(UniProtKB/TrEMBL: P15692-14)**

```
        10         20         30         40         50
MTDRQTDTAP SPSYHLLPGR RRTVDAAASR GQGPEPAPGG GVEGVGARGV
        60         70         80         90        100
ALKLFVQLLG CSRFGGAVVR AGEAEPSGAA RSASSGREEP QPEEGEEEEE
       110        120        130        140        150
KEEERGPQWR LGARKPGSWT GEAAVCADSA PAARAPQALA RASGRGGRVA
```

```
       160        170        180        190        200
RRGAEESGPP HSPSRRGSAS RAGPGRASET MNFLLSWVHW SLALLLYLHH
       210        220        230        240        250
AKWSQAAPMA EGGGQNHHEV VKFMDVYQRS YCHPIETLVD IFQEYPDEIE
       260        270        280        290        300
YIFKPSCVPL MRCGGCCNDE GLECVPTEES NITMQIMRIK PHQGQHIGEM
       310        320        330        340        350
SFLQHNKCEC RPKKDRARQE KKSVRGKGKG QKRKRKKSRY KSWSVYVGAR
       360        370        380        390        400
CCLMPWSLPG PHPCGPCSER RKHLFVQDPQ TCKCSCKNTD SRCKARQLEL
       410
NERTCRCDKP RR
```

**VEGFA Isoform 15 (Human)(SEQ ID NO: 19)**
**(UniProtKB/TrEMBL: P15692-15)**

```
        10         20         30         40         50
MTDRQTDTAP SPSYHLLPGR RRTVDAAASR GQGPEPAPGG GVEGVGARGV
        60         70         80         90        100
ALKLFVQLLG CSRFGGAVVR AGEAEPSGAA RSASSGREEP QPEEGEEEEE
       110        120        130        140        150
KEEERGPQWR LGARKPGSWT GEAAVCADSA PAARAPQALA RASGRGGRVA
       160        170        180        190        200
RRGAEESGPP HSPSRRGSAS RAGPGRASET MNFLLSWVHW SLALLLYLHH
       210        220        230        240        250
AKWSQAAPMA EGGGQNHHEV VKFMDVYQRS YCHPIETLVD IFQEYPDEIE
       260        270        280        290        300
YIFKPSCVPL MRCGGCCNDE GLECVPTEES NITMQIMRIK PHQGQHIGEM
       310        320        330        340        350
SFLQHNKCEC RPKKDRARQE NPCGPCSERR KHLFVQDPQT CKCSCKNTDS
       360        370
RCKARQLELN ERTCRSLTRK D
```

**VEGFA Isoform 16 (Human)(SEQ ID NO: 20)**
**(UniProtKB/TrEMBL: P15692-16)**

```
        10         20         30         40         50
MTDRQTDTAP SPSYHLLPGR RRTVDAAASR GQGPEPAPGG GVEGVGARGV
        60         70         80         90        100
ALKLFVQLLG CSRFGGAVVR AGEAEPSGAA RSASSGREEP QPEEGEEEEE
       110        120        130        140        150
KEEERGPQWR LGARKPGSWT GEAAVCADSA PAARAPQALA RASGRGGRVA
       160        170        180        190        200
```

```
RRGAEESGPP HSPSRRGSAS RAGPGRASET MNFLLSWVHW SLALLLYLHH
       210        220        230        240        250
AKWSQAAPMA EGGGQNHHEV VKFMDVYQRS YCHPIETLVD IFQEYPDEIE
       260        270        280        290        300
YIFKPSCVPL MRCGGCCNDE GLECVPTEES NITMQIMRIK PHQGQHIGEM
       310        320        330        340        350
SFLQHNKCEC RPKKDRARQE KKSVRGKGKG QKRKRKKSRP CGPCSERRKH
       360        370        380
LFVQDPQTCK CSCKNTDSRC KARQLELNER TCRCDKPRR
```

**VEGFA Isoform 17 (Human)(SEQ ID NO: 21)**
**(UniProtKB/TrEMBL: P15692-17)**

```
        10         20         30         40         50
MTDRQTDTAP SPSYHLLPGR RRTVDAAASR GQGPEPAPGG GVEGVGARGV
        60         70         80         90        100
ALKLFVQLLG CSRFGGAVVR AGEAEPSGAA RSASSGREEP QPEEGEEEEE
       110        120        130        140        150
KEEERGPQWR LGARKPGSWT GEAAVCADSA PAARAPQALA RASGRGGRVA
       160        170        180        190        200
RRGAEESGPP HSPSRRGSAS RAGPGRASET MNFLLSWVHW SLALLLYLHH
       210        220        230        240        250
AKWSQAAPMA EGGGQNHHEV VKFMDVYQRS YCHPIETLVD IFQEYPDEIE
       260        270        280        290        300
YIFKPSCVPL MRCGGCCNDE GLECVPTEES NITMQIMRIK PHQGQHIGEM
       310        320        330        340        350
SFLQHNKCEC RPKKDRARQE NPCGPCSERR KHLFVQDPQT CKCSCKNTDS

RCKM
```

**VEGFA Isoform 18 (Human)(SEQ ID NO: 22)**
**(UniProtKB/TrEMBL: P15692-18)**

```
        10         20         30         40         50
MTDRQTDTAP SPSYHLLPGR RRTVDAAASR GQGPEPAPGG GVEGVGARGV
        60         70         80         90        100
ALKLFVQLLG CSRFGGAVVR AGEAEPSGAA RSASSGREEP QPEEGEEEEE
       110        120        130        140        150
KEEERGPQWR LGARKPGSWT GEAAVCADSA PAARAPQALA RASGRGGRVA
       160        170        180        190        200
RRGAEESGPP HSPSRRGSAS RAGPGRASET MNFLLSWVHW SLALLLYLHH
       210        220        230        240        250
AKWSQAAPMA EGGGQNHHEV VKFMDVYQRS YCHPIETLVD IFQEYPDEIE
```

```
       260        270        280        290        300
YIFKPSCVPL MRCGGCCNDE GLECVPTEES NITMQIMRIK PHQGQHIGEM
       310
SFLQHNKCEC RCDKPRR
```

**rhVEGF-165 (SEQ ID NO: 23)**

APMAEGGGQNHHEVVKFMDVYQRSYCHPIETLVDIFQEYPDEIEYIFKPSCVPLMRCGGCCNDEGLECVPTEESNITMQIMRIKPHQGQHIGEMSFLQHNKCECRPK
KDRARQENPCGPCSERRKHLFVQDPQTCKCSCKNTDSRCKARQLELNERTCRCDKPRR

**proHB-EGF (SEQ ID NO: 24)**

```
        1 mkllpsvvlk lflaavlsal vtgeslerlr rglaagtsnp dpptvstdql lplgggrdrk
       61 vrdlqeadld llrvtlsskp qalatpnkee hgkrkkkgkg lgkkrdpclr kykdfcihge
      121 ckyvkelrap scichpgyhg erchglslpv enrlytydht tilavvavvl ssvcllvivg
      181 llmfryhrrg gydveneekv klgmtnsh
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20060040860 A
- WO 02086497 A
- WO 2016198121 A
- US 4522811 A
- US 6468798 B
- US 6695227 B
- US 7431222 B
- US 20050224608 A
- US 4268460 A
- US 4253468 A
- US 4046146 A
- US 3826255 A
- US 4649911 A
- US 4510829 A


### Non-patent literature cited in the description


- **FERRARA ; NAPOLEONE ; HANS-PETER GERBER ; JENNIFER LECOUTE.** The biology of VEGF and its receptors. *Nature medicine,* 2003, vol. 9.6, 669-676
- **K. A. HOUCK ; D. W. LEUNG ; A. M. ROWLAND ; J. WINER ; N. FERRARA.** Dual regulation of vascular endothelial growth factor bioavailability by genetic and proteolytic mechanisms. *J. Biol. Chem.,* December 1992, vol. 267 (36), 26031-7
- **D. KRILLEKE et al.** The heparin-binding domain confers diverse functions of VEGF-A in development and disease: a structure-function study. *Biochem. Soc. Trans.,* December 2009, vol. 37, 1201-6
- Remington: The Science and Practice of Pharmacy. 2005
- Drugs and the Pharmaceutical Sciences: a Series of Textbooks and Monographs. Dekker
- **SAYANI, AMYN P. ; YIE W. CHIEN.** Systemic delivery of peptides and proteins across absorptive mucosae. *Critical reviews in therapeutic drug carrier systems,* 1995, vol. 13 (1-2), 85-184
- **NIVEN, RALPH W.** Delivery of biotherapeutics by inhalation aerosol. *Critical Reviews™ in Therapeutic Drug Carrier Systems,* 1995, vol. 12 (2-3
- **AGU, REMIGIUS UCHENNA et al.** The lung as a route for systemic delivery of therapeutic proteins and peptides. *Respiratory research,* 2001, vol. 2 (4), 198
- **SAYANI, AMYN P. ; YIE W. CHIEN.** Systemic delivery of peptides and proteins across absorptive mucosae. *Critical reviews in therapeutic drug carrier systems,* 1995, vol. 13 (1-2), 85-184
- **NEWMAN, S. P.** Aerosols and the Lung. 197-22
- **WRIGHT, B. M.** *Lancet,* 1958, vol. 3, 24-25
- **MERCER et al.** *Am. Ind. HYR. Assoc. J.,* 1968, vol. 29, 66-78
- **T. T. MERCER.** *Chest,* 1981, vol. 80 (6), 813-817
- **WEIBEL ER ; GOMEZ DM.** A principle for counting tissue structures on random sections. *J. Appl. Physiol,* 1962, vol. 17, 343-8
- **Y. LI et al.** The effect of heparin administration in animal models of sepsis: a prospective study in Escherichia coli-challenged mice and a systematic review and metaregression analysis of published studies. *Crit. Care Med.,* May 2011, vol. 39 (5), 1104-12
- **W. SCHERLE.** A simple method for volumetry of organs in quantitative stereology. *Mikroskopie,* June 1970, vol. 26 (1), 57-60
- **M. OCHS ; C. MÜHLFELD.** Quantitative microscopy of the lung: a problem-based approach. Part 1: basic principles of lung stereology. *Am. J. Physiol. Lung Cell. Mol. Physiol.,* July 2013, vol. 305 (1), L15-22
- **M. PRESTA et al.** Fibroblast growth factor/fibroblast growth factor receptor system in angiogenesis. *Cytokine Growth Factor Rev.,* April 2005, vol. 16 (2), 159-178
- **S. GUIMOND et al.** Activating and inhibitory heparin sequences for FGF-2 (basic FGF). Distinct requirements for FGF-1, FGF-2, and FGF-4. *J. Biol. Chem.,* November 1993, vol. 268 (32), 23906-14
- **D. COLTRINI et al.** Different effects of mucosal, bovine lung and chemically modified heparin on selected biological properties of basic fibroblast growth factor. *Biochem. J.,* October 1994, 583-90
- **P. S. PULIGANDLA et al.** Management of congenital diaphragmatic hernia: A systematic review from the APSA outcomes and evidence based practice committee. *J. Pediatr. Surg.,* November 2015, vol. 50 (11), 1958-1970

- **S. TESSLER et al.** Heparin modulates the interaction of VEGF165 with soluble and cell associated flk-1 receptors. *J. Biol. Chem.,* April 1994, vol. 269 (17), 12456-61
- **E. WIJELATH et al.** Multiple mechanisms for exogenous heparin modulation of vascular endothelial growth factor activity. *J. Cell. Biochem.,* June 2010, vol. 111 (2), 461-468
- **N. ITO ; L. CLAESSON-WELSH.** Dual effects of heparin on VEGF binding to VEGF receptor-1 and transduction of biological responses. *Angiogenesis,* 1999, vol. 3 (2), 159-66
- **S. KOCH ; S. TUGUES ; X. LI ; L. GUALANDI ; L. CLAESSON-WELSH.** Signal transduction by vascular endothelial growth factor receptors. *Biochem. J.,* July 2011, vol. 437 (2), 169-83
- **L. FUX ; N. ILAN ; R. D. SANDERSON ; I. VLODAVSKY.** Heparanase: busy at the cell surface. *Trends Biochem. Sci.,* October 2009, vol. 34 (10), 511-519
- **T. TAKAHASHI ; S. YAMAGUCHI ; K. CHIDA ; M. SHIBUYA.** A single autophosphorylation site on KDR/Flk-1 is essential for VEGF-A-dependent activation of PLC-gamma and DNA synthesis in vascular endothelial cells. *EMBO J.,* June 2001, vol. 20 (11), 2768-2778
- **M. A. EAST ; D. I. LANDIS ; M. A. THOMPSON ; B. H. ANNEX.** Effect of single dose of intravenous heparin on plasma levels of angiogenic growth factors. *Am. J. Cardiol.,* May 2003, vol. 91 (10), 1234-6
- **L. W. CLEGG ; F. MAC GABHANN.** Site-Specific Phosphorylation of VEGFR2 Is Mediated by Receptor Trafficking: Insights from a Computational Model. *PLoS Comput. Biol.,* June 2015, vol. 11 (6), e1004158
- **T. TAKAHASHI et al.** A single autophosphorylation site on KDR/Flk-1 is essential for VEGF-A-dependent activation of PLC-gamma and DNA synthesis in vascular endothelial cells. *EMBO J.,* June 2001, vol. 20 (11), 2768-2778
- **J. XU et al.** Role of PKC and MAPK in cytosolic PLA2 phosphorylation and arachadonic acid release in primary murine astrocytes. *J. Neurochem.,* October 2002, vol. 83 (2), 259-70
- **X. ZHU et al.** IL-5-induced integrin adhesion of human eosinophils caused by ERK1/2-mediated activation of cPLA2. *J. Leukoc. Biol.,* November 2002, vol. 72 (5), 1046-53
- **E. HOFER ; B. SCHWEIGHOFER.** Signal transduction induced in endothelial cells by growth factor receptors involved in angiogenesis. *Thromb. Haemost.,* March 2007, vol. 97 (3), 355-63
- **R. R. THIAGARAJAN et al.** Extracorporeal Life Support Organization Registry International Report 2016. *ASAIO J.,* 2017, vol. 63 (1), 60-67
- **WEIBEL ER ; GOMEZ DM.** A principle for counting tissue structures on random sections. *J. Appl. Physiol.,* 1962, vol. 17, 343-8